(19) Europäisches Patentamt / European Patent Office / Office européen des brevets

(11) **EP 4 768 583 A1**

(12) **EUROPEAN PATENT APPLICATION**
published in accordance with Art. 153(4) EPC

(43) Date of publication:
**01.07.2026 Bulletin 2026/27**

(21) Application number: **24855727.4**

(22) Date of filing: **16.08.2024**

(51) International Patent Classification (IPC):
***C12N 15/113*** *(2010.01)* ***A61K 31/7125*** *(2006.01)*
***A61K 31/713*** *(2006.01)* ***A61K 31/7088*** *(2006.01)*
***A61P 3/00*** *(2006.01)*

(52) Cooperative Patent Classification (CPC):
**A61K 31/7088; A61K 31/7125; A61K 31/713; A61P 3/00; C12N 15/113**

(86) International application number:
**PCT/CN2024/112606**

(87) International publication number:
**WO 2025/040002 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.08.2023 CN 202311053101**

(71) Applicant: **Rigerna Therapeutics (Suzhou) Co., Ltd.**
**Suzhou, Jiangsu 215128 (CN)**

(72) Inventors:
- **HUANG, Yuanyu**
  **Suzhou, Jiangsu 215128 (CN)**
- **LI, Haitao**
  **Suzhou, Jiangsu 215128 (CN)**
- **KONG, Lina**
  **Suzhou, Jiangsu 215128 (CN)**

(74) Representative: **Elzaburu S.L.P.**
**Paseo de la Castellana 259C**
**Torre de Cristal, planta 28**
**28046 Madrid (ES)**

Remarks:
The complete document including Reference Table(s) and the Sequence Listing(s) can be downloaded from the EPO website

# (54) INHBE GENE-TARGETED DOUBLE-STRANDED OLIGONUCLEOTIDE, CONJUGATE, COMPOSITION AND USE THEREOF

(57) Provided are an INHBE gene-targeted double-stranded oligonucleotide, a conjugate, and a composition, which relate to the technical field of nucleic acid drugs. The double-stranded oligonucleotide comprises a sense strand and an antisense strand. The antisense strand is complementary or substantially complementary to the sense strand; the sense strand comprises a nucleotide sequence identical or substantially identical to at least 15 contiguous nucleotides in a sequence of SEQ ID NO: 309, and said substantially identical means that there is no more than three nucleotide differences between the sense strand and the at least 15 contiguous nucleotides in the sequence of SEQ ID NO: 309. The double-stranded oligonucleotide can effectively inhibit the INHBE gene and treat patients suffering from metabolic diseases or metabolic syndrome and related diseases such as diabetes, hypertension and cardiovascular diseases.

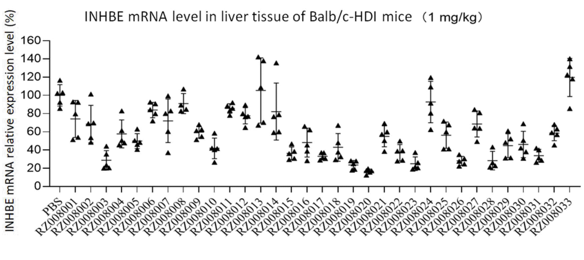


FIG. 1

EP 4 768 583 A1

## Description

### CROSS-REFERENCE TO RELATED APPLICATIONS

The present application claims the priority to China patent application 202311053101.0 filed on August 21, 2023 with the China National Intellectual Property Administration, titled "INHBE gene-targeted double-stranded oligonucleotide, conjugate, composition and use thereof", the contents of which are incorporated herein by reference in its entirety.

### TECHNICAL FIELD

The present disclosure belongs to the technical field of nucleic acid drugs, and particularly relates to a double-stranded oligonucleotide targeting INHBE gene, conjugate of the double-stranded oligonucleotide, pharmaceutical composition comprising the double-stranded oligonucleotide and/or the double-stranded oligonucleotide conjugate, and use thereof.

### BACKGROUND

Metabolic disorders are common, and common drug treatments include lipid-lowering agents such as statins and other drugs. However, these drug treatments are often limited by frequent administration and drug-drug interactions. Pharmaceutical agents that can effectively silence disease-related genes, such as inhibiting INHBE, and thereby inhibit metabolic disorders, have not yet appeared in the market.

Therefore, there is an urgent need in the art for drugs that effectively inhibit the target INHBE gene, thereby treating subjects suffering from metabolic disorders or metabolic syndromes and related diseases such as diabetes, hypertension and cardiovascular diseases.

### SUMMARY

In view of this, the present disclosure provides double-stranded oligonucleotides targeting the INHBE gene, conjugates, compositions and uses thereof. The present disclosure inhibits the expression of the INHBE gene by administering to a subject an INHBE specific inhibitor (double-stranded oligonucleotide, conjugate, composition, etc.) to treat a disease or disorder associated with the INHBE gene.

In order to solve the above technical problems, the present disclosure adopts the following technical solutions: In the first aspect of the present disclosure, the present disclosure provides a double-stranded oligonucleotide targeting the INHBE gene, wherein the double-stranded oligonucleotide comprises a sense strand and an antisense strand, wherein the antisense strand is complementary or substantially complementary to the sense strand; said substantially complementary means that the sense strand and the antisense strand have a mismatch of no more than 3 nucleotides in the duplex region.

Further, the double-stranded oligonucleotide comprises a sense strand and an antisense strand, wherein the antisense strand comprises at least 15 contiguous nucleotides of any one of the sequences shown in SEQ ID NO:155 - SEQ ID NO: 308 in Table 1, or a nucleotide sequence having no more than 3 nucleotide differences from the at least 15 contiguous nucleotides.

The sense strand comprises a nucleotide sequence that is at least partially reverse complementary or substantially complementary to the antisense strand to form a duplex region; said substantially complementary means that the sense strand and the antisense strand have a mismatch of no more than 3 nucleotides in the duplex region.

In some optional embodiments of the present disclosure, each nucleotide in the double-stranded oligonucleotide is independently selected from unmodified or modified nucleotides.

In the second aspect of the present disclosure, the present disclosure provides a double-stranded oligonucleotide conjugate, wherein the conjugate comprising the double-stranded oligonucleotide and one or more ligands capable of binding to a cellular receptor.

In some optional embodiments of the present disclosure, the ligand is conjugated to the sense strand and/or the antisense strand.

In the third aspect of the present disclosure, the present disclosure provides a composition, wherein the composition comprising any one of:

(I) the double-stranded oligonucleotide according to the first aspect; and/or
(II) the conjugate according to the second aspect.

In the fourth aspect of the present disclosure, the present disclosure provides a use of any one of:

(I) the double-stranded oligonucleotide according to the first aspect; and/or
(II) the conjugate according to the second aspect; and/or
(III) the composition according to the third aspect,

in the manufacture of a medicament for the prevention and/or treatment of a disease or disorder mediated by INHBE gene.

In some optional embodiments of the present disclosure, the disease or disorder comprises, but is not limited to, having or being at the risk of developing metabolic disorder, type 2 diabetes, obesity, elevated triglyceride levels, lipodystrophy, liver inflammation, fatty liver disease, hypercholesterolemia, elevated liver enzymes, non-alcoholic steatohepatitis (NASH), cardiovascular disease, cardiomyopathy, hypertension, and/or heart failure.

In the fifth aspect of the present disclosure, the present disclosure provides a pharmaceutical composition comprising any one of:

(I) the double-stranded oligonucleotide according to the first aspect; and/or
(II) the conjugate according to the second aspect; and/or
(III) the composition according to the third aspect, and

a pharmaceutically acceptable excipient or auxiliary agent.

In the sixth aspect of the present disclosure, the present disclosure provides a method for reducing the expression or activity of INHBE gene, comprising contacting a cell with any one of:

(I) the double-stranded oligonucleotide according to the first aspect; and/or
(II) the conjugate according to the second aspect; and/or
(III) the composition according to the third aspect; and/or
(IV) the pharmaceutical composition according to the fifth aspect.

In the seventh aspect of the present disclosure, the present disclosure provides a method for preventing and/or treating a disease or disorder mediated by INHBE gene, comprising administering to a subject pharmaceutically acceptable amount of any one of:

(I) the double-stranded oligonucleotide according to the first aspect; and/or
(II) the conjugate according to the second aspect; and/or
(III) the composition according to the third aspect; and/or
(IV) the pharmaceutical composition according to the fifth aspect.

It is well known to those skilled in the art that modified nucleotide groups can be introduced into the double-stranded oligonucleotide conjugates described in the present invention by using nucleoside monomers with corresponding modifications: methods for preparing nucleoside monomers with corresponding modifications and methods for introducing modified nucleotide groups into double-stranded oligonucleotide conjugates are also well known to those skilled in the art. All modified nucleoside monomers are commercially available or prepared using known methods.

## BRIEF DESCRIPTION OF THE DRAWINGS

FIG. 1 Inhibitory activity of target genes in Balb/c-HDI mice after administration of siRNA conjugates.
FIG. 2 Inhibitory activity of target genes in C57BL/6J mice after administration of siRNA conjugates.
FIG. 3 Inhibitory activity of target genes in C57BL/6J mice after administration of different doses of RZM08019.
FIG. 4 Triglyceride levels in serum of BKS-DB mice after repeated administration of RZM08019.
FIG. 5 Total cholesterol levels in serum of BKS-DB mice after repeated administration of RZM08019.
FIG. 6 Inhibitory activity of target genes in Huh7 cells after administration of siRNA conjugates.

## DETAILED EMBODIMENTS

The technical solutions in the examples of the present disclosure will be described clearly and completely below. Obviously, the described examples are only part of the embodiments of the present disclosure, but not all of them. Based on the examples in the present disclosure, all other embodiments obtained by a person of ordinary skill in the art without creative effort fall within the protection scope of the present disclosure.

**Interpretation of terminology**

Unless otherwise stated, the following definitions as used herein shall apply. For purposes of the present disclosure, the chemical elements are consistent with the CAS version of the Periodic Table of the Elements, and Handbook of Chemistry and Physics, 75th Edition, 1994. In addition, general principles of organic chemistry can be referred to in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference. Alkylene groups may be substituted or unsubstituted.

As used in the present disclosure, "halogen" or "halo" refers to any of the radio-stable atoms of Column 7 of the Periodic Table of the Elements, such as fluorine, chlorine, bromine, or iodine, with fluorine and chlorine being preferred, and fluorine being more preferred.

As used in the present disclosure,"alkyl" refers to a linear or branched alkane chain that is fully saturated (i.e., contains no double or triple bonds). Alkyl may have 1 to 6 carbon atoms (whenever it appears herein, a numerical range such as "1 to 6" refers to each integer in the given range; e.g.," 1 to 6 carbon atoms" means that alkyl may consist of 1 carbon atom, 2 carbon atoms, 3 carbon atoms, 4 carbon atoms, 5 carbon atoms, or 6 carbon atoms, although this definition also encompasses the term "alkyl" where no numerical range is specified). By way of example only,"C1-C4 alkyl" means having one to four carbon atoms in the alkyl chain, i.e., the C1-C4 alkyl is selected from methyl, ethyl, propyl, isopropyl, n-butyl, isobutyl, sec-butyl, or tert-butyl. Typical alkyl groups include, but are limited to, methyl, ethyl, propyl, isopropyl, butyl, isobutyl, tert-butyl, pentyl, hexyl, etc. Alkyl groups may be substituted or unsubstituted.

As used herein, "alkoxy" refers to the formula -OR, wherein R is alkyl as defined above, e.g.,"C1-6 alkoxy," which includes, but is not limited to, methoxy, ethoxy, n-propoxy, 1-methylethoxy (isopropoxy), n-butoxy, isobutoxy, sec-butoxy, tert-butoxy, etc.

As used in the present disclosure, "alkylene" refers to a fully saturated linear or branched alkylene chain of formula -R-. By way of example only, "C1-10 alkylene" means having one to ten carbon atoms in the alkyl chain, i.e., the C1-10 alkylene is selected from methylene ($-CH_2-$), ethylene ($=CH_2CH_3$), 1,2-ethylene ($-CH_2CH_2-$), n-propylene ($-CH_2CH_2CH_2-$), isopropylene ($-CH_2CH(CH_3)-$), etc.

As used in the present disclosure, the articles "a", "an" and "the" are intended to include "at least one" or "one or more". Thus, as used herein, these articles refer to articles with one or more (i.e., at least one) objects. For example, "a component" refers to one or more components, i.e., there may be more than one component may be considered for adoption or use in the implementation of the embodiment.

In the present disclosure, the terms "include", "comprise", "having", "may", "comprising" and variants thereof are generally intended to be open-ended transitional phrases, terms, or words that do not exclude the possibility of additional acts or structures. The term "consisting of" generally means that no other component (or likewise, feature, integer, step, etc.) can be present. Unless the context clearly requires otherwise, indefinite nouns also include plural referents.

In the present disclosure, the terms "optionally", "optional" or "option" generally mean that the event or circumstance described subsequently may or may not occur, and the description includes instances in which the event or circumstance occurs and instances in which it does not occur.

As used in the present disclosure, "comprising" is an open-ended expression, that is, it includes the contents specified in the present disclosure, but does not exclude other aspects of contents.

As used in the present disclosure, "optionally substituted" is used to define a variable that may be unsubstituted.

As used in the present disclosure, "unsubstituted" means that the specified group bears no substituents.

As used in the present disclosure, "substituted", "substituted" and "substituted" are used interchangeably to mean that one or more hydrogen atoms in a given structure are replaced with a specified substituent. Unless otherwise indicated, a substituted group may have a substituent substituted at each substitutable position of the group. When more than one position in a given formula can be substituted with one or more substituents selected from a specified group, then the substituents may be substituted identically or differently at each substitutable position.

As used in the present disclosure, "each... independently selected from", "...each independently selected from" and "... independently selected from" are interchangeable and should be interpreted broadly, which may mean that specific options expressed between the same symbols in different groups do not affect each other, or that specific options expressed between the same symbols in the same group do not affect each other.

In the present disclosure, the terms "include", "comprise", "having", "may", "comprising" and variants thereof are generally intended to be open-ended transitional phrases, terms, or words that do not exclude the possibility of additional acts or structures. The term "consisting of" generally means that no other component (or likewise, feature, integer, step, etc.) can be present. Unless the context clearly requires otherwise, indefinite nouns also include plural referents.

As used in the present disclosure, "small interfering RNA (siRNA)" is a type of double-stranded RNA that includes a sense strand and an antisense strand. siRNA mediates targeted cleavage of RNA transcripts via RISC pathway by forming RNA-induced silencing complexes (RISC). Specifically, siRNAs direct specific degradation of mRNA sequences through known RNA interference (RNAi) processes, inhibiting the translation of mRNA into amino acids and consequently

into proteins.

In the present disclosure, a double-stranded oligonucleotide consists of two strands, wherein the strand that binds to the target sequence is referred to as the antisense strand or leader strand and the other strand is referred to as the sense strand or passenger strand. The term "antisense strand" refers to a strand of a double-stranded oligonucleotide that includes a region that is completely or substantially complementary to a target sequence. The term "sense strand" refers to a strand of a double-stranded oligonucleotide that includes a region substantially complementary to a region of the antisense strand as the term is defined herein. The term "complementary region" refers to a region on the antisense strand that is completely or substantially complementary to a target sequence. In cases where the complementary region is not perfectly complementary to the target sequence, the mismatch may be located in the interior or terminal region of the molecule. As used herein, the term "complementary" refers to the ability of a first polynucleotide to hybridize to a second polynucleotide under certain conditions, e.g., stringent conditions. Herein, "double-stranded oligonucleotide" and "siRNA" may be used interchangeably.

In the present disclosure, the expressions "complementary" and "reverse complementary" are used interchangeably and have meanings well known to those skilled in the art, i.e., in a double-stranded nucleic acid molecule, the bases of one strand each pair with bases on the other strand in a complementary manner.

In the present disclosure, unless otherwise specified, "substantially reverse complementary" or "substantially complementary" means that there are no more than three base mismatches between the two nucleotide sequences involved; "essentially reverse complementary" means that there are no more than one base mismatch between the two nucleotide sequences; and "completely reverse complementary" means that there are no base mismatches between the two nucleotide sequences.

In the present disclosure, a "nucleotide difference" between one nucleotide sequence and another nucleotide sequence means that the nucleotide type at the same position in the former nucleotide sequence is changed compared with that in the latter nucleotide sequence. For example, when one nucleotide base in the latter nucleotide sequence is A and the corresponding nucleotide base at the same position in the former nucleotide sequence is U, C, G or T, it is determined that there is a nucleotide difference between the two nucleotide sequences at that position. In some embodiments, when a nucleotide at a position is replaced by an abased nucleotide or equivalent thereof, a nucleotide difference at that position can also be considered to have occurred.

In the present disclosure,"fluoro-modified nucleotide" or "2'-fluoro-modified nucleotide" refers to a nucleotide in which the hydroxyl group at the 2'-position of the ribose group of the nucleotide is substituted with fluorine, and "non-fluoro-modified nucleotide" refers to a nucleotide or nucleotide analog in which the hydroxyl group at the 2'-position of the ribose group of the nucleotide is substituted with a non-fluorine group. The "methoxy modified nucleotide" or "2'-O-methoxyethyl modified nucleotide" refers to a nucleotide formed by substituting the 2'hydroxyl group of the ribose group with methoxy or methoxyethyl, and the "methoxy modified nucleotide" is also described as 2'-OMe or 2'-O-methyl modified nucleotide, and the "2'-O-methoxyethyl modified nucleotide" can be used alternatively and is also described as 2'-MOE modification; and the 2'-deoxynucleotide refers to a nucleotide in which the 2' position of the ribose group is hydrogen.

The terms "RNAi", "iRNA", "RNAi agent", "RNAi reagent", "RNA interfering agent", and "RNA inhibitor" can be used interchangeably herein, refer to a molecule or agent that comprises RNA and can mediate targeted cleavage of RNA transcripts through the RNA-induced silencing complex (RISC) pathway. RNA directing sequence-specific degradation of mRNA through a process known as RNA interference (RNAi) is well known in the art. In one embodiment, an RNAi agent of the present disclosure includes a single stranded or double stranded RNA that interacts with a target RNA sequence to direct cleavage of the target RNA. Thus, in one aspect, the present disclosure relates to the term "siRNA" may also be used to refer to RNAi as described above.

In some embodiments, "RNAi" as used in the compositions, uses, and methods of the present disclosure is double stranded RNA, and "RNAi agent" includes the double stranded RNA, and "RNAi agent" may refer to "double stranded RNAi agent", "double stranded RNA (dsRNA) molecule", "dsRNA reagent", "siRNA agent" or "dsRNA agent".

In the present disclosure, the term "dsRNA" or "siRNA" refers to a complex of ribonucleic acid molecules that has a double-stranded structure comprising two antiparallel and substantially complementary nucleic acid strands, said to have "sense" and "antisense" orientations relative to the target RNA.

Furthermore, as used herein, an "RNAi reagent" or "RNAi agent" can include ribonucleotides having chemical modifications and/or ligands; RNAi agents can include substantial modifications at multiple nucleotides. The term "modified nucleotide" refers to a nucleotide that independently has modified sugar moieties, modified internucleotide linkages, and/or modified nucleobases. Thus, the term modified nucleotide encompasses substitution, addition or removal of e.g. functional groups or atoms to an internucleotide bond, sugar or nucleobase. Modifications suitable for use in the agents of the present disclosure include all types of modifications disclosed herein or known in the art. As used in siRNA molecules, any such modification may be encompassed by an "RNAi agent."

In the present disclosure, the term "nucleotide overhang" or "overhang" refers to at least one unpaired nucleotide that protrudes from the duplex structure (e.g., dsRNA) of an iRNA. For example, nucleotide overhangs are present when the 3'-end of one strand of dsRNA extends beyond the 5'-end of the other strand or vice versa. The one or more overhangs

may be on the sense strand, the antisense strand, or any combination thereof. Additionally, one or more nucleotides of the overhang may be present on the 5'-end, the 3'-end, or both ends of the antisense or sense strand of the dsRNA.

In the present disclosure, the term "ligand" generally refers to any compound or molecule capable of covalently or otherwise chemically binding to a biologically active substance, such as an oligonucleotide. In certain embodiments, the ligand is capable of interacting directly or indirectly with another compound, such as a receptor, the receptor with which the ligand interacts may be present on the cell surface, or alternatively may be an intracellular and/or intercellular receptor, the interaction of the ligand with the receptor may result in a biochemical reaction, or may be merely a physical interaction or binding.

The term "linked", when referring to an association between two molecules, means that the two molecules are linked by a covalent bond or that the two molecules are associated via a noncovalent bond (e.g., hydrogen or ionic bond).

Each nucleotide in the sense strand and antisense strand is independently a modified or unmodified nucleotide. In the context of the present invention, unless otherwise specified, "conjugating" means that two or more chemical moieties, each having a specific function, are covalently linked to each other; accordingly, "conjugate" means a compound formed by covalent linkage between the individual chemical moieties. Further, "siRNA conjugate" refers to a compound formed by covalently attachment of one or more chemical moieties having a specific function to an siRNA. Hereinafter, the siRNA conjugate of the present invention is also sometimes referred to simply as "conjugate". siRNA conjugates shall be understood as a general term for siRNA conjugates, either the first siRNA conjugate or the second siRNA conjugate, or siRNA sense strand conjugate or siRNA antisense strand conjugate, depending on the context.

In the present disclosure, the term "pharmaceutical composition" or "composition" may refer to those used for treatment of a disease, or may be used for *in vitro* culture experiments of cells. The term "pharmaceutical composition" when used in the treatment of disease generally refers to unit dosage forms and may be prepared by any of the methods well known in the pharmaceutical art. All methods include the step of combining the active ingredient with excipients which serve as one or more accessory components. Generally, compositions are prepared by uniformly and sufficiently combining active siRNA with liquid excipients, finely divided solid excipients, or both.

In the present disclosure, the term "pharmaceutically acceptable" means that the substance or composition must be chemically and/or toxicologically compatible with the other ingredients comprising the formulation and/or the mammal being treated therewith. Preferably, as used in the present disclosure, "pharmaceutically acceptable" means approved by a federal regulatory agency or national government or listed in the United States Pharmacopeia or other generally recognized pharmacopeia for use in animals, particularly humans.

As used herein, "pharmaceutically acceptable carrier or excipient" may include any solvent, solid excipient, diluent or other liquid excipient, and the like, suitable for the particular target dosage form. In addition to any conventional excipients, the extent to which siRNAs of the present disclosure are incompatible, e.g., produce any undesirable biological effects or interact in a deleterious manner with any other component of a pharmaceutically acceptable composition, their use is also contemplated by the present disclosure.

As used in the present disclosure,"treat", "alleviate" or "ameliorate" are used interchangeably herein. These terms refer to methods of obtaining beneficial or desired results, including but not limited to therapeutic benefits. "Therapeutic benefit" means eradication or improvement of the underlying disorder being treated. Here, the therapeutic benefit is obtained by eradicating or ameliorating one or more physiological symptoms associated with the underlying disorder, such that improvement is observed in the subject, although the subject may still suffer from the underlying disorder.

As used in the present disclosure,"preventing" and "prophylacting" are used interchangeably, i.e., to refer to a method of obtaining a beneficial or desired result, including but not limited to a prophylactic benefit. To obtain a "prophylactic benefit", a conjugate, RNAi agent, or composition may be administered to a subject at the risk of a particular disease, or to a subject exhibiting one or more physiological symptoms of a disease, even if a diagnosis of the disease may not have been made.

In the present disclosure, the term "administer" generally refers to introducing a pharmaceutical formulation of the present disclosure into the body of a subject by any route of introduction or delivery. Any method known to those skilled in the art for contacting cells, organs or tissues with the drug may be employed. The administration may include, but is not limited to intravenous, intraarterial, intranasal, intraabdominal, intramuscular, subcutaneous, or oral. The daily dose may be divided into one, two or more doses in a suitable form to be administered at one, two or more times during a certain time period.

In the present disclosure, the term "contacting" generally refers to two or more different types of substances contacting together in any order, in any manner, and for any length of time. Contacting can occur *in vivo, ex vivo* or *in vitro*. In some embodiments, it may mean bringing an RNAi agent or composition of the present disclosure into direct contact with a cell or tissue. In other embodiments, the term refers to indirect contact of an RNAi agent or composition of the disclosure with a cell or tissue.

In the present disclosure, the term "subject" generally refers to a human or non-human animal (including mammals) in need of diagnosis, prognosis, amelioration, prevention and/or treatment of a disease, such as humans, non-human primates (apes, gibbons, gorillas, chimpanzees, orangutans, macaques), livestock (dogs and cats), farm animals (horses, cattle, goats, sheep, pigs), and laboratory animals (mice, rats, rabbits, guinea pigs). Human subjects include fetal, neonatal, infant, adolescent and adult subjects. Subjects include animal disease models.

In the present disclosure, the term "modulates gene expression" means that the expression of a gene, or the level of an RNA molecule or equivalent RNA molecule encoding one or more proteins or protein subunits, is up-regulated or down-regulated such that the expression, level or activity is greater or less than that observed in the absence of the modulator. For example, the term "modulate" may mean "inhibit," although use of the word "modulate" is not limited to this definition.

In addition to any conventional excipients, the extent to which siRNAs of the present disclosure are incompatible, e.g., the produced any undesirable biological effects or interact in a deleterious manner with any other component of a pharmaceutically acceptable composition, and their use are also contemplated by the present disclosure.

**Double-stranded oligonucleotides targeting INHBE gene**

In a first aspect of the present disclosure, the present disclosure provides a double-stranded oligonucleotide targeting INHBE gene capable of inhibiting INHBE gene expression in a mammal, including a human, monkey, rat, or mouse. The double-stranded oligonucleotide can inhibit the expression of INHBE gene in cells *in vitro* and also inhibit the expression of INHBE gene in organisms *in vivo.*

Specifically, the present disclosure provides a double-stranded oligonucleotide targeting the INHBE gene, wherein the double-stranded oligonucleotide comprises a sense strand and an antisense strand, wherein the antisense strand is complementary or substantially complementary to the sense strand; said substantially complementary means that the sense strand and the antisense strand have a mismatch of no more than 3 nucleotides in the duplex region.

Wherein the sense strand comprises a nucleotide sequence that is identical or substantially identical to at least 15 contiguous nucleotides in the sequence of SEQ ID NO: 309; and said substantially identical means that there are no more than 3 nucleotide differences between the sense strand and at least 15 contiguous nucleotides in the sequence of SEQ ID NO: 309.

In some optional embodiments of the present disclosure, the sense strand comprises a nucleotide sequence having no more than 2 nucleotide differences from at least 15 contiguous nucleotides in the sequence of SEQ ID NO: 309, preferably no more than 1 nucleotide difference.

In some optional embodiments of the present disclosure, the antisense strand is complementary or substantially complementary to at least 15, 16, 17, 18, 19, 20, 21, 22, or 23 contiguous nucleotides of the nucleotide sequence of SEQ ID NO: 309, said substantially complementary means having a mismatch of no more than 3 nucleotides in the complementary region.

In some optional embodiments of the present disclosure, the double-stranded oligonucleotide comprises a sense strand and an antisense strand, wherein the antisense strand comprises at least 15 contiguous nucleotides of any one of the sequences shown in SEQ ID NO:155 - SEQ ID NO: 308 in Table 1, or a nucleotide sequence having no more than 3 nucleotide differences from the at least 15 contiguous nucleotides.

The sense strand comprises a nucleotide sequence that is at least partially reverse complementary or substantially complementary to the antisense strand to form a duplex region; said substantially complementary means that the sense strand and the antisense strand have a mismatch of no more than 3 nucleotides in the duplex region.

In some optional embodiments of the present disclosure, the antisense strand comprises at least 15, at least 16, at least 17, at least 18, at least 19, at least 20, at least 21 contiguous nucleotides of any one of the sequences shown in SEQ ID NO: 155 - SEQ ID NO: 308 in Table 1, or a nucleotide sequence having no more than 3 nucleotide differences from the contiguous nucleotides.

In some optional embodiments of the present disclosure, the antisense strand comprises at least 17, at least 18, at least 19, at least 20, at least 21 contiguous nucleotides of any one of the sequences shown in SEQ ID NO: 155 - SEQ ID NO: 308 in Table 1, or a nucleotide sequence having no more than 2 nucleotide differences from the contiguous nucleotides.

In some optional embodiments of the present disclosure, the antisense strand comprises at least 17, at least 18, at least 19, at least 20, at least 21 contiguous nucleotides of any one of the sequences shown in SEQ ID NO: 155 - SEQ ID NO: 308 in Table 1, or a nucleotide sequence having no more than 1 nucleotide difference from the contiguous nucleotides.

In some optional embodiments of the present disclosure, the antisense strand comprises at least 17, at least 18, at least 19, at least 20, at least 21 contiguous nucleotides of any one of the nucleotide sequences shown in SEQ ID NO: 155 - SEQ ID NO: 308 in Table 1.

In some specific embodiments of the present disclosure, the antisense strand is selected from or comprises any one of the nucleotide sequences shown in SEQ ID NO: 155 - SEQ ID NO: 308 in Table 1.

In some optional embodiments of the present disclosure, as numbered in the 5' to 3' direction, positions 2-19 of the antisense strand comprise at least 15 nucleotides of the nucleotides at positions 2-19 of any one of the nucleotide

sequences shown in SEQ ID NO. 155 to SEQ ID NO. 308 in Table 1, or a nucleotide sequence having no more than 3 nucleotide differences from the at least 15 nucleotides.

In some optional embodiments of the present disclosure, the sense strand comprises at least 13, at least 14, at least 15, at least 16, at least 17, at least 18, at least 19 contiguous nucleotides of any one of the sequences shown in SEQ ID NO. 1 to SEQ ID NO. 154 in Table 1, or a nucleotide sequence having no more than 3 nucleotide differences from the contiguous nucleotides.

In some optional embodiments of the present disclosure, the sense strand comprises at least 13, at least 14, at least 15, at least 16, at least 17, at least 18, at least 19 contiguous nucleotides of any one of the sequences shown in SEQ ID NO. 1 to SEQ ID NO. 154 in Table 1, or a nucleotide sequence having no more than 2 nucleotide differences from the contiguous nucleotides.

In some optional embodiments of the present disclosure, the sense strand comprises at least 13, at least 14, at least 15, at least 16, at least 17, at least 18, at least 19 contiguous nucleotides of any one of the sequences shown in SEQ ID NO. 1 to SEQ ID NO. 154 in Table 1, or a nucleotide sequence having no more than 1 nucleotide difference from the contiguous nucleotides.

In some optional embodiments of the present disclosure, the sense strand comprises at least 15, at least 16, at least 17, at least 18, at least 19 contiguous nucleotides of any one of the nucleotide sequences shown in SEQ ID NO. 1 to SEQ ID NO. 154 in Table 1.

In some specific embodiments of the present disclosure, the sense strand is selected from or comprises any one of the nucleotide sequences shown in SEQ ID NO. 1 to SEQ ID NO. 154 in Table 1.

In some optional embodiments of the present disclosure, the double-stranded oligonucleotide comprises one or more of the duplex groups shown in Table 1 with the following serial numbers: RN008015, RN008125, RN008128, RN008142, RN008145, RN008148.

In some optional embodiments of the present disclosure, each nucleotide in the double-stranded oligonucleotide is independently selected from unmodified or modified nucleotides.

Wherein the nucleotide has the structural formula

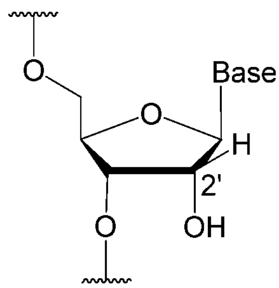

,

wherein Base represents a nucleobase, and the nucleobase on each nucleotide is independently selected from uracil (U), thymine (T), cytosine (C), adenine (A), or guanine (G).

In some optional embodiments of the present disclosure, substantially all nucleotides of the sense strand or the antisense strand are selected from modified nucleotides. Wherein "substantially all nucleotides of the sense strand are selected from modified nucleotides" means that most, but not all, of the nucleotides in the sense strand are modified nucleotides, and it may comprise no more than 5, 4, 3, 2, or 1 unmodified nucleotide(s). "substantially all nucleotides of the antisense strand are selected from modified nucleotides " means that most, but not all, of the nucleotides in the antisense strand are modified nucleotides, and it may comprise no more than 5, 4, 3, 2, or 1 unmodified nucleotide(s).

In some specific embodiments of the present disclosure, all nucleotides of the sense strand or the antisense strand are selected from modified nucleotides.

In some optional embodiments of the present disclosure, the modified nucleotides are each independently selected from 2'-halo, 2'-deoxy, 2'-O-$(CH_2)_n$-$R_1$ modified nucleotides, or nucleotide analogs; wherein the nucleotide analogs are selected from one or more of peptide nucleic acid (PNA), Morpholino (MNA), bridged nucleic acid (BNA), locked nucleic acid (LNA), glycol nucleic acid (GNA), threose nucleic acid (TNA) and unlocked nucleic acid (UNA).

n is selected from 0, 1, or 2; $R_1$ is selected from optionally substituted $C_{1-6}$ alkyl, optionally substituted $C_{1-6}$ alkoxy or -Si$(R_{1a})_3$; each $R_{1a}$ is independently selected from optionally substituted $C_{1-6}$ alkyl or optionally substituted $C_{1-6}$ alkoxy.

In the present disclosure, a 2'-halogen modified nucleotide refers to a nucleotide in which the 2'-hydroxyl group is substituted with a halogen atom. Illustratively, the 2'-fluoro modified nucleotide has the structural formula

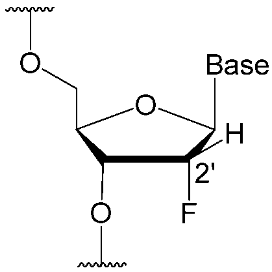

.

In the present disclosure, a 2'-deoxy modified nucleotide refers to a nucleotide in which the 2'-hydroxyl group is substituted with a hydrogen atom. Its structural formula is

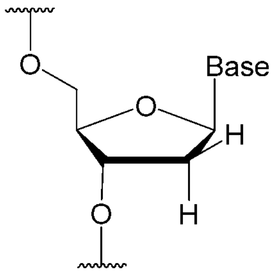

.

In the present disclosure, a 2'-O-$(CH_2)_n$-$R_1$ modified nucleotide refers to a nucleotide in which the hydrogen atom of the 2'-hydroxyl group is substituted with -$(CH_2)_n$-$R_1$. The structural formula of the 2'-O-$(CH_2)_n$-$R_1$ modified nucleotide is

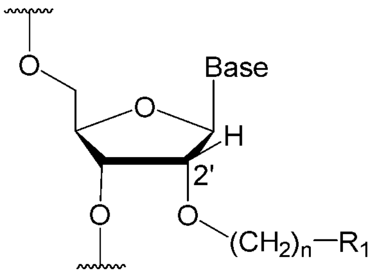

.

For example: when n is selected from 0, 2'-O-$(CH_2)_n$-$R_1$ may be selected from 2'-O-methyl (also referred to as 2'-methoxy), 2'-O-methoxymethyl, 2'-O-TBDMS, 2'-O-TIPS, or 2'-O-TOM. When n is selected from 1, 2'-O-$(CH_2)_n$-$R_1$ may be selected from 2'-O-$CH_2$-O-$CH_2$-$CH_3$ (2'-O-ethoxymethyl) or 2'-O-$CH_2$-O-$CH_2$-$CF_3$ (also referred to as 2'-O-(2,2,2-trifluoroethoxymethyl)). When n is selected from 2, 2'-O-$(CH_2)_n$-$R_1$ may be selected from 2'-O-$CH_2$-$CH_2$-O-$CH_3$ (also referred to as 2'-O-methoxyethyl, 2'-O-MOE). Wherein, the structural formula of TBDMS is

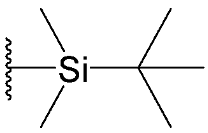

,

the structural formula of TIPS is

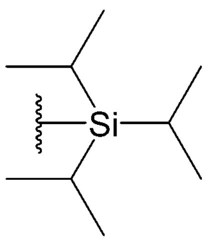

,

and the structural formula of TOM is

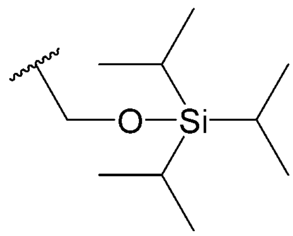

.

In some optional embodiments of the present disclosure, 2'-O-$(CH_2)_n$-$R_1$ is selected from 2'-O-$CH_3$, 2'-O-$CH_2$-O-$CH_3$, 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$ or 2'-O-$CH_2$-$CH_2$-O-$CH_3$.

In some optional embodiments of the present disclosure, the sense strand or the antisense strand comprises a 3' overhang having at least 1 nucleotide.

In some optional embodiments of the present disclosure, the antisense strand comprises a 3' overhang having at least 1 nucleotide.

In some optional embodiments of the present disclosure, the sense strand or the antisense strand comprises a 3 'overhang having at least 2 nucleotides.

In some optional embodiments of the present disclosure, the antisense strand comprises a 3' overhang having at least 2 nucleotides.

In some specific embodiments of the present disclosure, the antisense strand comprises a 3' overhang having 2 nucleotides.

In some optional embodiments of the present disclosure, the sense strand and/or the antisense strand independently comprise one or more phosphorothioate linkages.

In some specific embodiments of the present disclosure, the sense strand comprises two contiguous phosphorothioate linkages between terminal nucleotides at the 5'-end.

In some specific embodiments of the present disclosure, the antisense strand comprises two contiguous phosphorothioate linkages between terminal nucleotides respectively at the 3' and 5'-end.

In some optional embodiments of the present disclosure, all nucleotides of the sense strand and all nucleotides of the antisense strand are selected from modified nucleotides; wherein the duplex region formed by the sense strand and the antisense strand is represented by the following formula (I):

SS: 5'- (N)a' - (X)p' - (N)b' - (X)q' - (N)c' - (X)r' - (N) d' -3'

AS: 3' - (N)a - (X)p - (N)b - (X)q - (N)c -5'  (I),

wherein SS represents sense strand, AS represents antisense strand;
each N is independently selected from 2'-fluoro modified nucleotides, 2'-O-methyl modified nucleotides or 2'-deoxy modified nucleotides;
each X is independently selected from 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$, 2'-O-$CH_2$-$CH_2$-O-$CH_3$;
the a, a', p, p', b, b', q, q', c, c', r', d' each independently represents the number of nucleotides, wherein: a' is selected from an integer of 3-8; p' is selected from an integer of 0-3; b' is selected from an integer of 4-13; q' is selected from an integer of 0-4; c' is selected from an integer of 3-9; r' is selected from an integer of 0-3; d' is selected from an integer of 0-9; a is selected from an integer of 4-7; p is selected from an integer of 0-1;b is selected from an integer of 4-8; q is selected from an integer of 0-4; c is selected from an integer of 6-10; and p', q', r', p, q are not simultaneously 0, and $0 \le q'+r' \le 4$.

In some optional embodiments of the present disclosure, a' is selected from an integer of 3-8, p' is selected from 0 or 1, b' is selected from an integer of 4-13, q' is selected from 0 or 1, c' is selected from an integer of 3-9; r' is selected from 0 or 1, d' is selected from an integer of 1-8, a is selected from an integer of 4-7, p is selected from 1, b is selected from an integer of 4-8, q is selected from 0 or 1, c is selected from an integer of 6-10.

In some optional embodiments of the present disclosure, each N is independently selected from 2'-O-methyl modified nucleotides, 2'-fluoro modified nucleotides.

In some optional embodiments of the present disclosure, each X is independently selected from 2'-O-methyl modified nucleotides, 2'-O-MOE (methoxyethyl) modified nucleotides, 2'-O-TBDMS modified nucleotides, 2'-O-TIPS

modified nucleotides, 2'-O-TOM modified nucleotides, 2'-O-$CH_2$-O-$CH_2$-$CH_3$ modified nucleotides, 2'-O-$CH_2$-O-$CH_2$-$CF_3$ modified nucleotides.

Preferably, each X is independently selected from 2'-O-methyl modified nucleotides, 2'-O-MOE modified nucleotides, 2'-O-$CH_2$-O-$CH_2$-$CH_3$ modified nucleotides, 2'-O-$CH_2$-O-$CH_2$-$CF_3$ modified nucleotides.

In some optional embodiments of the present disclosure, the duplex region comprises at least one 2'-O-methoxyethyl modified nucleotide or 2'-O-ethoxymethyl modified nucleotide.

In some optional embodiments of the present disclosure, the duplex region comprises at least one 2'-O-methoxyethyl modified nucleotide.

In some specific embodiments of the present disclosure, the duplex region comprises one 2'-O-methoxyethyl modified nucleotide.

In some optional embodiments of the present disclosure, in the direction from the 5'-end to the 3'-end, at least four of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides.

In some optional embodiments of the present disclosure, in the direction from the 5'-end to the 3'-end, at least five of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides.

In some optional embodiments of the present disclosure, in the direction from the 5'-end to the 3'-end, arbitrarily five of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides.

In some optional embodiments of the present disclosure, in the direction from the 5'-end to the 3'-end, the nucleotides at positions 2, 6, 9, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides.

In some optional embodiments of the present disclosure, in the direction from the 5'-end to the 3'-end, the nucleotides at positions 2, 6, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides.

In some optional embodiments of the present disclosure, the antisense strand comprises at least one 2'-O-methoxyethyl modified nucleotide or 2'-O-ethoxymethyl modified nucleotide.

In some optional embodiments of the present disclosure, the antisense strand comprises at least one 2'-O-methoxyethyl modified nucleotide.

In some specific embodiments of the present disclosure, the antisense strand comprises one 2'-O-methoxyethyl modified nucleotide.

In some optional embodiments of the present disclosure, in the direction from the 5'-end to the 3'-end, the nucleotide at position 15 of the antisense strand is selected from a 2'-O-methoxyethyl modified nucleotide; at least four of the nucleotides at positions 2, 6, 9, 12, 14, and 16 are selected from 2'-fluoro modified nucleotides; and the nucleotides at the remaining positions are selected from 2'-O-methyl modified nucleotides.

In some optional embodiments of the present disclosure, in the direction from the 5'-end to the 3'-end, the nucleotide at position 15 of the antisense strand is selected from a 2'-O-methoxyethyl modified nucleotide; at least five of the nucleotides at positions 2, 6, 9, 12, 14, and 16 are selected from 2'-fluoro modified nucleotides; and the nucleotides at the remaining positions are selected from 2'-O-methyl modified nucleotides.

In some specific embodiments of the present disclosure, in the direction from the 5'-end to the 3'-end, the nucleotide at position 15 of the antisense strand is selected from a 2'-O-methoxyethyl modified nucleotide; arbitrarily five of the nucleotides at positions 2, 6, 9, 12, 14, and 16 are selected from 2'-fluoro modified nucleotides; and the nucleotides at the remaining positions are selected from 2'-O-methyl modified nucleotides.

In some specific embodiments of the present disclosure, in the direction from the 5'-end to the 3'-end, the nucleotide at position 15 of the antisense strand is selected from a 2'-O-methoxyethyl modified nucleotide; the nucleotides at positions 2, 6, 9, 14, and 16 are selected from 2'-fluoro modified nucleotides; and the nucleotides at the remaining positions are selected from 2'-O-methyl modified nucleotides.

In some optional embodiments of the present disclosure, in the direction from the 5'-end to the 3'-end, the nucleotide at position 15 of the antisense strand is selected from a 2'-O-methoxyethyl modified nucleotide; the nucleotides at positions 2, 6, 12, 14, and 16 are selected from 2'-fluoro modified nucleotides; and the nucleotides at the remaining positions are selected from 2'-O-methyl modified nucleotides.

In some optional embodiments of the present disclosure, in the direction from the 5'-end to the 3'-end, at least three of the nucleotides at positions 7-10 of the sense strand are selected from 2'-fluoro modified nucleotides, and the

nucleotides at the remaining positions are selected from 2'-O-methyl modified nucleotides.

In some specific embodiments of the present disclosure, in the direction from the 5'-end to the 3'-end, the nucleotides at positions 7-10 of the sense strand are selected from 2'-fluoro modified nucleotides, and the nucleotides at the remaining positions are selected from 2'-O-methyl modified nucleotides.

In some optional embodiments of the present disclosure, the antisense strand comprises at least 15, at least 16, at least 17, at least 18, at least 19, at least 20, at least 21 contiguous nucleotides of any one of the modified antisense strand nucleotide sequences shown in Table 2, or a nucleotide sequence having no more than 3 nucleotide differences from the contiguous nucleotides.

In some optional embodiments of the present disclosure, the antisense strand comprises at least 15, at least 16, at least 17, at least 18, at least 19, at least 20, at least 21 contiguous nucleotides of any one of the modified antisense strand nucleotide sequences shown in Table 2, or a nucleotide sequence having no more than 2 nucleotide differences from the contiguous nucleotides.

In some optional embodiments of the present disclosure, the antisense strand comprises at least 15, at least 16, at least 17, at least 18, at least 19, at least 20, at least 21 contiguous nucleotides of any one of the modified antisense strand nucleotide sequences shown in Table 2, or a nucleotide sequence having no more than 1 nucleotide difference from the contiguous nucleotides.

In some optional embodiments of the present disclosure, the antisense strand comprises at least 17, at least 18, at least 19, at least 20, at least 21 contiguous nucleotides of any one of the modified antisense strand nucleotide sequences shown in Table 2.

In some specific embodiments of the present disclosure, the antisense strand is selected from or comprises any one of the modified antisense strand nucleotide sequences shown in Table 2.

In some optional embodiments of the present disclosure, the sense strand comprises at least 15, at least 16, at least 17, at least 18, at least 19 contiguous nucleotides of any one of the modified sense strand nucleotide sequences shown in Table 2, or a nucleotide sequence differing from the contiguous nucleotides by 1, 2, or 3 nucleotides.

In some specific embodiments of the present disclosure, the sense strand of the double-stranded oligonucleotide is selected from or comprises any of the modified sense strand nucleotide sequences shown in Table 2.

In some specific embodiments of the present disclosure, the sense strand of the double-stranded oligonucleotide is selected from or comprises any one of the modified sense strand nucleotide sequences shown in Table 2; and the antisense strand is selected from or comprises any one of the modified antisense strand nucleotide sequences shown in Table 2.

In some optional embodiments of the present disclosure, the double-stranded oligonucleotide is selected from or comprises one or more of the modified duplex groups shown in Table 2 with the following serial numbers: RX008157, RX008174, RX008177, RX008180, RX008182, RX008185.

In some specific embodiments of the present disclosure, the double-stranded oligonucleotide is selected from siRNA.

## Double-stranded oligonucleotide conjugates

In a second aspect of the present disclosure, the present disclosure provides a double-stranded oligonucleotide conjugate, wherein the conjugate comprising the double-stranded oligonucleotide according to the first aspect and one or more ligands capable of binding to a cellular receptor.

In some optional embodiments of the present disclosure, the ligand is conjugated to the sense strand and/or the antisense strand.

In some optional embodiments of the present disclosure, the ligand is conjugated to the 3'-end and/or 5'-end of the sense strand.

In some specific embodiments of the present disclosure, the ligand is conjugated to the 3'-end of the sense strand.

In some specific embodiments of the present disclosure, the cellular receptor is selected from asialoglycoprotein receptor.

In some specific embodiments of the present disclosure, the ligand is selected from clusters of galactose. The clusters of galactose comprise molecules having 2 to 4 terminal galactose derivatives. As used in the present disclosure, the galactose derivatives include galactose and/or galactose derivatives having an affinity to the asialoglycoprotein receptor which is equal to or greater than that of galactose.

In some specific embodiments of the present disclosure, the clusters of galactose comprise molecules having 2 to 4 terminal N-acetylgalactosamine (GalNAc) groups.

In some optional embodiments of the present disclosure, the ligand is selected from the structure of formula (101), or an isomer thereof, or a pharmaceutically acceptable salt thereof:

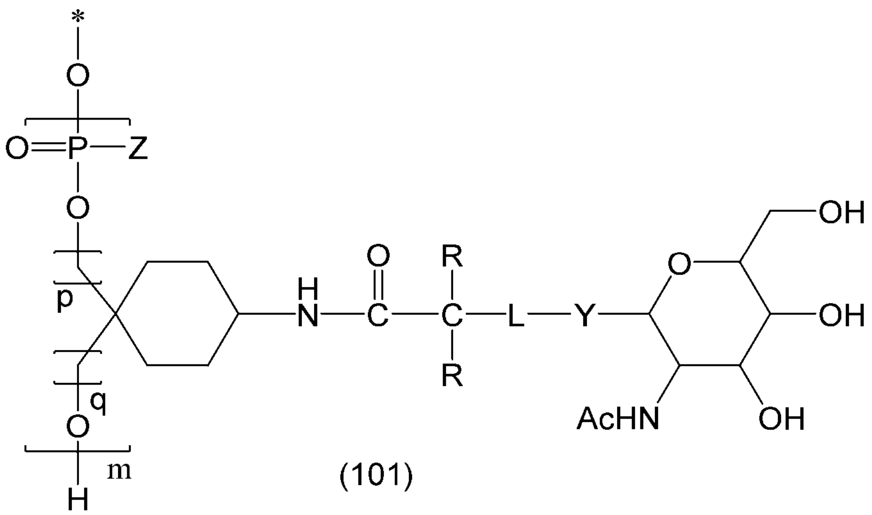

(101)

wherein, * represents the conjugation site between the ligand and the sense strand or the antisense strand;
m is selected from 1, 2, 3, or 4;
each Z is independently selected from hydroxyl or mercapto;
each p is independently selected from 1, 2, or 3;
each q is independently selected from 1, 2, or 3;
each R is independently selected from H, optionally substituted C1-C6 alkyl, or optionally substituted C1-C6 alkoxy;
each L is independently selected from optionally substituted C2-C20 alkylene or

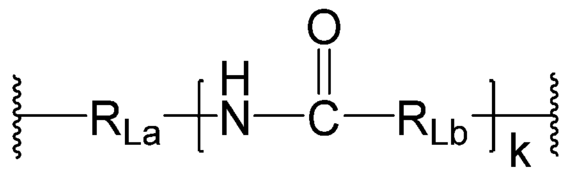

;

$R_{La}$ and $R_{Lb}$ are independently selected from optionally substituted C1-C10 alkylene, and k is selected from 1, 2, 3, 4, or 5;
each Y is independently selected from O, S, or NH.

In some optional embodiments of the present disclosure, m is selected from 1, 2, or 3.
In some specific embodiments of the present disclosure, m is selected from 3.
In some specific embodiments of the present disclosure, Z is selected from hydroxyl.
In some specific embodiments of the present disclosure, p is selected from 1.
In some specific embodiments of the present disclosure, q is selected from 1.
In some specific embodiments of the present disclosure, R is selected from H.
In some optional embodiments of the present disclosure, each L is independently selected from C1-C10 alkylene or

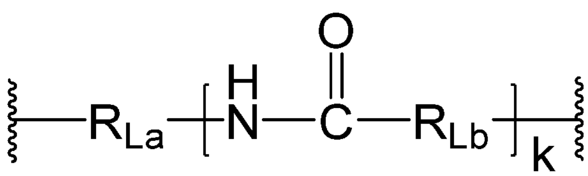

;

wherein, $R_{La}$ and $R_{Lb}$ are independently selected from C1-C5 alkylene, and k is 1, 2, or 3.
In some specific embodiments of the present disclosure, k is selected from 1.
In some optional embodiments of the present disclosure, each L is independently selected from

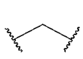, 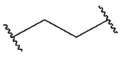, 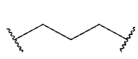, 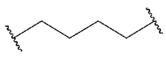, 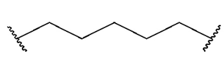, 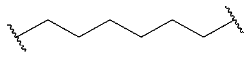,

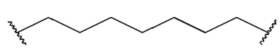, 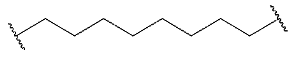, 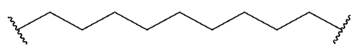,

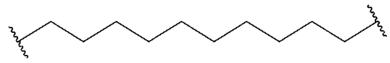, 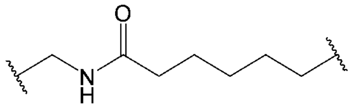, 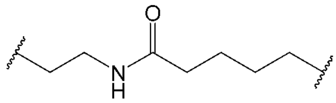,

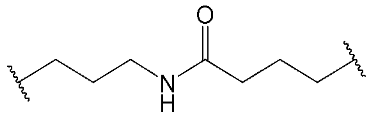, 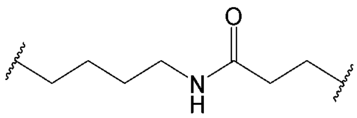 or 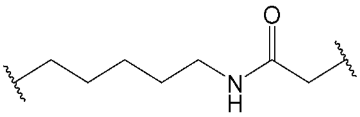.

In some specific embodiments of the present disclosure, Y is selected from O.

In some optional embodiments of the present disclosure, the ligand comprises at least one of the following structure, or an isomer thereof, or a pharmaceutically acceptable salt thereof:

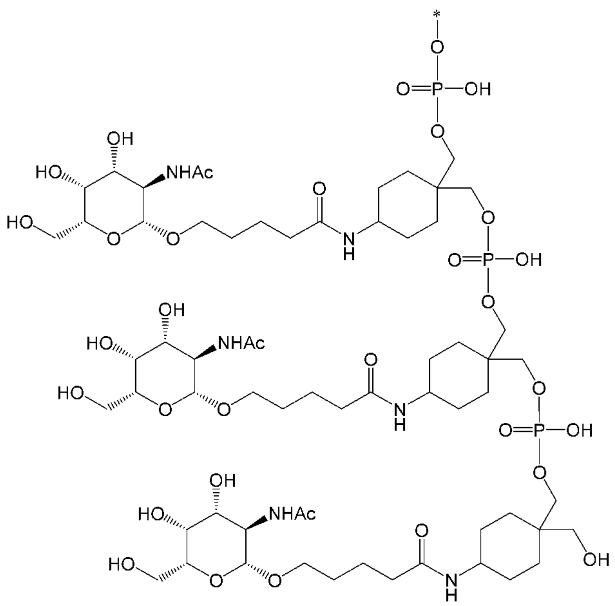

or

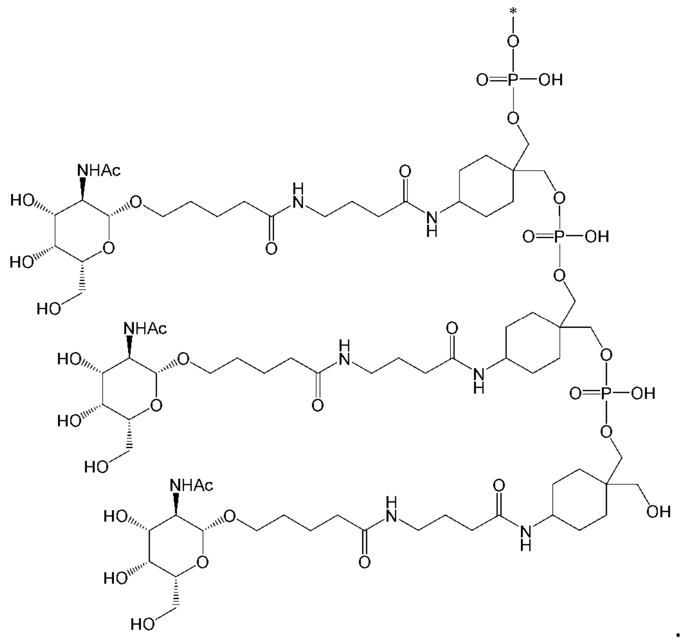

In some specific embodiments of the present disclosure, the ligand is selected from the structure of the following, or an isomer thereof, or a pharmaceutically acceptable salt thereof:

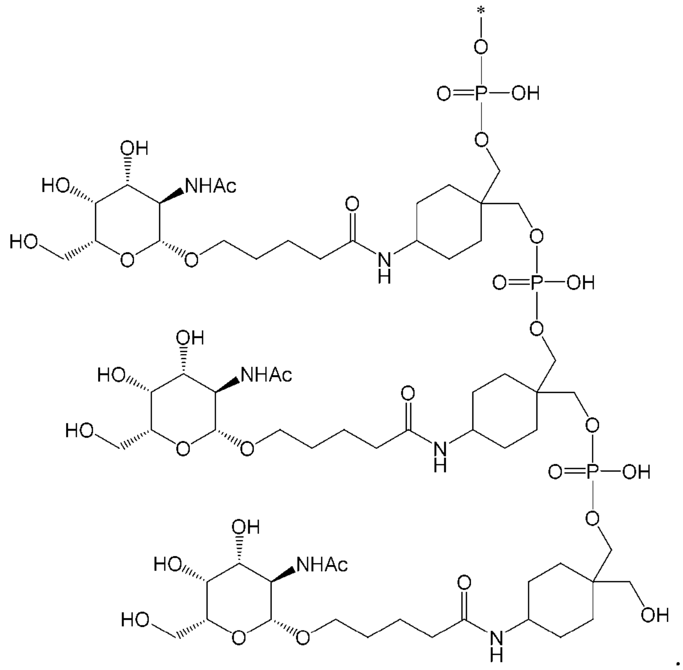

In some optional embodiments of the present disclosure, the double-stranded oligonucleotide conjugate is selected from one or more of the conjugates shown in Table 4 with the following serial numbers: RZ08040, RZ08041, RZ08042, RZ08043, RZ08044, RZ08045.

In some specific embodiments of the present disclosure, the ligand is selected from the structure shown in formula (201):

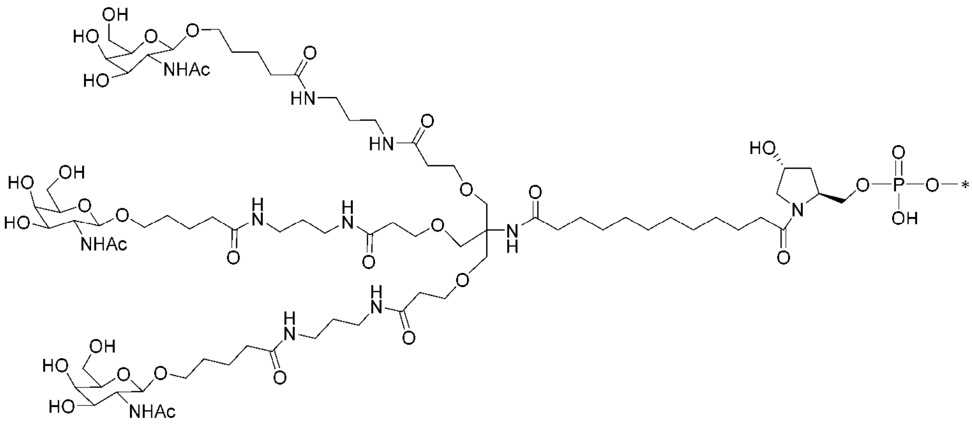

(201)

wherein, * represents the conjugation site between the ligand and the sense strand or the antisense strand.

**Composition**

In a third aspect of the present disclosure, the present disclosure provides a composition, wherein the composition comprising any one of:

(I) the double-stranded oligonucleotide according to the first aspect; and/or
(II) the conjugate according to the second aspect.

Use **for treatment of** diseases

In a fourth aspect of the present disclosure, the present disclosure provides a use of any one of:

(I) the double-stranded oligonucleotide according to the first aspect; and/or
(II) the conjugate according to the second aspect; and/or
(III) the composition according to the third aspect,

in the manufacture of a medicament for the prevention and/or treatment of diseases or disorders mediated by INHBE gene.

In some optional embodiments of the present disclosure, the disease or disorder comprises, but is not limited to, having or being at the risk of developing metabolic disorder, type 2 diabetes, obesity, elevated triglyceride levels, lipodystrophy, liver inflammation, fatty liver disease, hypercholesterolemia, elevated liver enzymes, non-alcoholic steatohepatitis (NASH), cardiovascular disease, cardiomyopathy, hypertension, and/or heart failure.

**Pharmaceutical composition**

In a fifth aspect of the present disclosure, the present disclosure provides a pharmaceutical composition, wherein the pharmaceutical composition comprising any one of:

(I) the double-stranded oligonucleotide according to the first aspect; and/or
(II) the conjugate according to the second aspect; and/or
(III) the composition according to the third aspect,

and a pharmaceutically acceptable excipient or auxiliary agent.

**Method for reducing the expression or activity of INHBE gene *in vitro***

In a sixth aspect of the present disclosure, the present disclosure provides a method for reducing the expression or activity of INHBE gene, comprising contacting a cell with any one of:

(I) the double-stranded oligonucleotide according to the first aspect; and/or

(II) the conjugate according to the second aspect; and/or
(III) the composition according to the third aspect; and/or
(IV) the pharmaceutical composition according to the fifth aspect.

## Method for treating diseases

In a seventh aspect of the present disclosure, the present disclosure provides a method for preventing and/or treating a disease or disorder mediated by INHBE gene, comprising administering to a subject pharmaceutically acceptable amount of any one of:

(I) the double-stranded oligonucleotide according to the first aspect; and/or
(II) the conjugate according to the second aspect; and/or
(III) the composition according to the third aspect; and/or
(IV) the pharmaceutical composition according to the fifth aspect.

The mRNA sequence encoding INHBE gene (SEQ ID NO: 309, NM_031479.5) related to the present invention is as follows:

1 agtagccaga catgagctgt gagggtcaag cacagctatc catcagatga tctactttca
61 gccttcctga gtcccagaca atagaagaca ggtggetgta cccttggcca agggtaggtg
121 tggcagtggt gtctgctgtc actgtgccct cattggcccc cagcaatcag actcaacaga
181 cggagcaact gccatccgag gctcctgaac cagggccatt caccaggagc atgcggctcc
241 ctgatgtcca gctctggctg gtgctgctgt gggcactggt gcgagcacag gggacagggt
301 ctgtgtgtcc ctcctgtggg ggctccaaac tggcacccca agcagaacga gctctggtgc
361 tggagctagc caagcagcaa atcctggatg ggttgcacct gaccagtcgt cccagaataa
421 ctcatcctcc accccaggca gcgctgacca gagccctccg gagactacag ccagggagtg
481 tggctccagg gaatggggag gaggtcatca gctttgctac tgtcacagac tccacttcag
541 cctacagctc cctgctcact ttcacctgt ccactcctcg gtcccaccac ctgtaccatg
601 cccgcctgtg gctgcacgtg ctccccaccc ttcctggcac tctttgcttg aggatcttcc
661 gatggggacc aaggaggagg cgccaagggt cccgcactct cctggctgag caccacatca
721 ccaacctggg ctggcatacc ttaactctgc cctctagtgg cttgaggggt gagaagtctg
781 gtgtcctgaa actgcaacta gactgcagac ccctagaagg caacagcaca gttactggac
841 aaccgaggcg gctcttggac acagcaggac accagcagcc cttcctagag cttaagatcc
901 gagccaatga gcctggagca ggccgggcca ggaggaggac ccccacctgt gagcctgcga
961 cccccttatg ttgcaggcga gaccattacg tagacttcca ggaactggga tggcgggact
1021 ggatactgca gcccgagggg taccagctga attactgcag tgggcagtgc cctccccacc
1081 tggctggcag cccaggcatt gctgcctctt tccattctgc cgtcttcagc ctcctcaaag
1141 ccaacaatcc ttggcctgcc agtacctcct gttgtgtccc tactgcccga aggcccctct
1201 ctctcctcta cctggatcat aatggcaatg tggtcaagac ggatgtgcca gatatggtgg
1261 tggaggcctg tggctgcagc tagcaagagg acctggggct ttggagtgaa gagaccaaga
1321 tgaagtttcc caggcacagg gcatctgtga ctggaggcat cagattcctg atccacaccc
1381 caacccaaca accacctggc aatatgactc acttgacccc tatgggaccc aaatgggcac
1441 tttcttgtct gagactctgg cttattccag gttggetgat gtgttgggag atgggtaaag
1501 cgtttcttct aaaggggtct acccagaaag catgatttcc tgccctaagt cctgtgagaa
1561 gatgtcaggg actagggagg gagggaggga aggcagagaa aaattactta gcctctccca
1621 agatgagaaa gtcctcaagt gaggggagga ggaagcagat agatggtcca gcaggcttga
1681 agcagggtaa gcaggctggc ccagggtaag ggctgttgag gtaccttaag ggaaggtcaa
1741 gagggagatg ggcaaggcgc tgagggagga tgcttagggg acccccagaa acaggagtca
1801 ggaaaatgag gcactaagcc taagaagttc cctggttttt cccaggggac aggacccact
1861 gggagacaag catttatact ttctttcttc ttttttattt ttttgagatc gagtctcgct
1921 ctgtcaccag gctggagtgc agtgacacga tcttggctca ctgcaacctc cgtctcctgg
1981 gttcaagtga ttcttctgcc tcagcctccc gagcagctgg gattacagge gcccactaat
2041 ttttgtattc ttagtagaaa cgaggtttca acatgttggc caggatggtc tcaatctctt
2101 gacctcttga tccacccgac ttggcctccc gaagtgatga gattatagge gtgagccacc
2161 gcgcctggct tatactttct taataaaaag gagaaagaaa atcaacaaat gtgagtcata
2221 aagaagggtt agggtgatgg tccagagcaa cagttcttca agtgtactct gtaggcttct
2281 gggaggtccc ttttcagggg tgtccacaaa gtcaaagcta ttttcataat aatactaaca
2341 tgttatttge cttttgaatt ctcattatct taaaattgta ttgtggagtt ttccagaggc

2401 cgtgtgacat gtgattacat catctttctg acatcattgt taatggaatg tgtgettgta

Unless otherwise indicated, the proportions of reagents used in each example of the present disclosure are calculated as volume ratios (v/v).

Unless otherwise indicated, reagents used in each example of the present disclosure were purchased from Beijing Ouhe Technology Co., Ltd., where information of major reagents is shown below.

| Reagent name | Abbreviation | CAS number |
|---|---|---|
| Lithium aluminum hydride ($LiAlH_4$) | - | 16853-85-3 |
| Wet palladium carbon (loading 10% by mass) | Pd/C | - |
| Palladium carbon hydroxide (loading 10% by mass) | $Pd(OH)_2/C$ | - |
| Benzotriazole- N,N,N',N"-tetramethylurea hexafluorophosphate | HBTU | 94790-37-1 |
| 2-(7-Azobenzotriazole)- N,N,N',N'-tetramethyluronium hexafluorophosphate | HATU | 148893-10-1 |
| 4,4'-Dimethoxytrityl chloride/4,4'-Dimethoxytriphenylchloromethane | DMTrCl | 40615-36-9 |
| Bis(diisopropylamino)(2-cyanoethoxy) phosphine | - | 102691-36-1 |
| 4,5-Dicyanoimidazole | DCI | 1122-28-7 |
| 4-Dimethylamino pyridine | DMAP | 1122-58-3 |
| Amino CPG (Aminoalkyl-CPG, Model C3006-1000) | 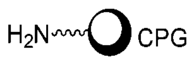 | |
| 4M hydrochloric acid in 1,4-dioxane | - | - |
| Trans-4-(Boc-amino)cyclohexyl carbaldehyde | - | 181308-57-6 |
| N-benzyloxycarbonyl-4-aminobutyric acid | - | 5105-78-2 |
| 5-(((2R, 3R, 4R, 5R, 6R)-3-Acetylamino-4,5-diacetoxy-6-(acetoxymethyl)-2-tetrahydropyranyl)oxy)pentanoic acid | Compound 4 | 1159408-54-4 |

## Preparation of Ligands

### Preparation Example 1. Preparation of Compound CR01008

(1.1) Synthesis of compound CR01008

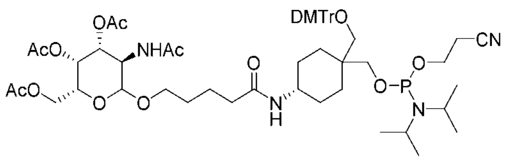

**CR01008**

The synthetic route of compound CR01008 is as follows:

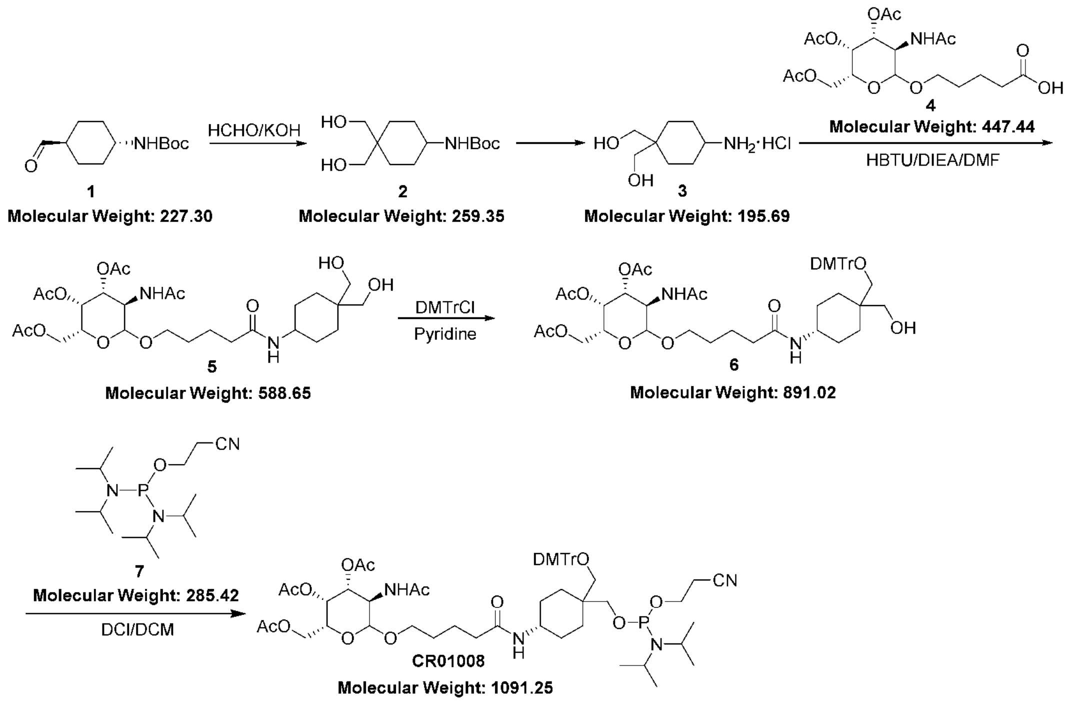


1 Molecular Weight: 227.30
2 Molecular Weight: 259.35
3 Molecular Weight: 195.69
4 Molecular Weight: 447.44
5 Molecular Weight: 588.65
6 Molecular Weight: 891.02
7 Molecular Weight: 285.42
CR01008 Molecular Weight: 1091.25

(1.1.1) Synthesis of Compound 2

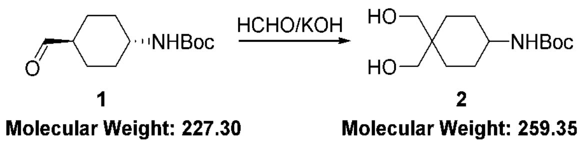


1 Molecular Weight: 227.30
2 Molecular Weight: 259.35

Compound 1 (trans-4-(Boc-amino)cyclohexane carboxaldehyde, 10.0 g, 1.0 eq) and formaldehyde aqueous solution (8.9 g, 37 wt%, 2.4 eq) were dissolved in 33 ml of methanol. Then, 13 ml of a 45.3 wt% KOH aqueous solution was added dropwise. After the addition was completed, the reaction mixture was stirred at 25°C for 30 minutes, then heated to 60°C and refluxed at 60°C for 2 hours. After the reaction was complete, the reaction solution was cooled to room temperature and distilled to dryness under reduced pressure to obtain a crude product as a white solid. A small amount of water was added to the crude product for slurry, followed by filtration to obtain Compound 2 as a white solid (9 g, 78.9% yield). MS-ESI (*m/z*) = 260 $[M + H]^+$.

(1.1.2) Synthesis of Compound 3

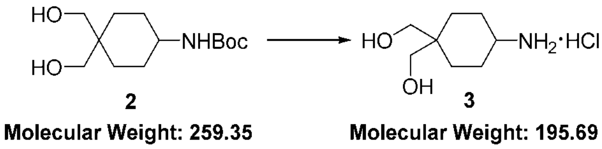

2 Molecular Weight: 259.35
3 Molecular Weight: 195.69

Compound 2 (9g, 1eq) prepared in step (1.1.1) was dissolved in 70 ml of 1,4-dioxane, a solution of hydrogen chloride in 1,4-dioxane (45 ml, 4M) was added, and the reaction was stirred at 25°C for 1 hour. After completion of the reaction, the reaction solution was distilled to dryness under reduced pressure to obtain Compound 3 as a white solid (6.8 g, 100% yield).

(1.1.3) Synthesis of Compound 5

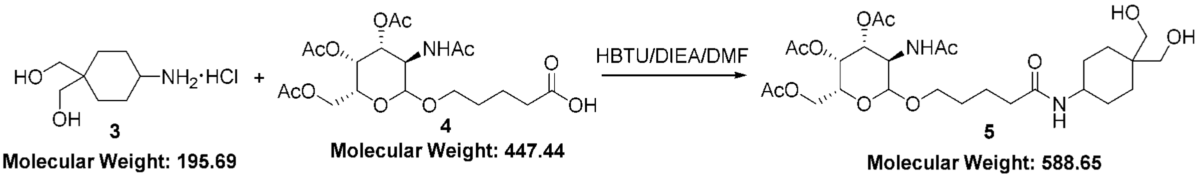


3
Molecular Weight: 195.69
4
Molecular Weight: 447.44
5
Molecular Weight: 588.65

Compound 3 (1.8 g, 2.0 eq) prepared in step (1.1.2), Compound 4 (5-(((2R, 3R, 4R, 5R, 6R)-3-acetylamino-4,5-diacetoxy-6-(acetoxymethyl)-2-tetrahydropyranyl)oxy)pentanoic acid, 2.1 g, 1.0 eq) and DIEA (N,N-diisopropylethylamine, 3.5 g, 6.0 eq) were dissolved in 15 ml of DMF. HBTU (1.9 g, 1.1 eq) was added, and the reaction mixture was stirred under a $N_2$ atmosphere at 25 °C for 3 hours. After the reaction was complete, the reaction solution was distilled to dryness under reduced pressure and undergo reverse-phase purification (22 vol % acetonitrile aqueous solution) to obtain Compound 5 as a white solid (1.78 g, 64.4% yield). MS-ESI (*m*/*z*) = 589[M + H]$^+$.

(1.1.4) Synthesis of Compound 6

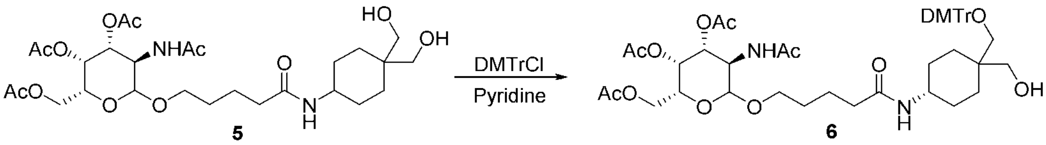


5
Molecular Weight: 588.65
6
Molecular Weight: 891.02

Compound 5 (1.54 g, 1.0 eq) prepared in step (1.1.3) was dissolved in 15 ml pyridine, the reaction system was cooled to 0°C with an ice-water bath, DMTrCl (4,4'-dimethoxytriphenylchloromethane, 1.32 g, 1.5 eq) was added at 0°C, the reaction was carried out at 25°C for 3 hours, and the reaction was quenched by adding 15 ml methanol to the reaction solution. After completion of the reaction, the reaction solution was distilled to dryness under reduced pressure and undergo reverse-phase purification (60 vol % acetonitrile aqueous solution) to obtain compound 6 as a yellow solid (1 g, 42.7% yield). MS-ESI (*m*/*z*) = 891 [M + H]$^+$.

(1.1.5) Synthesis of compound CR01008

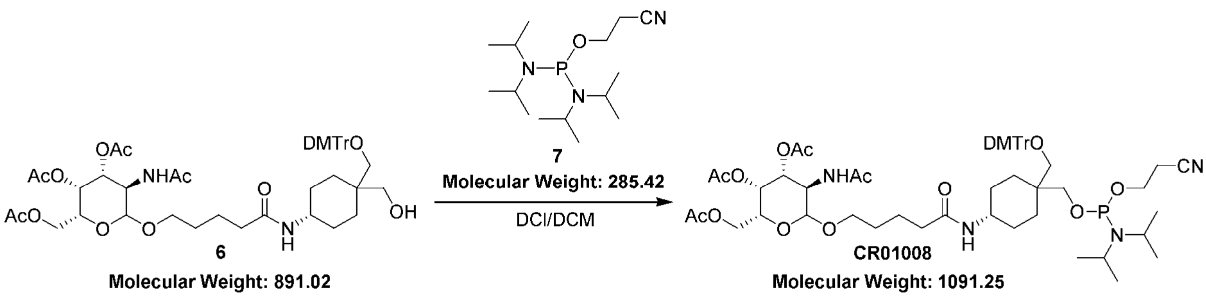


6
Molecular Weight: 891.02
CR01008
Molecular Weight: 1091.25

Compound 6 (1.08 g, 1.0 eq) prepared in step (1.1.4) was dissolved in 20 ml of anhydrous dichloromethane, with DCI (115 mg, 0.8 eq) and Compound 7 (bis (diisopropylamino)(2-cyanoethoxy) phosphine, 732 mg, 2.1 eq) added respectively, purged with nitrogen for three times, and stirred at 25 °C for 2 hours of reaction. After completion of the reaction, 20 ml of a saturated aqueous sodium bicarbonate solution was added to the reaction solution, and the solution was extracted three times (3×20 ml) with 20 ml of dichloromethane. The organic phases were combined, distilled to dryness under reduced pressure, and undergo reverse-phase purification (72 vol % acetonitrile aqueous solution), and then dried under vacuum for 12 hours to obtain compound CR01008 as a white powder (1 g, 76.0% yield). MS-ESI (m/z) = 1091 [M + Na]$^+$.

$^1$H NMR (400 MHz, DMSO-$d_6$) δ 1.05 (d, *J* = 6.7 Hz, 6H).1.14 (d, *J* = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, *J* = 5.9 Hz, 2H), 2.79 (d, *J* = 8.4 Hz, 1H), 2.87 (d, *J* = 8.4 Hz, 1H),3.36 (s, 1H), 3.58 - 3.39 (m, 3H), 3.69 - 3.60 (m, 2H), 3.75 (s, 7H), 3.90 (dt, *J* = 11.2, 8.8 Hz, 1H), 4.05 (s, 3H),4.51 (d, J = 8.4 Hz, 1H), 4.99 (dd, *J* = 11.3, 3.4 Hz, 1H), 5.24 (d, J = 3.4 Hz, 1H), 5.78 (s, 1H), 6.93 - 6.87 (m, 4H),7.35 - 7.21 (m, 7H), 7.44 - 7.37 (m, 2H), 7.66 (d, *J* = 7.8 Hz, 1H), 7.84 (d, *J* = 9.2 Hz, 1H).

**Preparation Example 2. Preparation of Compound CR01008Z**

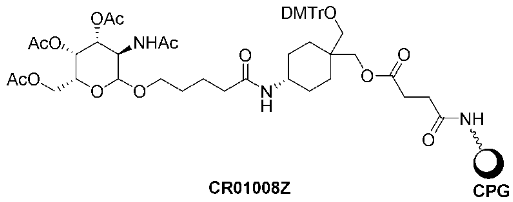

CR01008Z

Compound CR01008Z is obtained by attaching Compound 6 used for the synthesis of compound CR01008 to a solid-phase support CPG.

The synthetic route of compound CR01008Z is as follows:

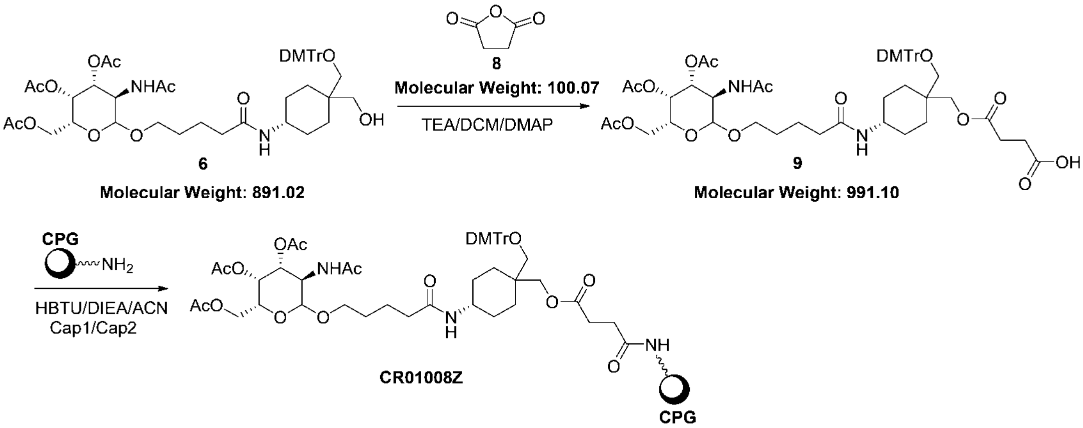


(1.2.1) <u>Synthesis of Compound 9</u>

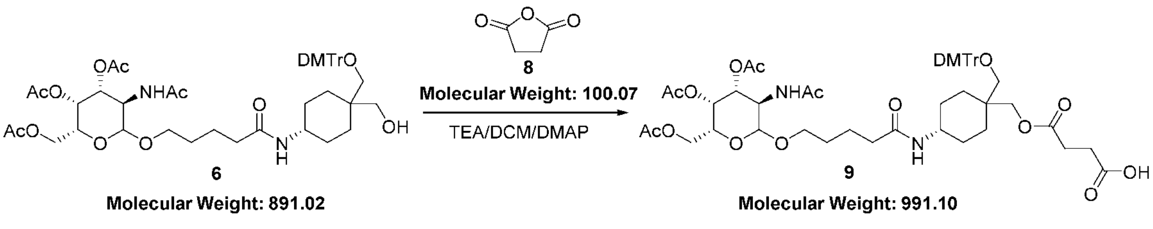


Compound 6 (500 mg) prepared in step (1.1.4) was dissolved in 10 ml of dichloromethane, with Compound 8 (succinic anhydride, 112 mg), DMAP (6.8 mg) and TEA (226.2 mg) added, purged with nitrogen three times, and stirred at 25°C for 16 hours of reaction. Then flash purification was performed to obtain compound 9 (300 mg, 53.6% yield). MS-ESI (m/z) = 1013 [M + Na]$^+$.

(1.2.2) Synthesis of compound CR01008Z

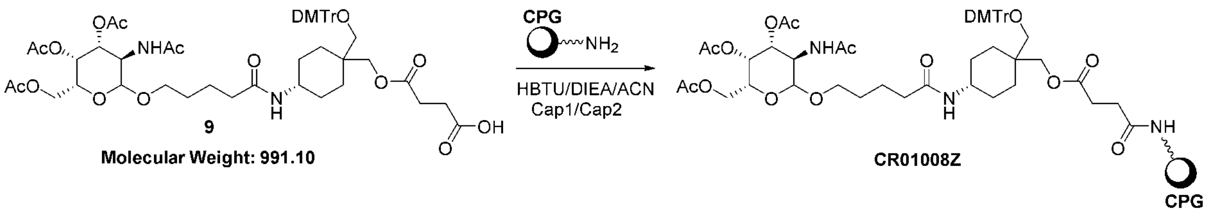


Compound 9 (50 mg) prepared in step (1.2.1), amino CPG (1.25 g, 80μmol/g, 0.1 mmol), HBTU (27 mg) and DIEA (12 mg) were added to a 20 ml sample flask, and shaken for 16 hours of reaction. After completion of the reaction, the reaction solution was filtered to obtain a filter cake, which was washed once with 10 ml of acetonitrile (1×10 ml) and dried in vacuum. The dried cake, DMAP (3 mg), Cap1 (10 ml, 200V) and Cap2 (1 ml, 20V) were added to a 20 ml sample flask and shaken for 6 hours of reaction. After completion of the reaction, the reaction solution was filtered to obtain a filter cake, which was washed once with 10 ml of acetonitrile (1×10 ml) and dried under vacuum to obtain compound CR01008Z (1.03 g, loading: 20-30 μmol/g).

Herein, Cap1 and Cap2 are capping reagents. Cap1 is a 20 vol% N-methylimidazole solution in a pyridine/acetonitrile mixture, and the volume ratio of pyridine to acetonitrile is 3:5; and Cap2 is a 20 vol% acetic anhydride solution in acetonitrile.

## Preparation Example 3. Preparation of Compound CR01013

The synthetic route of compound CR01013 is as follows:

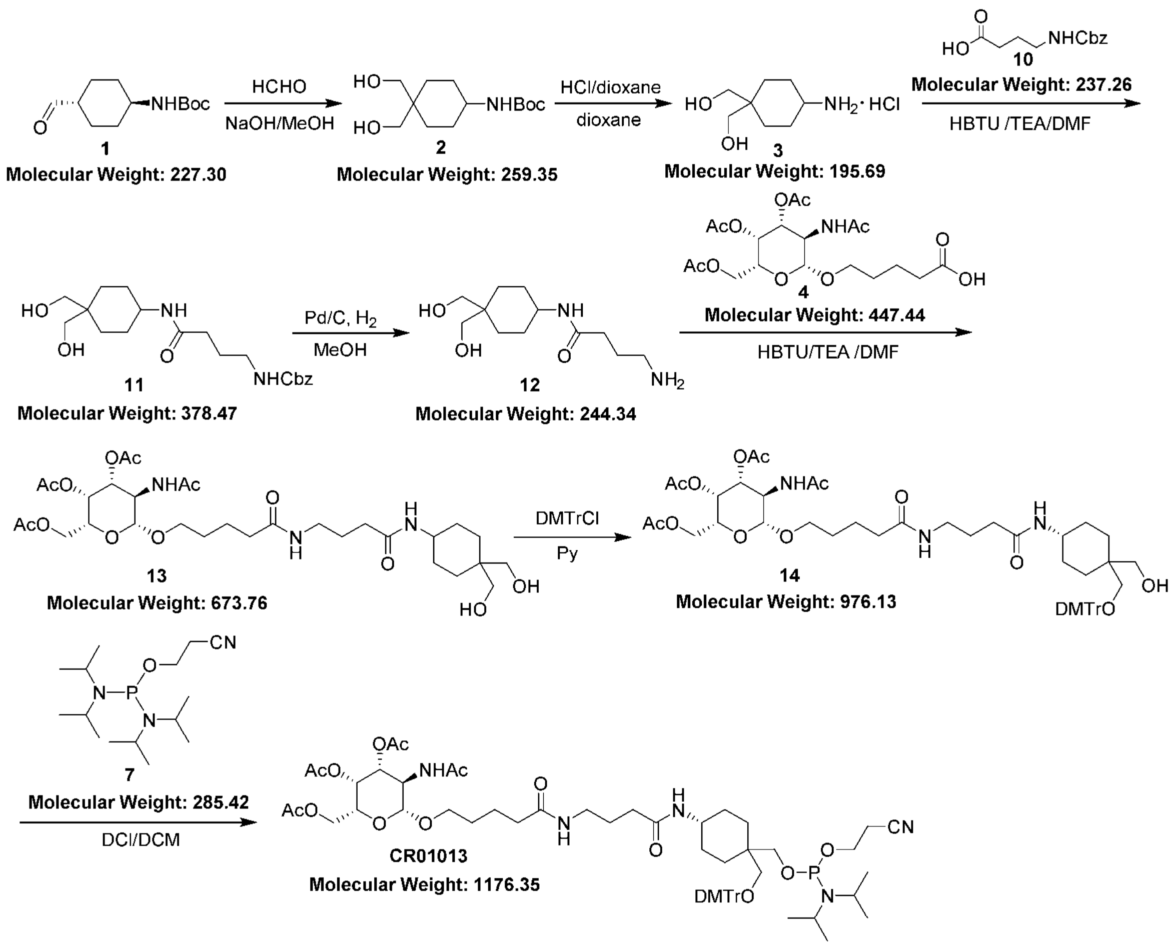


(1.3.1) Synthesis of Compound 2

Compound 1 (trans-4-(Boc-amino)cyclohexane carboxaldehyde, 4.9g) was dissolved in 17 ml of methanol. Then, an aqueous formaldehyde solution (4.21 g, concentration: 37 wt%) and an aqueous sodium hydroxide solution (6.5

ml, concentration: 45.3 wt%) were added dropwise. After the addition was completed, the reaction mixture was heated to 60°C and stirred at 60°C for 2 hours. After the reaction was complete, the reaction solution was cooled to 25°C and distilled to dryness under reduced pressure to obtain a crude product as a white solid. A small amount of water was added to the crude product for slurry, followed by filtration and drying to obtain Compound 2 as a white solid (4.8 g, 85.9% yield). ESI-MS(m/z) = 260.2[M+H]$^+$.

(1.3.2) Synthesis of Compound 3

Compound 2 (4.8g) prepared in step (1.3.1) was dissolved in 25 ml of 1,4-dioxane, with a solution of hydrogen chloride in 1,4-dioxane (25 ml, 4M) added, and stirred at 25°C for 2 hours of reaction. After completion of the reaction, the reaction solution was distilled to dryness under reduced pressure to obtain Compound 3 as a white solid (3.6 g, 99.4% yield).

(1.3.3) Synthesis of Compound 11

Compound 3 (3.6 g) prepared in Step (1.3.2) was dissolved in 36 ml of DMF, with TEA (5.62 g), compound 10 (N-benzyloxycarbonyl-4-aminobutyric acid, 5.28 g) and HBTU (8.43 g) added, and stirred at 25°C for 16 hours of reaction. After completion of the reaction, the reaction solution was added to 200 ml of saturated aqueous sodium bicarbonate solution, extracted three times (3×100 ml) with 100 ml of ethyl acetate, the organic phases were combined, washed once (1×50 ml) with 50 ml of saturated aqueous sodium chloride solution, dried over anhydrous sodium sulfate, distilled to dryness under reduced pressure, and purified by column chromatography on normal phase (eluent: dichloromethane/-methanol =10/1, v/v) to obtain Compound 11 as a white solid (2.3 g, 33.0% yield). ESI MS(m/z) = 379.5[M+H]$^+$.

(1.3.4) Synthesis of Compound 12

Compound 11 (2.3 g) prepared in step (1.3.3) was dissolved in 23 ml of methanol, with wet palladium carbon (230 mg, loading 10 mass %) added, purged with hydrogen for three times, and stirred at 25°C for 16 hours of reaction in a hydrogen atmosphere (15psi). After completion of the reaction, the reaction solution was filtered to obtain a filtrate, and the filtrate was distilled to dryness under reduced pressure to obtain compound 12 as a yellow oil (1.48 g, 99.8% yield).

(1.3.5) Synthesis of Compound 13

Compound 12 (1.48 g) prepared in step (1.3.4) was dissolved in DMF (15 ml), with triethylamine (TEA, 1.22 g), compound 4 (1.35 g) and HBTU (3.45 g) added, and stirred at 25°C for 16 hours of reaction. After completion of the reaction, the reaction solution was added to 150 ml of saturated aqueous sodium bicarbonate solution, extracted three times (3×50 ml) with 50 ml of ethyl acetate, the organic phases were combined, washed once (1×30 ml) with 30 ml of saturated aqueous sodium chloride solution, dried over anhydrous sodium sulfate, distilled to dryness under reduced pressure, and purified by column chromatography on reverse-phase(C18 column, eluent: water/acetonitrile =5/1, v/v), to obtain Compound 13 as a white solid (1.3 g, 31.8% yield). ESI-MS(m/z):674.3[M+H]$^+$.

(1.3.6) Synthesis of Compound 14

Compound 13 (1.1 g) prepared in step (1.3.5) was dissolved in 11 ml of pyridine, the reaction system was cooled to 0°C with an ice-water bath and DMTrCl (813 mg) was added portionwise at 0°C, and the reaction system was stirred at 0°C for 1 hour. After completion of the reaction, methanol was added to the reaction mixture for quench, the solvent was distilled off, and the mixture was purified by column chromatography on reverse-phase (eluent: water/acetonitrile = 14, v/v) to obtain Compound 14 as a white solid (800 mg, 50.3% yield). ESI-MS(m/z):976.5[M+H]$^+$.

(1.3.7) Synthesis of compound CR01013

Compound 14 (550 mg) was dissolved in 5 ml of dichloromethane (DCM) at 25°C, with 4,5-dicyanoimidazole (DCI, 53.2 mg) and compound 7 (2-cyanoethyl N,N, N', N'-tetraisopropylphosphoramidite, 255.4 mg) added, purged with nitrogen for three times , and stirred at 25°C for 1 hour of reaction under nitrogen atmosphere. After completion of the reaction, the reaction solution was washed twice (2×5 ml) with 5 ml of saturated aqueous sodium bicarbonate solution and once (1×30 ml) with 30 ml of saturated aqueous sodium chloride solution, the organic phase was separated, dried over anhydrous sodium sulfate, distilled to dryness under reduced pressure, and purified by column chromatography on normal phase (eluent: methylene chloride/methanol =20/1, v/v) to obtain compound CR01013 (532 mg, 80.4% yield) as a white solid. ESI-MS(m/z): 1176.7[M+H]$^+$.

$^1H$ NMR (400 MHz, DMSO-d6) δ 0.95 - 1.05 (d, J = 6.7 Hz, 5H), 1.06 - 1.15 (q, J = 7.6 Hz, 8H), 1.15 - 1.21 (t, J = 7.2 Hz, 14H), 1.72 - 1.80 (s, 3H), 1.84 - 1.92 (s, 3H), 1.94 - 2.07 (d, J = 16.0 Hz, 7H), 2.07 - 2.14 (s, 3H), 2.64 - 2.72 (q, J = 5.8 Hz, 2H), 2.74 - 2.89 (d, J = 8.5 Hz, 2H), 3.35 - 3.56 (m, 4H), 3.57 - 3.70 (m, 4H), 3.71 - 3.77 (s, 6H), 3.81 - 3.93 (m, 1H), 3.96 - 4.09 (d, J = 6.4 Hz, 3H), 6.82 - 6.97 (d, J = 8.7 Hz, 4H), 7.17 - 7.27 (t, J = 8.7 Hz, 5H), 7.27 - 7.34 (t, J = 7.6 Hz, 2H), 7.34 - 7.43 (d, J = 7.5 Hz, 2H).

**Preparation Example 4. Synthesis of Compound CR01013Z**

The synthetic route of compound CR01013Z is as follows:

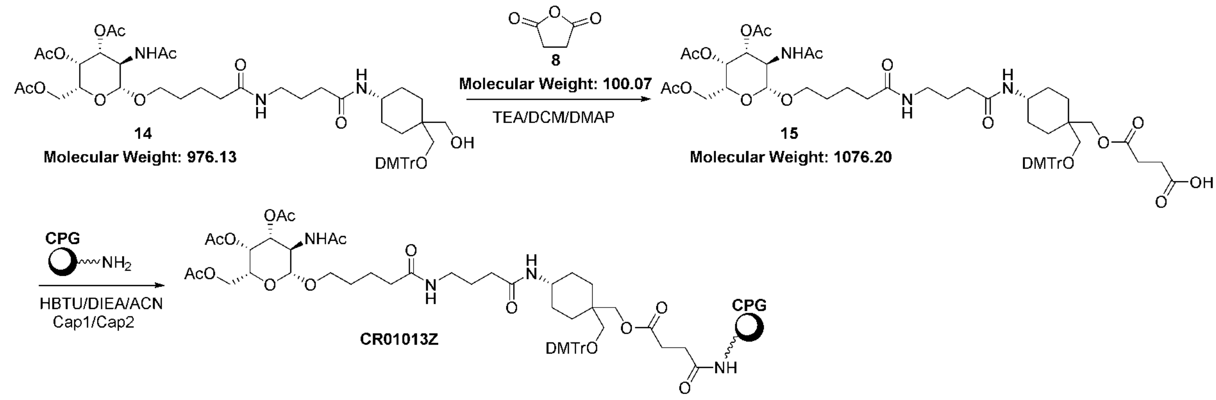


(1.4.1) Synthesis of Compound 15

Compound 14 (100 mg, 0.10 mmol) was dissolved in 2 ml of dichloromethane at 25°C, with Triethylamine (25.9 mg, 0.25 mmol), DMAP (1.25 mg, 0.01 mmol) and compound 8 (succinic anhydride, 15.4 mg, 0.15 mmol) added, and stirred at 25°C for 16 hours of reaction. After completion of the reaction, the solvent in the reaction solution was distilled and purified by column chromatography on reverse-phase (C18 column, eluent: water/acetonitrile =2/1, v/v) to obtain Compound 15 as a yellow oil (110 mg, 0.10 mmol, 100% yield). ESI-MS(m/z) = 1099.3[M+Na]$^+$.

(1.4.2) Synthesis of compound CR01013Z

Compound 15 (50 mg, 0.04 mmol) was dissolved in acetonitrile (10 ml), with HBTU (24.2 mg, 0.06 mmol), DIEA (11.0 mg, 0.08 mmol) and amino-CPG (1.06 g, loading 80μmol/g) added, and stirred at °C for 16 hours of reaction. After completion of the reaction, the reaction solution was filtered to obtain a filter cake, which was washed twice with 50 ml of dichloromethane (2×50 ml), three times with 50 ml of acetonitrile (3×50 ml) and once with 50 ml of ethyl acetate (1×50 ml), and dried in vacuum. Cap1 (4.8 ml), Cap2 (0.54 ml) and DMAP (2.59 mg) were added to the dried filter cake, and stirred at 25°C for 5 hours of reaction. After completion of the reaction, the reaction solution was filtered to obtain a filter cake, which was washed three times (3×50 ml) with 50 ml of acetonitrile and dried in vacuum to obtain compound CR01013Z (900 mg, loading 20-30 μmol/g).

Herein, Cap1 and Cap2 are capping reagents. Cap1 is a 20 vol% N-methylimidazole solution in a pyridine/acetonitrile mixture, and the volume ratio of pyridine to acetonitrile is 3:5; and Cap2 is a 20 vol% acetic anhydride solution in acetonitrile.

**Compound L96-PS**

The structural formula of compound L96-PS is as follows:

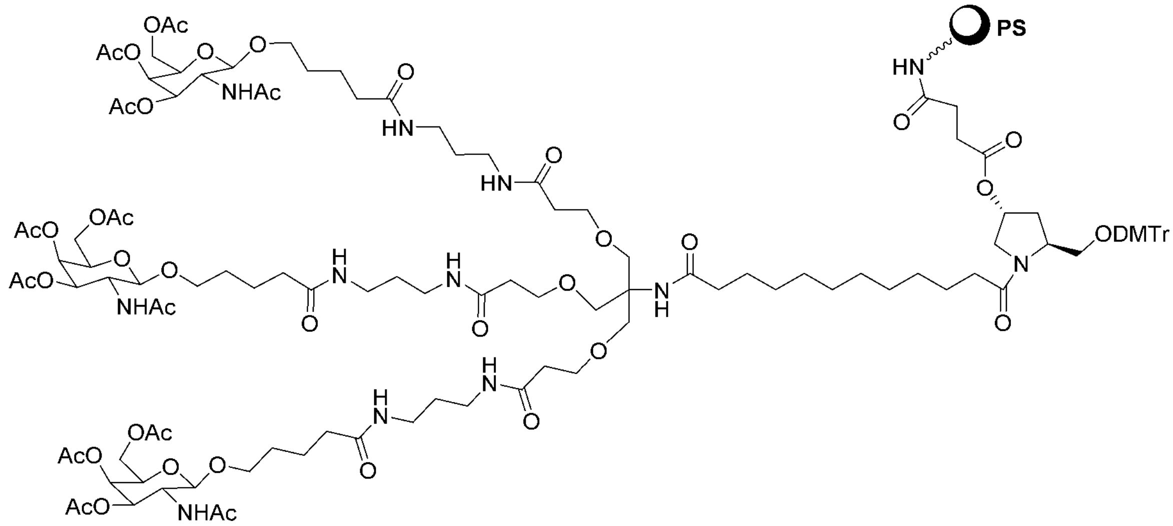

Herein, PS stands for Polystyrene resin solid phase carrier.

## Preparation of double-stranded oligonucleotides (siRNA)

### Preparation Example 5

(1.5.1) Synthesis of Sense strand SS

Using the phosphoramidite solid-phase synthesis method, nucleotide monomers are cyclically linked one by one in the 3'-5'direction according to the nucleotide sequence. Each linkage of a nucleotide monomer involves four steps: deprotection, coupling, capping, and oxidation or sulfurization. The synthesis conditions are specified as follows: Nucleoside monomer was prepared as an acetonitrile solution of nucleoside monomer at a concentration of 0.1 M.

The deprotection reaction conditions for each step were identical. The conditions for the deprotection reaction were as follows: temperature of 25 °C, reaction time of 70 seconds, and the deprotection reagent was a dichloroacetic acid solution in dichloromethane (3 vol%), with a molar ratio of 5 : 1 between dichloroacetic acid to the 4,4'-dimethoxytrityl protecting group on the solid support.

The conditions for the coupling reaction in each step were identical. The coupling reaction conditions were as follows: temperature of 25 °C, a molar ratio of 1:10 between the nucleic acid sequence attached to the solid support and the nucleoside monomer, a molar ratio of 1:65 between the nucleic acid sequence attached to the solid support and the coupling reagent, a reaction time of 600 seconds, a coupling reagent of 0.5 M 5-ethylthio-1H-tetrazole in acetonitrile, and a sulfurization reagent of 0.2 M phenylacetyl disulfide (PADS) in a mixed acetonitrile/pyridine solution (volume ratio of acetonitrile to pyridine was 1:1).

The conditions for the capping reaction in each step were identical. The capping reaction conditions were as follows: temperature of 25 °C; reaction time of 2 minutes. The capping reagent solution is a mixed solution of Cap1 and Cap2 with a molar ratio of 1:1, Cap1 is a 20 vol% N-methylimidazole solution in a pyridine/acetonitrile mixture, the volume ratio of pyridine to acetonitrile is 3:5, Cap2 is a acetic anhydride solution in acetonitrile at a concentration of 20 v/v%, and the molar ratio of N-methylimidazole in Cap1 capping reagent, acetic anhydride in Cap2 capping reagent and nucleic acid sequence linked on solid phase carrier is 1:1:1.

The conditions for the oxidation reaction in each step were identical. The oxidation reaction conditions were as follows: temperature of 25 °C; reaction time of 3 seconds; a sulfurization reagent of 0.05 M iodine water ; the molar ratio of iodine to nucleic acid sequence linked to the solid support in coupling reaction is 30:1; the oxidation reaction was carried out in water/pyridine mixed solvent (the volume ratio of water to pyridine was 1:9). The sulfurization reaction conditions were as follows: temperature of 25 °C; reaction time of 360 seconds; a sulfurization reagent of 0.2 M phenylacetyl disulfide in pyridine, the molar ratio of sulfurization reagent to nucleic acid sequence linked to the solid support in coupling reaction is 4:1; the sulfurization reaction was carried out in water/pyridine mixed solvent (the volume ratio of water to pyridine was 1:9).

After the final nucleoside monomer was linked, the nucleic acid sequence linked to the solid support was sequentially subjected to cleavage, deprotection, purification, and desalting, followed by lyophilization to obtain the sense strand, wherein:
The cleavage and deprotection conditions were as follows: the synthesized nucleotide sequence linked to the solid support was added to 25 wt% ammonia water with dosage of 0.5 ml/μmol at 55 °C for 16 hours of reaction, the solvent was

removed, and concentrated in vacuum to dryness. After ammonia treatment, the product was dissolved with 0.4 ml/μmol N-methylpyrrolidone relative to the amount of single-stranded nucleic acid, followed by the addition of 0.3 ml/μmol triethylamine and 0.6 ml/μmol triethylamine trifluorofluoride to remove 2'-O-TBDMS protection from ribose.

Purification and desalination conditions: Purification of nucleic acids was accomplished by gradient elution of NaCl using a preparative ion chromatography purification column (Source 15Q). Specifically, eluent 1 is 20mM sodium phosphate (pH=8.1), and solvent is water/acetonitrile mixed solution (volume ratio of water to acetonitrile is 9:1); eluent 2 is 1.5 M sodium chloride and 20mM sodium phosphate (pH=8.1), and solvent is water/acetonitrile mixed solution (volume ratio of water to acetonitrile is 9:1);the elution system is eluent 1: eluent 2=(100:0)-(50:50). The product-containing eluates were collected and combined, and desalting was then carried out using reverse chromatography purification column. The desalination conditions include desalination by dextran gel column, and the filler is dextran gel G25, eluted by deionized water.

Detection: the purity was determined by ion-exchange high-performance liquid chromatography (IEX-HPLC); and the molecular weight was determined by liquid chromatography-mass spectrometry (LC-MS; Liquid ChromatogRLhy-Mass SPlectrometry, model: LCT Premier, purchased from Waters Company) and the measured value of molecular weight was compared with the theoretical value, if the measured value was consistent with the theoretical value, it indicats that the obtained compound was conjugated to the 3'end of the sense strand of siRNA.

(1.5.2) Synthesis of antisense AS

The antisense strand was synthesized using universal solid-phase support. The deprotection, coupling, capping, oxidation or sulfurization reaction conditions, cleavage and deprotection conditions, purification and desalting conditions in the solid-phase synthesis method of the antisense strand were the same as those in step (1.5.1) for the synthesis of the sense strand.

Detection: the purity was determined by ion-exchange high-performance liquid chromatography (IEX-HPLC); and the molecular weight was determined by liquid chromatography-mass spectrometry (LC-MS, Liquid ChromatogRLhy-Mass SPlectrometry, model: LCT Premier, purchased from Waters Company) and the measured value of molecular weight was compared with the theoretical value, if the measured value was consistent with the theoretical value, it indicats that the antisense strands of siRNA were obtained.

(1.5.3) Synthesis of siRNA

The sense strand synthesized in step (1.5.1) and the antisense strand synthesized in step (1.5.2) are mixed in an equal molar ratio, dissolved in water for injection and heated to 95°C, then slowly cooled to room temperature and maintained at room temperature for 10 minutes to enable the sense strand and the antisense strand to form a double-stranded structure via hydrogen bonding, thereby obtaining the target siRNA.

Detection: Each siRNA was diluted to a concentration of 0.2 mg/ml (measured based on siRNA) using ultrapure water (Milli-Q ultrapure water system, resistivity 18.2MΩ*cm(25°C)), and the molecular weight was determined by liquid chromatography-mass spectrometry (LC-MS, Liquid ChromatogRLhy-Mass SPlectrometry, model: LCT Premier, purchased from Waters Company). The measured value consistenting with the theoretical value indicats that the synthesized siRNA was the designed target double-stranded nucleic acid sequence.

**Preparation of double-stranded oligonucleotide** (siRNA) **conjugates**

**Preparation Example 6**

(1.6.1) Synthesis of sense strand

Using the phosphoramidite solid-phase synthesis method, starting from the above-mentioned compounds (i.e., CR01008Z, CR01013Z, L96-PS) linked to the solid phase carrier, nucleotide monomers are cyclically linked one by one in the 3'-5'direction according to the nucleotide sequence (the compounds CR01008 and CR01013 can be regarded as a nucleoside monomer, respectively). Each linkage of a nucleotide monomer involves four steps: deprotection, coupling, capping, and oxidation or sulfurization. The deprotection, coupling, capping, oxidation or sulfurization reaction conditions, cleavage and deprotection conditions, purification and desalting conditions in the synthesis of the sense strand of the present Example were the same as those in step (1.5.1) of Preparation Example 5 for the synthesis of the sense strand.

(1.6.2) Synthesis of antisense strand

The antisense strand was synthesized using universal solid-phase support. The deprotection, coupling, capping,

oxidation or sulfurization reaction conditions, cleavage and deprotection conditions, purification and desalting conditions in the solid-phase synthesis method of the antisense strand were the same as those in step (1.5.2) of Preparation Example 5 for the synthesis of the antisense strand.

Detection: the purity was determined by ion-exchange high-performance liquid chromatography (IEX-HPLC); and the molecular weight was determined by liquid chromatography-mass spectrometry (LC-MS; Liquid ChromatogRLhy-Mass SPlectrometry, model: LCT Premier, purchased from Waters Company) and the measured value of molecular weight was compared with the theoretical value, if the measured value was consistent with the theoretical value, it indicats that the antisense strands of siRNA were obtained.

(1.6.3) Synthesis of siRNA

The sense strand synthesized in step (1.6.1) and the antisense strand synthesized in step (1.6.2) are mixed in an equal molar ratio, dissolved in water for injection and heated to 95°C, then slowly cooled to room temperature and maintained at room temperature for 10 minutes to enable the sense strand and the antisense strand to form a double-stranded structure via hydrogen bonding, thereby obtaining the target siRNA conjugate.

Detection: Each siRNA was diluted to a concentration of 0.2 mg/ml (measured based on siRNA) using ultrapure water (Milli-Q ultrapure water system, resistivity 18.2 MΩ*cm(25°C)), and the molecular weight was determined by liquid chromatography-mass spectrometry (LC-MS, Liquid ChromatogRLhy-Mass SPlectrometry, model: LCT Premier, purchased from Waters Company). The measured value consistenting with the theoretical value indicats that the synthesized siRNA conjugate was the designed target double-stranded nucleic acid sequence.

When the ligand is tri-cluster CR01008, the structural formula of the siRNA conjugate is:

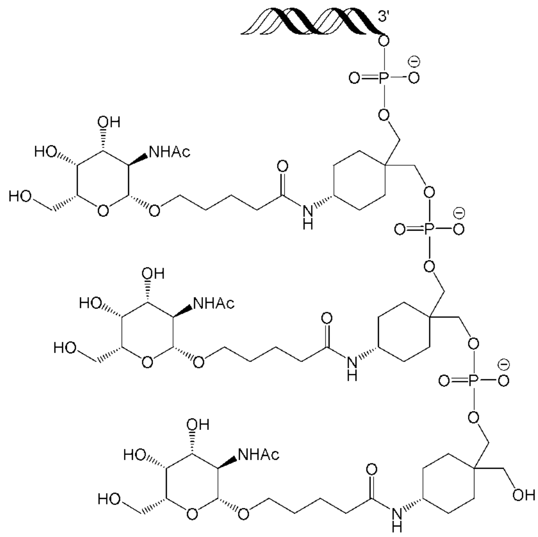

When the ligand is tri-cluster CR01013, the structural formula of the siRNA conjugate is:

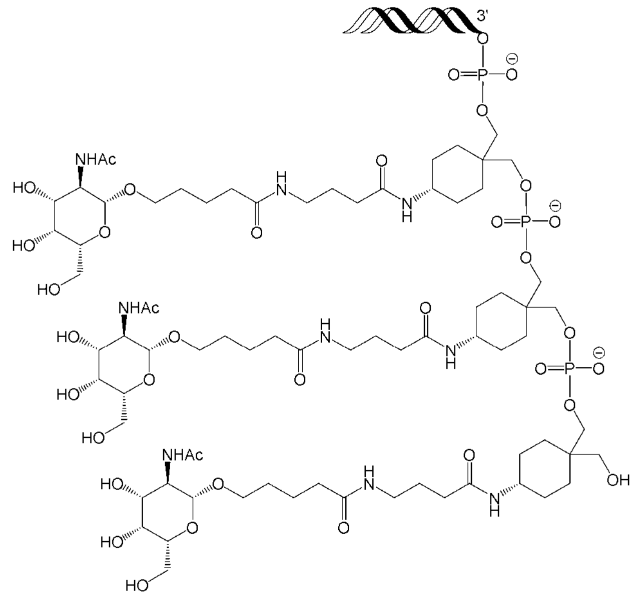

When the ligand is L96, the structural formula of the siRNA conjugate is:

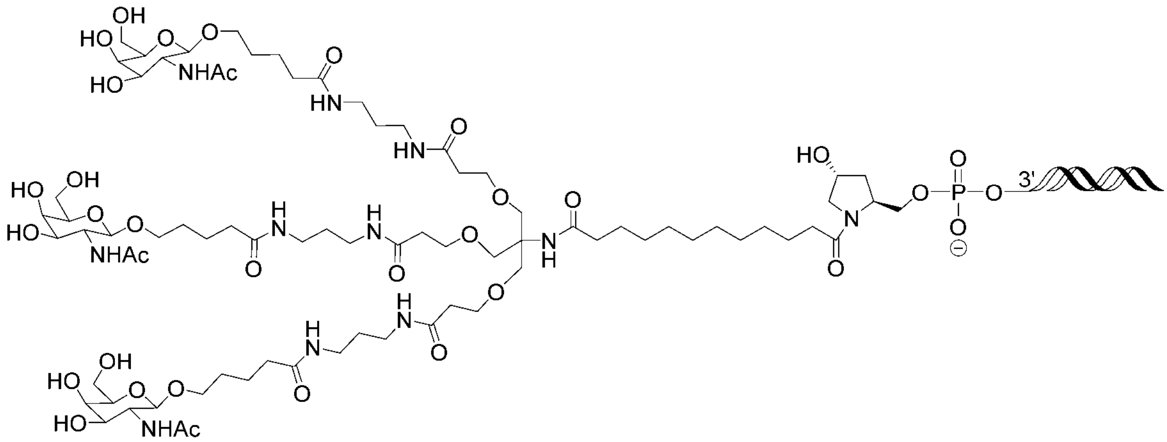

.

Herein, 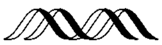 represents siRNA.

The unmodified double-stranded oligonucleotide sequences described in the present disclosure are shown in Table 1.

Table 1. Sequence listing of unmodified double-stranded oligonucleotides

| Serial number | Sense strand (5'-3') | SEQ ID | Antisense strand (5'-3') | SEQ ID |
|---|---|---|---|---|
| RN008001 | GUGAGGGUCAAGCACAGCU | NO.1 | AGCUGUGCUUGACCCUCACAG | NO.155 |
| RN008002 | GCUAUCCAUCAGAUGAUCU | NO.2 | AGAUCAUCUGAUGGAUAGCUG | NO.156 |
| RN008003 | GUCUGUGUGUCCCUCCUGU | NO.3 | ACAGGAGGGACACACAGACCC | NO.157 |
| RN008004 | GGAGCUAGCCAAGCAGCAA | NO.4 | UUGCUGCUUGGCUAGCUCCAG | NO.158 |
| RN008005 | GAGCUAGCCAAGCAGCAAA | NO.5 | UUUGCUGCUUGGCUAGCUCCA | NO.159 |
| RN008006 | CCAAGCAGCAAAUCCUGGA | NO.6 | UCCAGGAUUUGCUGCUUGGCU | NO.160 |
| RN008007 | CAAGCAGCAAAUCCUGGAU | NO.7 | AUCCAGGAUUUGCUGCUUGGC | NO.161 |
| RN008008 | GACCAGUCGUCCCAGAAUA | NO.8 | UAUUCUGGGACGACUGGUCAG | NO.162 |
| RN008009 | CAGAAUAACUCAUCCUCCA | NO.9 | UGGAGGAUGAGUUAUUCUGGG | NO.163 |
| RN008010 | GGAGUGUGGCUCCAGGGAA | NO.10 | UUCCCUGGAGCCACACUCCCU | NO.164 |

(continued)

| Serial number | Sense strand (5'-3') | SEQ ID | Antisense strand (5'-3') | SEQ ID |
|---|---|---|---|---|
| RN008011 | GAGUGUGGCUCCAGGGAAU | NO.11 | AUUCCCUGGAGCCACACUCCC | NO.165 |
| RN008012 | GGGAGGAGGUCAUCAGCUU | NO.12 | AAGCUGAUGACCUCCUCCCCA | NO.166 |
| RN008013 | GAGGUCAUCAGCUUUGCUA | NO. 13 | UAGCAAAGCUGAUGACCUCCU | NO.167 |
| RN008014 | GGUCAUCAGCUUUGCUACU | NO.14 | AGUAGCAAAGCUGAUGACCUC | NO.168 |
| RN008015 | CAGCUUUGCUACUGUCACA | NO.15 | UGUGACAGUAGCAAAGCUGAU | NO.169 |
| RN008016 | GCUUUGCUACUGUCACAGA | NO.16 | UCUGUGACAGUAGCAAAGCUG | NO.170 |
| RN008017 | GCUACUGUCACAGACUCCA | NO.17 | UGGAGUCUGUGACAGUAGCAA | NO.171 |
| RN008018 | GACUCCACUUCAGCCUACA | NO.18 | UGUAGGCUGAAGUGGAGUCUG | NO.172 |
| RN008019 | CUUCAGCCUACAGCUCCCU | NO.19 | AGGGAGCUGUAGGCUGAAGUG | NO.173 |
| RN008020 | CACCCUUCCUGGCACUCUU | NO.20 | AAGAGUGCCAGGAAGGGUGGG | NO.174 |
| RN008021 | CUUCCUGGCACUCUUUGCU | NO.21 | AGCAAAGAGUGCCAGGAAGGG | NO.175 |
| RN008022 | GGCACUCUUUGCUUGAGGA | NO.22 | UCCUCAAGCAAAGAGUGCCAG | NO.176 |
| RN008023 | ACUCUUUGCUUGAGGAUCU | NO.23 | AGAUCCUCAAGCAAAGAGUGC | NO.177 |
| RN008024 | CUCUUUGCUUGAGGAUCUU | NO.24 | AAGAUCCUCAAGCAAAGAGUG | NO.178 |
| RN008025 | GCUUGAGGAUCUUCCGAUG | NO.25 | CAUCGGAAGAUCCUCAAGCAA | NO.179 |
| RN008026 | GCUGAGCACCACAUCACCA | NO.26 | UGGUGAUGUGGUGCUCAGCCA | NO.180 |
| RN008027 | CUGAGCACCACAUCACCAA | NO.27 | UUGGUGAUGUGGUGCUCAGCC | NO.181 |
| RN008028 | CACAUCACCAACCUGGGCU | NO.28 | AGCCCAGGUUGGUGAUGUGGU | NO.182 |
| RN008029 | CCUUAACUCUGCCCUCUAG | NO.29 | CUAGAGGGCAGAGUUAAGGUA | NO.183 |
| RN008030 | CUGCCCUCUAGUGGCUUGA | NO.30 | UCAAGCCACUAGAGGGCAGAG | NO.184 |
| RN008031 | GAAGUCUGGUGUCCUGAAA | NO.31 | UUUCAGGACACCAGACUUCUC | NO.185 |
| RN008032 | CUGGUGUCCUGAAACUGCA | NO.32 | UGCAGUUUCAGGACACCAGAC | NO.186 |
| RN008033 | GUGUCCUGAAACUGCAACU | NO.33 | AGUUGCAGUUUCAGGACACCA | NO.187 |
| RN008034 | GAAGGCAACAGCACAGUUA | NO.34 | UAACUGUGCUGUUGCCUUCUA | NO.188 |
| RN008035 | CAACAGCACAGUUACUGGA | NO.35 | UCCAGUAACUGUGCUGUUGCC | NO.189 |
| RN008036 | GCGGCUCUUGGACACAGCA | NO.36 | UGCUGUGUCCAAGAGCCGCCU | NO.190 |
| RN008037 | GCAGCCCUUCCUAGAGCUU | NO.37 | AAGCUCUAGGAAGGGCUGCUG | NO.191 |
| RN008038 | AGCCCUUCCUAGAGCUUAA | NO.38 | UUAAGCUCUAGGAAGGGCUGC | NO.192 |
| RN008039 | CCUUCCUAGAGCUUAAGAU | NO.39 | AUCUUAAGCUCUAGGAAGGGC | NO.193 |
| RN008040 | CCUAGAGCUUAAGAUCCGA | NO.40 | UCGGAUCUUAAGCUCUAGGAA | NO.194 |
| RN008041 | GAGCUUAAGAUCCGAGCCA | NO.41 | UGGCUCGGAUCUUAAGCUCUA | NO.195 |
| RN008042 | GUUGCAGGCGAGACCAUUA | NO.42 | UAAUGGUCUCGCCUGCAACAU | NO.196 |
| RN008043 | GCGAGACCAUUACGUAGAC | NO.43 | GUCUACGUAAUGGUCUCGCCU | NO.197 |
| RN008044 | CGAGACCAUUACGUAGACU | NO.44 | AGUCUACGUAAUGGUCUCGCC | NO.198 |
| RN008045 | GAUGGCGGGACUGGAUACU | NO.45 | AGUAUCCAGUCCCGCCAUCCC | NO.199 |
| RN008046 | CCAGGCAUUGCUGCCUCUU | NO.46 | AAGAGGCAGCAAUGCCUGGGC | NO.200 |
| RN008047 | CCUCUUUCCAUUCUGCCGU | NO.47 | ACGGCAGAAUGGAAAGAGGCA | NO.201 |
| RN008048 | CCGUCUUCAGCCUCCUCAA | NO.48 | UUGAGGAGGCUGAAGACGGCA | NO.202 |
| RN008049 | GCCUCCUCAAAGCCAACAA | NO.49 | UUGUUGGCUUUGAGGAGGCUG | NO.203 |

(continued)

| Serial number | Sense strand (5'-3') | SEQ ID | Antisense strand (5'-3') | SEQ ID |
|---|---|---|---|---|
| RN008050 | CCUCCUCAAAGCCAACAAU | NO.50 | AUUGUUGGCUUUGAGGAGGCU | NO.204 |
| RN008051 | CUCCUGUUGUGUCCCUACU | NO.51 | AGUAGGGACACAACAGGAGGU | NO.205 |
| RN008052 | CUCUCCUCUACCUGGAUCA | NO.52 | UGAUCCAGGUAGAGGAGAGAG | NO.206 |
| RN008053 | CCUCUACCUGGAUCAUAAU | NO.53 | AUUAUGAUCCAGGUAGAGGAG | NO.207 |
| RN008054 | CCUGGAUCAUAAUGGCAAU | NO.54 | AUUGCCAUUAUGAUCCAGGUA | NO.208 |
| RN008055 | CAUAAUGGCAAUGUGGUCA | NO.55 | UGACCACAUUGCCAUUAUGAU | NO.209 |
| RN008056 | GCAAUGUGGUCAAGACGGA | NO.56 | UCCGUCUUGACCACAUUGCCA | NO.210 |
| RN008057 | GGUCAAGACGGAUGUGCCA | NO.57 | UGGCACAUCCGUCUUGACCAC | NO.211 |
| RN008058 | CAAGACGGAUGUGCCAGAU | NO.58 | AUCUGGCACAUCCGUCUUGAC | NO.212 |
| RN008059 | GCUGCAGCUAGCAAGAGGA | NO.59 | UCCUCUUGCUAGCUGCAGCCA | NO.213 |
| RN008060 | GCAGCUAGCAAGAGGACCU | NO.60 | AGGUCCUCUUGCUAGCUGCAG | NO.214 |
| RN008061 | GGAGUGAAGAGACCAAGAU | NO.61 | AUCUUGGUCUCUUCACUCCAA | NO.215 |
| RN008062 | AGUGAAGAGACCAAGAUGA | NO.62 | UCAUCUUGGUCUCUUCACUCC | NO.216 |
| RN008063 | GUGAAGAGACCAAGAUGAA | NO.63 | UUCAUCUUGGUCUCUUCACUC | NO.217 |
| RN008064 | GCAUCUGUGACUGGAGGCA | NO.64 | UGCCUCCAGUCACAGAUGCCC | NO.218 |
| RN008065 | CUGGAGGCAUCAGAUUCCU | NO.65 | AGGAAUCUGAUGCCUCCAGUC | NO.219 |
| RN008066 | GCAUCAGAUUCCUGAUCCA | NO.66 | UGGAUCAGGAAUCUGAUGCCU | NO.220 |
| RN008067 | CCCAACAACCACCUGGCAA | NO.67 | UUGCCAGGUGGUUGUUGGGUU | NO.221 |
| RN008068 | CCAACAACCACCUGGCAAU | NO.68 | AUUGCCAGGUGGUUGUUGGGU | NO.222 |
| RN008069 | CACCUGGCAAUAUGACUCA | NO.69 | UGAGUCAUAUUGCCAGGUGGU | NO.223 |
| RN008070 | CCUGGCAAUAUGACUCACU | NO.70 | AGUGAGUCAUAUUGCCAGGUG | NO.224 |
| RN008071 | CUGGCAAUAUGACUCACUU | NO.71 | AAGUGAGUCAUAUUGCCAGGU | NO.225 |
| RN008072 | GGGACCCAAAUGGGCACUU | NO.72 | AAGUGCCCAUUUGGGUCCCAU | NO.226 |
| RN008073 | GGACCCAAAUGGGCACUUU | NO.73 | AAAGUGCCCAUUUGGGUCCCA | NO.227 |
| RN008074 | CCCAAAUGGGCACUUUCUU | NO.74 | AAGAAAGUGCCCAUUUGGGUC | NO.228 |
| RN008075 | CAAAUGGGCACUUUCUUGU | NO.75 | ACAAGAAAGUGCCCAUUUGGG | NO.229 |
| RN008076 | GGCACUUUCUUGUCUGAGA | NO.76 | UCUCAGACAAGAAAGUGCCCA | NO.230 |
| RN008077 | CUCUGGCUUAUUCCAGGUU | NO.77 | AACCUGGAAUAAGCCAGAGUC | NO.231 |
| RN008078 | GUAAAGCGUUUCUUCUAAA | NO.78 | UUUAGAAGAAACGCUUUACCC | NO.232 |
| RN008079 | CCAGAAAGCAUGAUUUCCU | NO.79 | AGGAAAUCAUGCUUUCUGGGU | NO.233 |
| RN008080 | GCCCUAAGUCCUGUGAGAA | NO.80 | UUCUCACAGGACUUAGGGCAG | NO.234 |
| RN008081 | CCUAAGUCCUGUGAGAAGA | NO.81 | UCUUCUCACAGGACUUAGGGC | NO.235 |
| RN008082 | GAGAAGAUGUCAGGGACUA | NO.82 | UAGUCCCUGACAUCUUCUCAC | NO.236 |
| RN008083 | GGGAGGGAAGGCAGAGAAA | NO.83 | UUUCUCUGCCUUCCCUCCCUC | NO.237 |
| RN008084 | CCUCUCCCAAGAUGAGAAA | NO.84 | UUUCUCAUCUUGGGAGAGGCU | NO.238 |
| RN008085 | CCCAAGAUGAGAAAGUCCU | NO.85 | AGGACUUUCUCAUCUUGGGAG | NO.239 |
| RN008086 | CAAGAUGAGAAAGUCCUCA | NO.86 | UGAGGACUUUCUCAUCUUGGG | NO.240 |
| RN008087 | GGAGGAGGAAGCAGAUAGA | NO.87 | UCUAUCUGCUUCCUCCUCCCC | NO.241 |
| RN008088 | GAAGCAGAUAGAUGGUCCA | NO.88 | UGGACCAUCUAUCUGCUUCCU | NO.242 |

(continued)

| Serial number | Sense strand (5'-3') | SEQ ID | Antisense strand (5'-3') | SEQ ID |
|---|---|---|---|---|
| RN008089 | GCAGAUAGAUGGUCCAGCA | NO.89 | UGCUGGACCAUCUAUCUGCUU | NO.243 |
| RN008090 | GCAGGCUUGAAGCAGGGUA | NO.90 | UACCCUGCUUCAAGCCUGCUG | NO.244 |
| RN008091 | GGUAAGGGCUGUUGAGGUA | NO.91 | UACCUCAACAGCCCUUACCCU | NO.245 |
| RN008092 | GGCUGUUGAGGUACCUUAA | NO.92 | UUAAGGUACCUCAACAGCCCU | NO.246 |
| RN008093 | CCUUAAGGGAAGGUCAAGA | NO.93 | UCUUGACCUUCCCUUAAGGUA | NO.247 |
| RN008094 | AAGGGAAGGUCAAGAGGGA | NO.94 | UCCCUCUUGACCUUCCCUUAA | NO.248 |
| RN008095 | GGAAGGUCAAGAGGGAGAU | NO.95 | AUCUCCCUCUUGACCUUCCCU | NO.249 |
| RN008096 | UCAAGAGGGAGAUGGGCAA | NO.96 | UUGCCCAUCUCCCUCUUGACC | NO.250 |
| RN008097 | GCGCUGAGGGAGGAUGCUU | NO.97 | AAGCAUCCUCCCUCAGCGCCU | NO.251 |
| RN008098 | CGCUGAGGGAGGAUGCUUA | NO.98 | UAAGCAUCCUCCCUCAGCGCC | NO.252 |
| RN008099 | AGAAACAGGAGUCAGGAAA | NO.99 | UUUCCUGACUCCUGUUUCUGG | NO.253 |
| RN008100 | GAAACAGGAGUCAGGAAAA | NO.100 | UUUUCCUGACUCCUGUUUCUG | NO.254 |
| RN008101 | GAGGCACUAAGCCUAAGAA | NO.101 | UUCUUAGGCUUAGUGCCUCAU | NO.255 |
| RN008102 | GCACUAAGCCUAAGAAGUU | NO.102 | AACUUCUUAGGCUUAGUGCCU | NO.256 |
| RN008103 | CCUAAGAAGUUCCCUGGUU | NO.103 | AACCAGGGAACUUCUUAGGCU | NO.257 |
| RN008104 | CACUAAUUUUUGUAUUCUU | NO.104 | AAGAAUACAAAAAUUAGUGGG | NO.258 |
| RN008105 | GAGAAAGAAAAUCAACAAA | NO.105 | UUUGUUGAUUUUCUUUCUCCU | NO.259 |
| RN008106 | AAUCAACAAAUGUGAGUCA | NO.106 | UGACUCACAUUUGUUGAUUUU | NO.260 |
| RN008107 | CAACAAAUGUGAGUCAUAA | NO.107 | UUAUGACUCACAUUUGUUGAU | NO.261 |
| RN008108 | CAAAUGUGAGUCAUAAAGA | NO.108 | UCUUUAUGACUCACAUUUGUU | NO.262 |
| RN008109 | AAAUGUGAGUCAUAAAGAA | NO.109 | UUCUUUAUGACUCACAUUUGU | NO.263 |
| RN008110 | GUGAGUCAUAAAGAAGGGU | NO.110 | ACCCUUCUUUAUGACUCACAU | NO.264 |
| RN008111 | GAGUCAUAAAGAAGGGUUA | NO.111 | UAACCCUUCUUUAUGACUCAC | NO.265 |
| RN008112 | UAAAGAAGGGUUAGGGUGA | NO.112 | UCACCCUAACCCUUCUUUAUG | NO.266 |
| RN008113 | GGGUUAGGGUGAUGGUCCA | NO.113 | UGGACCAUCACCCUAACCCUU | NO.267 |
| RN008114 | GUUAGGGUGAUGGUCCAGA | NO.114 | UCUGGACCAUCACCCUAACCC | NO.268 |
| RN008115 | GGGUGAUGGUCCAGAGCAA | NO.115 | UUGCUCUGGACCAUCACCCUA | NO.269 |
| RN008116 | GUCCAGAGCAACAGUUCUU | NO.116 | AAGAACUGUUGCUCUGGACCA | NO.270 |
| RN008117 | CAGAGCAACAGUUCUUCAA | NO.117 | UUGAAGAACUGUUGCUCUGGA | NO.271 |
| RN008118 | GAGCAACAGUUCUUCAAGU | NO.118 | ACUUGAAGAACUGUUGCUCUG | NO.272 |
| RN008119 | GCAACAGUUCUUCAAGUGU | NO.119 | ACACUUGAAGAACUGUUGCUC | NO.273 |
| RN008120 | GUCCACAAAGUCAAAGCUA | NO.120 | UAGCUUUGACUUUGUGGACAC | NO.274 |
| RN008121 | UCCACAAAGUCAAAGCUAU | NO.121 | AUAGCUUUGACUUUGUGGACA | NO.275 |
| RN008122 | CCACAAAGUCAAAGCUAUU | NO.122 | AAUAGCUUUGACUUUGUGGAC | NO.276 |
| RN008123 | CACAAAGUCAAAGCUAUUU | NO.123 | AAAUAGCUUUGACUUUGUGGA | NO.277 |
| RN008124 | CAAAGCUAUUUUCAUAAUA | NO.124 | UAUUAUGAAAAUAGCUUUGAC | NO.278 |
| RN008125 | CUAUUUUCAUAAUAAUACU | NO.125 | AGUAUUAUUAUGAAAAUAGCU | NO.279 |
| RN008126 | CAUAAUAAUACUAACAUGU | NO.126 | ACAUGUUAGUAUUAUUAUGAA | NO.280 |
| RN008127 | CAUGUUAUUUGCCUUUUGA | NO.127 | UCAAAAGGCAAAUAACAUGUU | NO.281 |

(continued)

| Serial number | Sense strand (5'-3') | SEQ ID | Antisense strand (5'-3') | SEQ ID |
|---|---|---|---|---|
| RN008128 | GUUAUUUGCCUUUUGAAUU | NO.128 | AAUUCAAAAGGCAAAUAACAU | NO.282 |
| RN008129 | CAGCACAGUUACUGGACAA | NO.129 | UUGUCCAGUAACUGUGCUGUU | NO.283 |
| RN008130 | GCUUAAGAUCCGAGCCAAU | NO. 130 | AUUGGCUCGGAUCUUAAGCUC | NO.284 |
| RN008131 | CCAGCUGAAUUACUGCAGU | NO.131 | ACUGCAGUAAUUCAGCUGGUA | NO.285 |
| RN008132 | CUCAAAGCCAACAAUCCUU | NO.132 | AAGGAUUGUUGGCUUUGAGGA | NO.286 |
| RN008133 | GGAGGCAUCAGAUUCCUGA | NO.133 | UCAGGAAUCUGAUGCCUCCAG | NO.287 |
| RN008134 | GGCAAUAUGACUCACUUGA | NO.134 | UCAAGUGAGUCAUAUUGCCAG | NO.288 |
| RN008135 | GCCUCUCCCAAGAUGAGAA | NO.135 | UUCUCAUCUUGGGAGAGGCUA | NO.289 |
| RN008136 | CAGGCUUGAAGCAGGGUAA | NO.136 | UUACCCUGCUUCAAGCCUGCU | NO.290 |
| RN008137 | GCUUGAAGCAGGGUAAGCA | NO.137 | UGCUUACCCUGCUUCAAGCCU | NO.291 |
| RN008138 | CUAAGAAGUUCCCUGGUUU | NO.138 | AAACCAGGGAACUUCUUAGGC | NO.292 |
| RN008139 | CCCACUGGGAGACAAGCAU | NO.139 | AUGCUUGUCUCCCAGUGGGUC | NO.293 |
| RN008140 | CACUGGGAGACAAGCAUUU | NO.140 | AAAUGCUUGUCUCCCAGUGGG | NO.294 |
| RN008141 | GAGACAAGCAUUUAUACUU | NO.141 | AAGUAUAAAUGCUUGUCUCCC | NO.295 |
| RN008142 | CAAGCAUUUAUACUUUCUU | NO.142 | AAGAAAGUAUAAAUGCUUGUC | NO.296 |
| RN008143 | CCGCGCCUGGCUUAUACUU | NO.143 | AAGUAUAAGCCAGGCGCGGUG | NO.297 |
| RN008144 | GCCUGGCUUAUACUUUCUU | NO.144 | AAGAAAGUAUAAGCCAGGCGC | NO.298 |
| RN008145 | GCUUAUACUUUCUUAAUAA | NO.145 | UUAUUAAGAAAGUAUAAGCCA | NO.299 |
| RN008146 | GGGUGUCCACAAAGUCAAA | NO.146 | UUUGACUUUGUGGACACCCCU | NO.300 |
| RN008147 | GCCUUUUGAAUUCUCAUUA | NO.147 | UAAUGAGAAUUCAAAAGGCAA | NO.301 |
| RN008148 | GAAUUCUCAUUAUCUUAAA | NO.148 | UUUAAGAUAAUGAGAAUUCAA | NO.302 |
| RN008149 | CAUUAUCUUAAAAUUGUAU | NO.149 | AUACAAUUUUAAGAUAAUGAG | NO.303 |
| RN008150 | CAUGUGAUUACAUCAUCUU | NO.150 | AAGAUGAUGUAAUCACAUGUC | NO.304 |
| RN008151 | GAUUACAUCAUCUUUCUGA | NO.151 | UCAGAAAGAUGAUGUAAUCAC | NO.305 |
| RN008152 | CUUUCUGACAUCAUUGUUA | NO.152 | UAACAAUGAUGUCAGAAAGAU | NO.306 |
| RN008153 | CAUUGUUAAUGGAAUGUGU | NO.153 | ACACAUUCCAUUAACAAUGAU | NO.307 |
| RN008154 | GUUAAUGGAAUGUGUGCUU | NO.154 | AAGCACACAUUCCAUUAACAA | NO.308 |

The modified double-stranded oligonucleotide sequences described in the present disclosure are shown in Table 2.

Table 2. Sequence listing of modified double-stranded oligonucleotides

| Serial number | Modified sense strand (5'-3') | SEQ ID | Modified antisense strand (5'-3') | SEQ ID |
|---|---|---|---|---|
| RX008001 | GmsUmsGmAmGmGmGfUfCfAmAmGmCmAmCmAmGmCmUm | NO.325 | AmsGfsCmUmGmUfGmCmUmUmGmAmCmCfCmUfCmAmCmsAmsGm | NO.578 |
| RX008002 | GmsCmsUmAmUmCmCfAfUfCmAmGmAmUmGmAmUmCmUm | NO.326 | AmsGfsAmUmCmAfUmCmUmGmAmUmGmGfAmUfAmGmCmsUmsGm | NO.579 |

(continued)

| Serial number | Modified sense strand (5'-3') | SEQ ID | Modified antisense strand (5'-3') | SEQ ID |
|---|---|---|---|---|
| RX008003 | GmsUmsCmUmGmUmGfUfGfUmCmCmCmUmCmCmUmGmUm | NO.327 | AmsCfsAmGmGmAfGmGmGmAmCmAmCmAfCmAfGmAmCmsCmsCm | NO.580 |
| RX008004 | GmsGmsAmGmCmUmAfGfCfCmAmAmGmCmAmGmCmAmAm | NO.328 | UmsUfsGmCmUmGfCmUmUmGmGmCmUmAfGmCfUmCmCmsAmsGm | NO.581 |
| RX008005 | GmsAmsGmCmUmAmGfCfCfAmAmGmCmAmGmCmAmAmAm | NO.329 | UmsUfsUmGmCmUfGmCmUmUmGmGmCmUfAmGfCmUmCmsCmsAm | NO.582 |
| RX008006 | CmsCmsAmAmGmCmAfGfCfAmAmAmUmCmCmUmGmGmAm | NO.330 | UmsCfsCmAmGmGfAmUmUmUmGmCmUmGfCmUfUmGmGmsCmsUm | NO.583 |
| RX008007 | CmsAmsAmGmCmAmGfCfAfAmAmUmCmCmUmGmGmAmUm | NO.331 | AmsUfsCmCmAmGfGmAmUmUmUmGmCmUfGmCfUmUmGmsGmsCm | NO.584 |
| RX008008 | GmsAmsCmCmAmGmUfCfGfUmCmCmCmAmGmAmAmUmAm | NO.332 | UmsAfsUmUmCmUfGmGmGmAmCmGmAmCfUmGfGmUmCmsAmsGm | NO.585 |
| RX008009 | CmsAmsGmAmAmUmAfAfCfUmCmAmUmCmCmUmCmCmAm | NO.333 | UmsGfsGmAmGmGfAmUmGmAmGmUmUmAfUmUfCmUmGmsGmsGm | NO.586 |
| RX008010 | GmsGmsAmGmUmGmUfGfGfCmUmCmCmAmGmGmGmAmAm | NO.334 | UmsUfsCmCmCmUfGmGmAmGmCmCmAmCfAmCfUmCmCmsCmsUm | NO.587 |
| RX008011 | GmsAmsGmUmGmUmGfGfCfUmCmCmAmGmGmGmAmAmUm | NO.335 | AmsUfsUmCmCmCfUmGmGmAmGmCmCmAfCmAfCmUmCmsCmsCm | NO.588 |
| RX008012 | GmsGmsGmAmGmGmAfGfGfUmCmAmUmCmAmGmCmUmUm | NO.336 | AmsAfsGmCmUmGfAmUmGmAmCmCmUmCfCmUfCmCmCmsCmsAm | NO.589 |
| RX008013 | GmsAmsGmGmUmCmAfUfCfAmGmCmUmUmUmGmCmUmAm | NO.337 | UmsAfsGmCmAmAfAmGmCmUmGmAmUmGfAmCfCmUmCmsCmsUm | NO.590 |

(continued)

| Serial number | Modified sense strand (5'-3') | SEQ ID | Modified antisense strand (5'-3') | SEQ ID |
|---|---|---|---|---|
| RX008014 | GmsGmsUmCmAmUmCfAfGfCmUmUmUmGmCmUmAmCmUm | NO.338 | AmsGfsUmAmGmCfAmAmAmGmCmUmGmAfUmGfAmCmCmsUmsCm | NO.591 |
| RX008015 | CmsAmsGmCmUmUmUfGfCfUmAmCmUmGmUmCmAmCmAm | NO.339 | UmsGfsUmGmAmCfAmGmUmAmGmCmAmAfAmGfCmUmGmsAmsUm | NO.592 |
| RX008016 | GmsCmsUmUmUmGmCfUfAfCmUmGmUmCmAmCmAmGmAm | NO.340 | UmsCfsUmGmUmGfAmCmAmGmUmAmGmCfAmAfAmGmCmsUmsGm | NO.593 |
| RX008017 | GmsCmsUmAmCmUmGfUfCfAmCmAmGmAmCmUmCmCmAm | NO.341 | UmsGfsGmAmGmUfCmUmGmUmGmAmCmAfGmUfAmGmCmsAmsAm | NO.594 |
| RX008018 | GmsAmsCmUmCmCmAfCfUfUmCmAmGmCmCmUmAmCmAm | NO.342 | UmsGfsUmAmGmGfCmUmGmAmAmGmUmGfGmAfGmUmCmsUmsGm | NO.595 |
| RX008019 | CmsUmsUmCmAmGmCfCfUfAmCmAmGmCmUmCmCmCmUm | NO.343 | AmsGfsGmGmAmGfCmUmGmUmAmGmGmCfUmGfAmAmGmsUmsGm | NO.596 |
| RX008020 | CmsAmsCmCmCmUmUfCfCfUmGmGmCmAmCmUmCmUmUm | NO.344 | AmsAfsGmAmGmUfGmCmCmAmGmGmAmAfGmGfGmUmGmsGmsGm | NO.597 |
| RX008021 | CmsUmsUmCmCmUmGfGfCfAmCmUmCmUmUmUmGmCmUm | NO.345 | AmsGfsCmAmAmAfGmAmGmUmGmCmCmAfGmGfAmAmGmsGmsGm | NO.598 |
| RX008022 | GmsGmsCmAmCmUmCfUfUfUmGmCmUmUmGmAmGmGmAm | NO.346 | UmsCfsCmUmCmAfAmGmCmAmAmAmGmAfGmUfGmCmCmsAmsGm | NO.599 |
| RX008023 | AmsCmsUmCmUmUmUfGfCfUmUmGmAmGmGmAmUmCmUm | NO.347 | AmsGfsAmUmCmCfUmCmAmAmGmCmAmAfAmGfAmGmUmsGmsCm | NO.600 |
| RX008024 | CmsUmsCmUmUmUmGfCfUfUmGmAmGmGmAmUmCmUmUm | NO.348 | AmsAfsGmAmUmCfCmUmCmAmAmGmCmAfAmAfGmAmGmsUmsGm | NO.601 |

(continued)

| Serial number | Modified sense strand (5'-3') | SEQ ID | Modified antisense strand (5'-3') | SEQ ID |
|---|---|---|---|---|
| RX008025 | GmsCmsUmUmGmAmGfGfAfUmCmUmUmCmCmGmAmUmGm | NO.349 | CmsAfsUmCmGmGfAmAmGmAmUmCmCmUfCmAfAmGmCmsAmsAm | NO.602 |
| RX008026 | GmsCmsUmGmAmGmCfAfCfCmAmCmAmUmCmAmCmCmAm | NO.350 | UmsGfsGmUmGmAfUmGmUmGmGmUmGmCfUmCfAmGmCmsCmsAm | NO.603 |
| RX008027 | CmsUmsGmAmGmCmAfCfCfAmCmAmUmCmAmCmCmAmAm | NO.351 | UmsUfsGmGmUmGfAmUmGmUmGmGmUmGfCmUfCmAmGmsCmsCm | NO.604 |
| RX008028 | CmsAmsCmAmUmCmAfCfCfAmAmCmCmUmGmGmGmCmUm | NO.352 | AmsGfsCmCmCmAfGmGmUmUmGmGmUmGfAmUfGmUmGmsGmsUm | NO.605 |
| RX008029 | CmsCmsUmUmAmAmCfUfCfUmGmCmCmCmUmCmUmAmGm | NO.353 | CmsUfsAmGmAmGfGmGmCmAmGmAmGmUfUmAfAmGmGmsUmsAm | NO.606 |
| RX008030 | CmsUmsGmCmCmCmUfCfUfAmGmUmGmGmCmUmUmGmAm | NO.354 | UmsCfsAmAmGmCfCmAmCmUmAmGmAmGfGmGfCmAmGmsAmsGm | NO.607 |
| RX008031 | GmsAmsAmGmUmCmUfGfGfUmGmUmCmCmUmGmAmAmAm | NO.355 | UmsUfsUmCmAmGfGmAmCmAmCmCmAmGfAmCfUmUmCmsUmsCm | NO.608 |
| RX008032 | CmsUmsGmGmUmGmUfCfCfUmGmAmAmAmCmUmGmCmAm | NO.356 | UmsGfsCmAmGmUfUmUmCmAmGmGmAmCfAmCfCmAmGmsAmsCm | NO.609 |
| RX008033 | GmsUmsGmUmCmCmUfGfAfAmAmCmUmGmCmAmAmCmUm | NO.357 | AmsGfsUmUmGmCfAmGmUmUmUmCmAmGfGmAfCmAmCmsCmsAm | NO.610 |
| RX008034 | GmsAmsAmGmGmCmAfAfCfAmGmCmAmCmAmGmUmUmAm | NO.358 | UmsAfsAmCmUmGfUmGmCmUmGmUmUmGfCmCfUmUmCmsUmsAm | NO.611 |
| RX008035 | CmsAmsAmCmAmGmCfAfCfAmGmUmUmAmCmUmGmGmAm | NO.359 | UmsCfsCmAmGmUfAmAmCmUmGmUmGmCfUmGfUmUmGmsCmsCm | NO.612 |

(continued)

| Serial number | Modified sense strand (5'-3') | SEQ ID | Modified antisense strand (5'-3') | SEQ ID |
|---|---|---|---|---|
| RX008036 | GmsCmsGmGmCmUmCfUfUfGmGmAmCmAmCmAmGmCmAm | NO.360 | UmsGfsCmUmGmUfGmUmCmCmAmAmGmAfGmCfCmGmCmsCmsUm | NO.613 |
| RX008037 | GmsCmsAmGmCmCmCfUfUfCmCmUmAmGmAmGmCmUmUm | NO.361 | AmsAfsGmCmUmCfUmAmGmGmAmAmGmGfGmCfUmGmCmsUmsGm | NO.614 |
| RX008038 | AmsGmsCmCmCmUmUfCfCfUmAmGmAmGmCmUmUmAmAm | NO.362 | UmsUfsAmAmGmCfUmCmUmAmGmGmAmAfGmGfGmCmUmsGmsCm | NO.615 |
| RX008039 | CmsCmsUmUmCmCmUfAfGfAmGmCmUmUmAmAmGmAmUm | NO.363 | AmsUfsCmUmUmAfAmGmCmUmCmUmAmGfGmAfAmGmGmsGmsCm | NO.616 |
| RX008040 | CmsCmsUmAmGmAmGfCfUfUmAmAmGmAmUmCmCmGmAm | NO.364 | UmsCfsGmGmAmUfCmUmUmAmAmGmCmUfCmUfAmGmGmsAmsAm | NO.617 |
| RX008041 | GmsAmsGmCmUmUmAfAfGfAmUmCmCmGmAmGmCmCmAm | NO.365 | UmsGfsGmCmUmCfGmGmAmUmCmUmUmAfAmGfCmUmCmsUmsAm | NO.618 |
| RX008042 | GmsUmsUmGmCmAmGfGfCfGmAmGmAmCmCmAmUmUmAm | NO.366 | UmsAfsAmUmGmGfUmCmUmCmGmCmCmUfGmCfAmAmCmsAmsUm | NO.619 |
| RX008043 | GmsCmsGmAmGmAmCfCfAfUmUmAmCmGmUmAmGmAmCm | NO.367 | GmsUfsCmUmAmCfGmUmAmAmUmGmGmUfCmUfCmGmCmsCmsUm | NO.620 |
| RX008044 | CmsGmsAmGmAmCmCfAfUfUmAmCmGmUmAmGmAmCmUm | NO.368 | AmsGfsUmCmUmAfCmGmUmAmAmUmGmGfUmCfUmCmGmsCmsCm | NO.621 |
| RX008045 | GmsAmsUmGmGmCmGfGfGfAmCmUmGmGmAmUmAmCmUm | NO.369 | AmsGfsUmAmUmCfCmAmGmUmCmCmCmGfCmCfAmUmCmsCmsCm | NO.622 |
| RX008046 | CmsCmsAmGmGmCmAfUfUfGmCmUmGmCmCmUmCmUmUm | NO.370 | AmsAfsGmAmGmGfCmAmGmCmAmAmUmGfCmCfUmGmGmsGmsCm | NO.623 |

(continued)

| Serial number | Modified sense strand (5'-3') | SEQ ID | Modified antisense strand (5'-3') | SEQ ID |
|---|---|---|---|---|
| RX008047 | CmsCmsUmCmUmUmUfCfCfAmUmUmCmUmGmCmCmGmUm | NO.371 | AmsCfsGmGmCmAfGmAmAmUmGmGmAmAfAmGfAmGmGmsCmsAm | NO.624 |
| RX008048 | CmsCmsGmUmCmUmUfCfAfGmCmCmUmCmCmUmCmAmAm | NO.372 | UmsUfsGmAmGmGfAmGmGmCmUmGmAmAfGmAfCmGmGmsCmsAm | NO.625 |
| RX008049 | GmsCmsCmUmCmCmUfCfAfAmAmGmCmCmAmAmCmAmAm | NO.373 | UmsUfsGmUmUmGfGmCmUmUmUmGmAmGfGmAfGmGmCmsUmsGm | NO.626 |
| RX008050 | CmsCmsUmCmCmUmCfAfAfAmGmCmCmAmAmCmAmAmUm | NO.374 | AmsUfsUmGmUmUfGmGmCmUmUmUmGmAfGmGfAmGmGmsCmsUm | NO.627 |
| RX008051 | CmsUmsCmCmUmGmUfUfGfUmGmUmCmCmCmUmAmCmUm | NO.375 | AmsGfsUmAmGmGfGmAmCmAmCmAmAmCfAmGfGmAmGmsGmsUm | NO.628 |
| RX008052 | CmsUmsCmUmCmCmUfCfUfAmCmCmUmGmGmAmUmCmAm | NO.376 | UmsGfsAmUmCmCfAmGmGmUmAmGmAmGfGmAfGmAmGmsAmsGm | NO.629 |
| RX008053 | CmsCmsUmCmUmAmCfCfUfGmGmAmUmCmAmUmAmAmUm | NO.377 | AmsUfsUmAmUmGfAmUmCmCmAmGmGmUfAmGfAmGmGmsAmsGm | NO.630 |
| RX008054 | CmsCmsUmGmGmAmUfCfAfUmAmAmUmGmGmCmAmAmUm | NO.378 | AmsUfsUmGmCmCfAmUmUmAmUmGmAmUfCmCfAmGmGmsUmsAm | NO.631 |
| RX008055 | CmsAmsUmAmAmUmGfGfCfAmAmUmGmUmGmGmUmCmAm | NO.379 | UmsGfsAmCmCmAfCmAmUmUmGmCmCmAfUmUfAmUmGmsAmsUm | NO.632 |
| RX008056 | GmsCmsAmAmUmGmUfGfGfUmCmAmAmGmAmCmGmGmAm | NO.380 | UmsCfsCmGmUmCfUmUmGmAmCmCmAmCfAmUfUmGmCmsCmsAm | NO.633 |
| RX008057 | GmsGmsUmCmAmAmGfAfCfGmGmAmUmGmUmGmCmCmAm | NO.381 | UmsGfsGmCmAmCfAmUmCmCmGmUmCmUfUmGfAmCmCmsAmsCm | NO.634 |

(continued)

| Serial number | Modified sense strand (5'-3') | SEQ ID | Modified antisense strand (5'-3') | SEQ ID |
|---|---|---|---|---|
| RX008058 | CmsAmsAmGmAmCmGfGfAfUmGmUmGmCmCmAmGmAmUm | NO.382 | AmsUfsCmUmGmGfCmAmCmAmUmCmCmGfUmCfUmUmGmsAmsCm | NO.635 |
| RX008059 | GmsCmsUmGmCmAmGfCfUfAmGmCmAmAmGmAmGmGmAm | NO.383 | UmsCfsCmUmCmUfUmGmCmUmAmGmCmUfGmCfAmGmCmsCmsAm | NO.636 |
| RX008060 | GmsCmsAmGmCmUmAfGfCfAmAmGmAmGmGmAmCmCmUm | NO.384 | AmsGfsGmUmCmCfUmCmUmUmGmCmUmAfGmCfUmGmCmsAmsGm | NO.637 |
| RX008061 | GmsGmsAmGmUmGmAfAfGfAmGmAmCmCmAmAmGmAmUm | NO.385 | AmsUfsCmUmUmGfGmUmCmUmCmUmUmCfAmCfUmCmCmsAmsAm | NO.638 |
| RX008062 | AmsGmsUmGmAmAmGfAfGfAmCmCmAmAmGmAmUmGmAm | NO.386 | UmsCfsAmUmCmUfUmGmGmUmCmUmCmUfUmCfAmCmUmsCmsCm | NO.639 |
| RX008063 | GmsUmsGmAmAmGmAfGfAfCmCmAmAmGmAmUmGmAmAm | NO.387 | UmsUfsCmAmUmCfUmUmGmGmUmCmUmCfUmUfCmAmCmsUmsCm | NO.640 |
| RX008064 | GmsCmsAmUmCmUmGfUfGfAmCmUmGmGmAmGmGmCmAm | NO.388 | UmsGfsCmCmUmCfCmAmGmUmCmAmCmAfGmAfUmGmCmsCmsCm | NO.641 |
| RX008065 | CmsUmsGmGmAmGmGfCfAfUmCmAmGmAmUmUmCmCmUm | NO.389 | AmsGfsGmAmAmUfCmUmGmAmUmGmCmCfUmCfCmAmGmsUmsCm | NO.642 |
| RX008066 | GmsCmsAmUmCmAmGfAfUfUmCmCmUmGmAmUmCmCmAm | NO.390 | UmsGfsGmAmUmCfAmGmGmAmAmUmCmUfGmAfUmGmCmsCmsUm | NO.643 |
| RX008067 | CmsCmsCmAmAmCmAfAfCfCmAmCmCmUmGmGmCmAmAm | NO.391 | UmsUfsGmCmCmAfGmGmUmGmGmUmUmGfUmUfGmGmGmsUmsUm | NO.644 |
| RX008068 | CmsCmsAmAmCmAmAfCfCfAmCmCmUmGmGmCmAmAmUm | NO.392 | AmsUfsUmGmCmCfAmGmGmUmGmGmUmUfGmUfUmGmGmsGmsUm | NO.645 |

(continued)

| Serial number | Modified sense strand (5'-3') | SEQ ID | Modified antisense strand (5'-3') | SEQ ID |
|---|---|---|---|---|
| RX008069 | CmsAmsCmCmUmGmGfCfAfAmUmAmUmGmAmCmUmCmAm | NO.393 | UmsGfsAmGmUmCfAmUmAmUmUmGmCmCfAmGfGmUmGmsGmsUm | NO.646 |
| RX008070 | CmsCmsUmGmGmCmAfAfUfAmUmGmAmCmUmCmAmCmUm | NO.394 | AmsGfsUmGmAmGfUmCmAmUmAmUmUmGfCmCfAmGmGmsUmsGm | NO.647 |
| RX008071 | CmsUmsGmGmCmAmAfUfAfUmGmAmCmUmCmAmCmUmUm | NO.395 | AmsAfsGmUmGmAfGmUmCmAmUmAmUmUfGmCfCmAmGmsGmsUm | NO.648 |
| RX008072 | GmsGmsGmAmCmCmCfAfAfAmUmGmGmGmCmAmCmUmUm | NO.396 | AmsAfsGmUmGmCfCmCmAmUmUmUmGmGfGmUfCmCmCmsAmsUm | NO.649 |
| RX008073 | GmsGmsAmCmCmCmAfAfAfUmGmGmGmCmAmCmUmUmUm | NO.397 | AmsAfsAmGmUmGfCmCmCmAmUmUmUmGfGmGfUmCmCmsCmsAm | NO.650 |
| RX008074 | CmsCmsCmAmAmAmUfGfGfGmCmAmCmUmUmUmCmUmUm | NO.398 | AmsAfsGmAmAmAfGmUmGmCmCmCmAmUfUmUfGmGmGmsUmsCm | NO.651 |
| RX008075 | CmsAmsAmAmUmGmGfGfCfAmCmUmUmUmCmUmUmGmUm | NO.399 | AmsCfsAmAmGmAfAmAmGmUmGmCmCmCfAmUfUmUmGmsGmsGm | NO.652 |
| RX008076 | GmsGmsCmAmCmUmUfUfCfUmUmGmUmCmUmGmAmGmAm | NO.400 | UmsCfsUmCmAmGfAmCmAmAmGmAmAmAfGmUfGmCmCmsCmsAm | NO.653 |
| RX008077 | CmsUmsCmUmGmGmCfUfUfAmUmUmCmCmAmGmGmUmUm | NO.401 | AmsAfsCmCmUmGfGmAmAmUmAmAmGmCfCmAfGmAmGmsUmsCm | NO.654 |
| RX008078 | GmsUmsAmAmAmGmCfGfUfUmUmCmUmUmCmUmAmAmAm | NO.402 | UmsUfsUmAmGmAfAmGmAmAmAmCmGmCfUmUfUmAmCmsCmsCm | NO.655 |
| RX008079 | CmsCmsAmGmAmAmAfGfCfAmUmGmAmUmUmUmCmCmUm | NO.403 | AmsGfsGmAmAmAfUmCmAmUmGmCmUmUfUmCfUmGmGmsGmsUm | NO.656 |

(continued)

| Serial number | Modified sense strand (5'-3') | SEQ ID | Modified antisense strand (5'-3') | SEQ ID |
|---|---|---|---|---|
| RX008080 | GmsCmsCmCmUmAmAfGfUfCmCmUmGmUmGmAmGmAmAm | NO.404 | UmsUfsCmUmCmAfCmAmGmGmAmCmUmUfAmGfGmGmCmsAmsGm | NO.657 |
| RX008081 | CmsCmsUmAmAmGmUfCfCfUmGmUmGmAmGmAmAmGmAm | NO.405 | UmsCfsUmUmCmUfCmAmCmAmGmGmAmCfUmUfAmGmGmsGmsCm | NO.658 |
| RX008082 | GmsAmsGmAmAmGmAfUfGfUmCmAmGmGmGmAmCmUmAm | NO.406 | UmsAfsGmUmCmCfCmUmGmAmCmAmUmCfUmUfCmUmCmsAmsCm | NO.659 |
| RX008083 | GmsGmsGmAmGmGmGfAfAfGmGmCmAmGmAmGmAmAmAm | NO.407 | UmsUfsUmCmUmCfUmGmCmCmUmUmCmCfCmUfCmCmCmsUmsCm | NO.660 |
| RX008084 | CmsCmsUmCmUmCmCfCfAfAmGmAmUmGmAmGmAmAmAm | NO.408 | UmsUfsUmCmUmCfAmUmCmUmUmGmGmGfAmGfAmGmGmsCmsUm | NO.661 |
| RX008085 | CmsCmsCmAmAmGmAfUfGfAmGmAmAmAmGmUmCmCmUm | NO.409 | AmsGfsGmAmCmUfUmUmCmUmCmAmUmCfUmUfGmGmGmsAmsGm | NO.662 |
| RX008086 | CmsAmsAmGmAmUmGfAfGfAmAmAmGmUmCmCmUmCmAm | NO.410 | UmsGfsAmGmGmAfCmUmUmUmCmUmCmAfUmCfUmUmGmsGmsGm | NO.663 |
| RX008087 | GmsGmsAmGmGmAmGfGfAfAmGmCmAmGmAmUmAmGmAm | NO.411 | UmsCfsUmAmUmCfUmGmCmUmUmCmCmUfCmCfUmCmCmsCmsCm | NO.664 |
| RX008088 | GmsAmsAmGmCmAmGfAfUfAmGmAmUmGmGmUmCmCmAm | NO.412 | UmsGfsGmAmCmCfAmUmCmUmAmUmCmUfGmCfUmUmCmsCmsUm | NO.665 |
| RX008089 | GmsCmsAmGmAmUmAfGfAfUmGmGmUmCmCmAmGmCmAm | NO.413 | UmsGfsCmUmGmGfAmCmCmAmUmCmUmAfUmCfUmGmCmsUmsUm | NO.666 |
| RX008090 | GmsCmsAmGmGmCmUfUfGfAmAmGmCmAmGmGmGmUmAm | NO.414 | UmsAfsCmCmCmUfGmCmUmUmCmAmAmGfCmCfUmGmCmsUmsGm | NO.667 |

(continued)

| Serial number | Modified sense strand (5'-3') | SEQ ID | Modified antisense strand (5'-3') | SEQ ID |
|---|---|---|---|---|
| RX008091 | GmsGmsUmAmAmGmGfGfCfUmGmUmUmGmAmGmGmUmAm | NO.415 | UmsAfsCmCmUmCfAmAmCmAmGmCmCmCfUmUfAmCmCmsCmsUm | NO.668 |
| RX008092 | GmsGmsCmUmGmUmUfGfAfGmGmUmAmCmCmUmUmAmAm | NO.416 | UmsUfsAmAmGmGfUmAmCmCmUmCmAmAfCmAfGmCmCmsCmsUm | NO.669 |
| RX008093 | CmsCmsUmUmAmAmGfGfGfAmAmGmGmUmCmAmAmGmAm | NO.417 | UmsCfsUmUmGmAfCmCmUmUmCmCmCmUfUmAfAmGmGmsUmsAm | NO.670 |
| RX008094 | AmsAmsGmGmGmAmAfGfGfUmCmAmAmGmAmGmGmGmAm | NO.418 | UmsCfsCmCmUmCfUmUmGmAmCmCmUmUfCmCfCmUmUmsAmsAm | NO.671 |
| RX008095 | GmsGmsAmAmGmGmUfCfAfAmGmAmGmGmGmAmGmAmUm | NO.419 | AmsUfsCmUmCmCfCmUmCmUmUmGmAmCfCmUfUmCmCmsCmsUm | NO.672 |
| RX008096 | UmsCmsAmAmGmAmGfGfGfAmGmAmUmGmGmGmCmAmAm | NO.420 | UmsUfsGmCmCmCfAmUmCmUmCmCmCmUfCmUfUmGmAmsCmsCm | NO.673 |
| RX008097 | GmsCmsGmCmUmGmAfGfGfGmAmGmGmAmUmGmCmUmUm | NO.421 | AmsAfsGmCmAmUfCmCmUmCmCmCmUmCfAmGfCmGmCmsCmsUm | NO.674 |
| RX008098 | CmsGmsCmUmGmAmGfGfGfAmGmGmAmUmGmCmUmUmAm | NO.422 | UmsAfsAmGmCmAfUmCmCmUmCmCmCmUfCmAfGmCmGmsCmsCm | NO.675 |
| RX008099 | AmsGmsAmAmAmCmAfGfGfAmGmUmCmAmGmGmAmAmAm | NO.423 | UmsUfsUmCmCmUfGmAmCmUmCmCmUmGfUmUfUmCmUmsGmsGm | NO.676 |
| RX008100 | GmsAmsAmAmCmAmGfGfAfGmUmCmAmGmGmAmAmAmAm | NO.424 | UmsUfsUmUmCmCfUmGmAmCmUmCmCmUfGmUfUmUmCmsUmsGm | NO.677 |
| RX008101 | GmsAmsGmGmCmAmCfUfAfAmGmCmCmUmAmAmGmAmAm | NO.425 | UmsUfsCmUmUmAfGmGmCmUmUmAmGmUfGmCfCmUmCmsAmsUm | NO.678 |

(continued)

| Serial number | Modified sense strand (5'-3') | SEQ ID | Modified antisense strand (5'-3') | SEQ ID |
|---|---|---|---|---|
| RX008102 | GmsCmsAmCmUmAmAfGfCfCmUmAmAmGmAmAmGmUmUm | NO.426 | AmsAfsCmUmUmCfUmUmAmGmGmCmUmUfAmGfUmGmCmsCmsUm | NO.679 |
| RX008103 | CmsCmsUmAmAmGmAfAfGfUmUmCmCmCmUmGmGmUmUm | NO.427 | AmsAfsCmCmAmGfGmGmAmAmCmUmUmCfUmUfAmGmGmsCmsUm | NO.680 |
| RX008104 | CmsAmsCmUmAmAmUfUfUfUmUmGmUmAmUmUmCmUmUm | NO.428 | AmsAfsGmAmAmUfAmCmAmAmAmAmAmUfUmAfGmUmGmsGmsGm | NO.681 |
| RX008105 | GmsAmsGmAmAmAmGfAfAfAmAmUmCmAmAmCmAmAmAm | NO.429 | UmsUfsUmGmUmUfGmAmUmUmUmUmCmUfUmUfCmUmCmsCmsUm | NO.682 |
| RX008106 | AmsAmsUmCmAmAmCfAfAfAmUmGmUmGmAmGmUmCmAm | NO.430 | UmsGfsAmCmUmCfAmCmAmUmUmUmGmUfUmGfAmUmUmsUmsUm | NO.683 |
| RX008107 | CmsAmsAmCmAmAmAfUfGfUmGmAmGmUmCmAmUmAmAm | NO.431 | UmsUfsAmUmGmAfCmUmCmAmCmAmUmUfUmGfUmUmGmsAmsUm | NO.684 |
| RX008108 | CmsAmsAmAmUmGmUfGfAfGmUmCmAmUmAmAmAmGmAm | NO.432 | UmsCfsUmUmUmAfUmGmAmCmUmCmAmCfAmUfUmUmGmsUmsUm | NO.685 |
| RX008109 | AmsAmsAmUmGmUmGfAfGfUmCmAmUmAmAmAmGmAmAm | NO.433 | UmsUfsCmUmUmUfAmUmGmAmCmUmCmAfCmAfUmUmUmsGmsUm | NO.686 |
| RX008110 | GmsUmsGmAmGmUmCfAfUfAmAmAmGmAmAmGmGmGmUm | NO.434 | AmsCfsCmCmUmUfCmUmUmUmAmUmGmAfCmUfCmAmCmsAmsUm | NO.687 |
| RX008111 | GmsAmsGmUmCmAmUfAfAfAmGmAmAmGmGmGmUmUmAm | NO.435 | UmsAfsAmCmCmCfUmUmCmUmUmUmAmUfGmAfCmUmCmsAmsCm | NO.688 |
| RX008112 | UmsAmsAmAmGmAmAfGfGfGmUmUmAmGmGmGmUmGmAm | NO.436 | UmsCfsAmCmCmCfUmAmAmCmCmCmUmUfCmUfUmUmAmsUmsGm | NO.689 |

(continued)

| Serial number | Modified sense strand (5'-3') | SEQ ID | Modified antisense strand (5'-3') | SEQ ID |
|---|---|---|---|---|
| RX008113 | GmsGmsGmUmUmAmGfGfGfUmGmAmUmGmGmUmCmCmAm | NO.437 | UmsGfsGmAmCmCfAmUmCmAmCmCmCmUfAmAfCmCmCmsUmsUm | NO.690 |
| RX008114 | GmsUmsUmAmGmGmGfUfGfAmUmGmGmUmCmCmAmGmAm | NO.438 | UmsCfsUmGmGmAfCmCmAmUmCmAmCmCfCmUfAmAmCmsCmsCm | NO.691 |
| RX008115 | GmsGmsGmUmGmAmUfGfGfUmCmCmAmGmAmGmCmAmAm | NO.439 | UmsUfsGmCmUmCfUmGmGmAmCmCmAmUfCmAfCmCmCmsUmsAm | NO.692 |
| RX008116 | GmsUmsCmCmAmGmAfGfCfAmAmCmAmGmUmUmCmUmUm | NO.440 | AmsAfsGmAmAmCfUmGmUmUmGmCmUmCfUmGfGmAmCmsCmsAm | NO.693 |
| RX008117 | CmsAmsGmAmGmCmAfAfCfAmGmUmUmCmUmUmCmAmAm | NO.441 | UmsUfsGmAmAmGfAmAmCmUmGmUmUmGfCmUfCmUmGmsGmsAm | NO.694 |
| RX008118 | GmsAmsGmCmAmAmCfAfGfUmUmCmUmUmCmAmAmGmUm | NO.442 | AmsCfsUmUmGmAfAmGmAmAmCmUmGmUfUmGfCmUmCmsUmsGm | NO.695 |
| RX008119 | GmsCmsAmAmCmAmGfUfUfCmUmUmCmAmAmGmUmGmUm | NO.443 | AmsCfsAmCmUmUfGmAmAmGmAmAmCmUfGmUfUmGmCmsUmsCm | NO.696 |
| RX008120 | GmsUmsCmCmAmCmAfAfAfGmUmCmAmAmAmGmCmUmAm | NO.444 | UmsAfsGmCmUmUfUmGmAmCmUmUmUmGfUmGfGmAmCmsAmsCm | NO.697 |
| RX008121 | UmsCmsCmAmCmAmAfAfGfUmCmAmAmAmGmCmUmAmUm | NO.445 | AmsUfsAmGmCmUfUmUmGmAmCmUmUmUfGmUfGmGmAmsCmsAm | NO.698 |
| RX008122 | CmsCmsAmCmAmAmAfGfUfCmAmAmAmGmCmUmAmUmUm | NO.446 | AmsAfsUmAmGmCfUmUmUmGmAmCmUmUfUmGfUmGmGmsAmsCm | NO.699 |
| RX008123 | CmsAmsCmAmAmAmGfUfCfAmAmAmGmCmUmAmUmUmUm | NO.447 | AmsAfsAmUmAmGfCmUmUmUmGmAmCmUfUmUfGmUmGmsGmsAm | NO.700 |

(continued)

| Serial number | Modified sense strand (5'-3') | SEQ ID | Modified antisense strand (5'-3') | SEQ ID |
|---|---|---|---|---|
| RX008124 | CmsAmsAmAmGmCmUfAfUfUmUmUmCmAmUmAmAmUmAm | NO.448 | UmsAfsUmUmAmUfGmAmAmAmAmUmAmGfCmUfUmUmGmsAmsCm | NO.701 |
| RX008125 | CmsUmsAmUmUmUmUfCfAfUmAmAmUmAmAmUmAmCmUm | NO.449 | AmsGfsUmAmUmUfAmUmUmAmUmGmAmAfAmAfUmAmGmsCmsUm | NO.702 |
| RX008126 | CmsAmsUmAmAmUmAfAfUfAmCmUmAmAmCmAmUmGmUm | NO.450 | AmsCfsAmUmGmUfUmAmGmUmAmUmUmAfUmUfAmUmGmsAmsAm | NO.703 |
| RX008127 | CmsAmsUmGmUmUmAfUfUfUmGmCmCmUmUmUmUmGmAm | NO.451 | UmsCfsAmAmAmAfGmGmCmAmAmAmUmAfAmCfAmUmGmsUmsUm | NO.704 |
| RX008128 | GmsUmsUmAmUmUmUfGfCfCmUmUmUmUmGmAmAmUmUm | NO.452 | AmsAfsUmUmCmAfAmAmAmGmGmCmAmAfAmUfAmAmCmsAmsUm | NO.705 |
| RX008129 | CmsAmsGmCmAmCmAfGfUfUmAmCmUmGmGmAmCmAmAm | NO.453 | UmsUfsGmUmCmCfAmGmUmAmAmCmUmGfUmGfCmUmGmsUmsUm | NO.706 |
| RX008130 | GmsCmsUmUmAmAmGfAfUfCmCmGmAmGmCmCmAmAmUm | NO.454 | AmsUfsUmGmGmCfUmCmGmGmAmUmCmUfUmAfAmGmCmsUmsCm | NO.707 |
| RX008131 | CmsCmsAmGmCmUmGfAfAfUmUmAmCmUmGmCmAmGmUm | NO.455 | AmsCfsUmGmCmAfGmUmAmAmUmUmCmAfGmCfUmGmGmsUmsAm | NO.708 |
| RX008132 | CmsUmsCmAmAmAmGfCfCfAmAmCmAmAmUmCmCmUmUm | NO.456 | AmsAfsGmGmAmUfUmGmUmUmGmGmCmUfUmUfGmAmGmsGmsAm | NO.709 |
| RX008133 | GmsGmsAmGmGmCmAfUfCfAmGmAmUmUmCmCmUmGmAm | NO.457 | UmsCfsAmGmGmAfAmUmCmUmGmAmUmGfCmCfUmCmCmsAmsGm | NO.710 |
| RX008134 | GmsGmsCmAmAmUmAfUfGfAmCmUmCmAmCmUmUmGmAm | NO.458 | UmsCfsAmAmGmUfGmAmGmUmCmAmUmAfUmUfGmCmCmsAmsGm | NO.711 |

(continued)

| Serial number | Modified sense strand (5'-3') | SEQ ID | Modified antisense strand (5'-3') | SEQ ID |
|---|---|---|---|---|
| RX008135 | GmsCmsCmUmCmUmCfCfCfAmAmGmAmUmGmAmGmAmAm | NO.459 | UmsUfsCmUmCmAfUmCmUmUmGmGmGmAfGmAfGmGmCmsUmsAm | NO.712 |
| RX008136 | CmsAmsGmGmCmUmUfGfAfAmGmCmAmGmGmGmUmAmAm | NO.460 | UmsUfsAmCmCmCfUmGmCmUmUmCmAmAfGmCfCmUmGmsCmsUm | NO.713 |
| RX008137 | GmsCmsUmUmGmAmAfGfCfAmGmGmGmUmAmAmGmCmAm | NO.461 | UmsGfsCmUmUmAfCmCmCmUmGmCmUmUfCmAfAmGmCmsCmsUm | NO.714 |
| RX008138 | CmsUmsAmAmGmAmAfGfUfUmCmCmCmUmGmGmUmUmUm | NO.462 | AmsAfsAmCmCmAfGmGmGmAmAmCmUmUfCmUfUmAmGmsGmsCm | NO.715 |
| RX008139 | CmsCmsCmAmCmUmGfGfGfAmGmAmCmAmAmGmCmAmUm | NO.463 | AmsUfsGmCmUmUfGmUmCmUmCmCmCmAfGmUfGmGmGmsUmsCm | NO.716 |
| RX008140 | CmsAmsCmUmGmGmGfAfGfAmCmAmAmGmCmAmUmUmUm | NO.464 | AmsAfsAmUmGmCfUmUmGmUmCmUmCmCfCmAfGmUmGmsGmsGm | NO.717 |
| RX008141 | GmsAmsGmAmCmAmAfGfCfAmUmUmUmAmUmAmCmUmUm | NO.465 | AmsAfsGmUmAmUfAmAmAmUmGmCmUmUfGmUfCmUmCmsCmsCm | NO.718 |
| RX008142 | CmsAmsAmGmCmAmUfUfUfAmUmAmCmUmUmUmCmUmUm | NO.466 | AmsAfsGmAmAmAfGmUmAmUmAmAmAmUfGmCfUmUmGmsUmsCm | NO.719 |
| RX008143 | CmsCmsGmCmGmCmCfUfGfGmCmUmUmAmUmAmCmUmUm | NO.467 | AmsAfsGmUmAmUfAmAmGmCmCmAmGmGfCmGfCmGmGmsUmsGm | NO.720 |
| RX008144 | GmsCmsCmUmGmGmCfUfUfAmUmAmCmUmUmUmCmUmUm | NO.468 | AmsAfsGmAmAmAfGmUmAmUmAmAmGmCfCmAfGmGmCmsGmsCm | NO.721 |
| RX008145 | GmsCmsUmUmAmUmAfCfUfUmUmCmUmUmAmAmUmAmAm | NO.469 | UmsUfsAmUmUmAfAmGmAmAmAmGmUmAfUmAfAmGmCmsCmsAm | NO.722 |

(continued)

| Serial number | Modified sense strand (5'-3') | SEQ ID | Modified antisense strand (5'-3') | SEQ ID |
|---|---|---|---|---|
| RX008146 | GmsGmsGmUmGmUmCfCfAfCmAmAmAmGmUmCmAmAmAm | NO.470 | UmsUfsUmGmAmCfUmUmUmGmUmGmGmAfCmAfCmCmCmsCmsUm | NO.723 |
| RX008147 | GmsCmsCmUmUmUmUfGfAfAmUmUmCmUmCmAmUmUmAm | NO.471 | UmsAfsAmUmGmAfGmAmAmUmUmCmAmAfAmAfGmGmCmsAmsAm | NO.724 |
| RX008148 | GmsAmsAmUmUmCmUfCfAfUmUmAmUmCmUmUmAmAmAm | NO.472 | UmsUfsUmAmAmGfAmUmAmAmUmGmAmGfAmAfUmUmCmsAmsAm | NO.725 |
| RX008149 | CmsAmsUmUmAmUmCfUfUfAmAmAmAmUmUmGmUmAmUm | NO.473 | AmsUfsAmCmAmAfUmUmUmUmAmAmGmAfUmAfAmUmGmsAmsGm | NO.726 |
| RX008150 | CmsAmsUmGmUmGmAfUfUfAmCmAmUmCmAmUmCmUmUm | NO.474 | AmsAfsGmAmUmGfAmUmGmUmAmAmUmCfAmCfAmUmGmsUmsCm | NO.727 |
| RX008151 | GmsAmsUmUmAmCmAfUfCfAmUmCmUmUmUmCmUmGmAm | NO.475 | UmsCfsAmGmAmAfAmGmAmUmGmAmUmGfUmAfAmUmCmsAmsCm | NO.728 |
| RX008152 | CmsUmsUmUmCmUmGfAfCfAmUmCmAmUmUmGmUmUmAm | NO.476 | UmsAfsAmCmAmAfUmGmAmUmGmUmCmAfGmAfAmAmGmsAmsUm | NO.729 |
| RX008153 | CmsAmsUmUmGmUmUfAfAfUmGmGmAmAmUmGmUmGmUm | NO.477 | AmsCfsAmCmAmUfUmCmCmAmUmUmAmAfCmAfAmUmGmsAmsUm | NO.730 |
| RX008154 | GmsUmsUmAmAmUmGfGfAfAmUmGmUmGmUmGmCmUmUm | NO.478 | AmsAfsGmCmAmCfAmCmAmUmUmCmCmAfUmUfAmAmCmsAmsAm | NO.731 |
| RX008155 | CmsAmsAmGmCmAmGfCfAfAfAmUmCmCmUmGmGmAmUm | NO.479 | AmsUfsCmCmAmGfGmAmUfUmUmGmCmUfG(moe)CfUmUmGmsGmsCm | NO.732 |
| RX008156 | GmsGmsUmCmAmUmCfAfGfCfUmUmUmGmCmUmAmCmUm | NO.480 | AmsGfsUmAmGmCfAmAmAfGmCmUmGmAfU(moe)GfAmCmCmsUmsCm | NO.733 |

(continued)

| Serial number | Modified sense strand (5'-3') | SEQ ID | Modified antisense strand (5'-3') | SEQ ID |
|---|---|---|---|---|
| RX008157 | CmsAmsGmCmUmUmUfGfCfUfAmCmUmGmUmCmAmCmAm | NO.481 | UmsGfsUmGmAmCfAmGmUfAmGmCmAmAfA(moe)GfCmUmGmsAmsUm | NO.734 |
| RX008158 | CmsAmsCmCmCmUmUfCfCfUfGmGmCmAmCmUmCmUmUm | NO.482 | AmsAfsGmAmGmUfGmCmCfAmGmGmAmAfG(moe)GfGmUmGmsGmsGm | NO.735 |
| RX008159 | CmsUmsCmUmUmUmGfCfUfUfGmAmGmGmAmUmCmUmUm | NO.483 | AmsAfsGmAmUmCfCmUmCfAmAmGmCmAfA(moe)AfGmAmGmsUmsGm | NO.736 |
| RX008160 | GmsCmsUmUmGmAmGfGfAfUfCmUmUmCmCmGmAmUmGm | NO.484 | CmsAfsUmCmGmGfAmAmGfAmUmCmCmUfC(moe)AfAmGmCmsAmsAm | NO.737 |
| RX008161 | CmsGmsAmGmAmCmCfAfUfUfAmCmGmUmAmGmAmCmUm | NO.485 | AmsGfsUmCmUmAfCmGmUfAmAmUmGmGfU(moe)CfUmCmGmsCmsCm | NO.738 |
| RX008162 | CmsCmsUmCmCmUmCfAfAfAfGmCmCmAmAmCmAmAmUm | NO.486 | AmsUfsUmGmUmUfGmGmCfUmUmUmGmAfG(moe)GfAmGmGmsCmsUm | NO.739 |
| RX008163 | CmsCmsUmCmUmAmCfCfUfGfGmAmUmCmAmUmAmAmUm | NO.487 | AmsUfsUmAmUmGfAmUmCfCmAmGmGmUfA(moe)GfAmGmGmsAmsGm | NO.740 |
| RX008164 | CmsCmsUmGmGmAmUfCfAfUfAmAmUmGmGmCmAmAmUm | NO.488 | AmsUfsUmGmCmCfAmUmUfAmUmGmAmUfC(moe)CfAmGmGmsUmsAm | NO.741 |
| RX008165 | GmsCmsAmAmUmGmUfGfGfUfCmAmAmGmAmCmGmGmAm | NO.489 | UmsCfsCmGmUmCfUmUmGfAmCmCmAmCfA(moe)UfUmGmCmsCmsAm | NO.742 |
| RX008166 | GmsGmsAmGmUmGmAfAfGfAfGmAmCmCmAmAmGmAmUm | NO.490 | AmsUfsCmUmUmGfGmUmCfUmCmUmUmCfA(moe)CfUmCmCmsAmsAm | NO.743 |
| RX008167 | GmsUmsGmAmAmGmAfGfAfCfCmAmAmGmAmUmGmAmAm | NO.491 | UmsUfsCmAmUmCfUmUmGfGmUmCmUmCfU(moe)UfCmAmCmsUmsCm | NO.744 |

(continued)

| Serial number | Modified sense strand (5'-3') | SEQ ID | Modified antisense strand (5'-3') | SEQ ID |
|---|---|---|---|---|
| RX008168 | GmsCmsAmCmUmAmAfGfCfCfUmAmAmGmAmAmGmUmUm | NO.492 | AmsAfsCmUmUmCfUmUmAfGmGmCmUmUfA(moe)GfUmGmCmsCmsUm | NO.745 |
| RX008169 | CmsAmsCmUmAmAmUfUfUfUfUmGmUmAmUmUmCmUmUm | NO.493 | AmsAfsGmAmAmUfAmCmAfAmAmAmAmUfU(moe)AfGmUmGmsGmsGm | NO.746 |
| RX008170 | UmsCmsCmAmCmAmAfAfGfUfCmAmAmAmGmCmUmAmUm | NO.494 | AmsUfsAmGmCmUfUmUmGfAmCmUmUmUfG(moe)UfGmGmAmsCmsAm | NO.747 |
| RX008171 | CmsCmsAmCmAmAmAfGfUfCfAmAmAmGmCmUmAmUmUm | NO.495 | AmsAfsUmAmGmCfUmUmUfGmAmCmUmUfU(moe)GfUmGmGmsAmsCm | NO.748 |
| RX008172 | CmsAmsCmAmAmAmGfUfCfAfAmAmGmCmUmAmUmUmUm | NO.496 | AmsAfsAmUmAmGfCmUmUfUmGmAmCmUfU(moe)UfGmUmGmsGmsAm | NO.749 |
| RX008173 | CmsAmsAmAmGmCmUfAfUfUfUmUmCmAmUmAmAmUmAm | NO.497 | UmsAfsUmUmAmUfGmAmAfAmAmUmAmGfC(moe)UfUmUmGmsAmsCm | NO.750 |
| RX008174 | CmsUmsAmUmUmUmUfCfAfUfAmAmUmAmAmUmAmCmUm | NO.498 | AmsGfsUmAmUmUfAmUmUfAmUmGmAmAfA(moe)AfUmAmGmsCmsUm | NO.751 |
| RX008175 | CmsAmsUmAmAmUmAfAfUfAfCmUmAmAmCmAmUmGmUm | NO.499 | AmsCfsAmUmGmUfUmAmGfUmAmUmUmAfU(moe)UfAmUmGmsAmsAm | NO.752 |
| RX008176 | CmsAmsUmGmUmUmAfUfUfUfGmCmCmUmUmUmUmGmAm | NO.500 | UmsCfsAmAmAmAfGmGmCfAmAmAmUmAfA(moe)CfAmUmGmsUmsUm | NO.753 |
| RX008177 | GmsUmsUmAmUmUmUfGfCfCfUmUmUmUmGmAmAmUmUm | NO.501 | AmsAfsUmUmCmAfAmAmAfGmGmCmAmAfA(moe)UfAmAmCmsAmsUm | NO.754 |
| RX008178 | GmsGmsAmGmGmCmAfUfCfAfGmAmUmUmCmCmUmGmAm | NO.502 | UmsCfsAmGmGmAfAmUmCfUmGmAmUmGfC(moe)CfUmCmCmsAmsGm | NO.755 |

(continued)

| Serial number | Modified sense strand (5'-3') | SEQ ID | Modified antisense strand (5'-3') | SEQ ID |
|---|---|---|---|---|
| RX008179 | CmsAmsCmUmGmGmGfAfGfAfCmAmAmGmCmAmUmUmUm | NO.503 | AmsAfsAmUmGmCfUmUmGfUmCmUmCmCfC(moe)AfGmUmGmsGmsGm | NO.756 |
| RX008180 | CmsAmsAmGmCmAmUfUfUfAfUmAmCmUmUmUmCmUmUm | NO.504 | AmsAfsGmAmAmAfGmUmAfUmAmAmAmUfG(moe)CfUmUmGmsUmsCm | NO.757 |
| RX008181 | GmsCmsCmUmGmGmCfUfUfAfUmAmCmUmUmUmCmUmUm | NO.505 | AmsAfsGmAmAmAfGmUmAfUmAmAmGmCfC(moe)AfGmGmCmsGmsCm | NO.758 |
| RX008182 | GmsCmsUmUmAmUmAfCfUfUfUmCmUmUmAmAmUmAmAm | NO.506 | UmsUfsAmUmUmAfAmGmAfAmAmGmUmAfU(moe)AfAmGmCmsCmsAm | NO.759 |
| RX008183 | GmsGmsGmUmGmUmCfCfAfCfAmAmAmGmUmCmAmAmAm | NO.507 | UmsUfsUmGmAmCfUmUmUfGmUmGmGmAfC(moe)AfCmCmCmsCmsUm | NO.760 |
| RX008184 | GmsCmsCmUmUmUmUfGfAfAfUmUmCmUmCmAmUmUmAm | NO.508 | UmsAfsAmUmGmAfGmAmAfUmUmCmAmAfA(moe)AfGmGmCmsAmsAm | NO.761 |
| RX008185 | GmsAmsAmUmUmCmUfCfAfUfUmAmUmCmUmUmAmAmAm | NO.509 | UmsUfsUmAmAmGfAmUmAfAmUmGmAmGfA(moe)AfUmUmCmsAmsAm | NO.762 |
| RX008186 | CmsAmsUmUmAmUmCfUfUfAfAmAmAmUmUmGmUmAmUm | NO.510 | AmsUfsAmCmAmAfUmUmUfUmAmAmGmAfU(moe)AfAmUmGmsAmsGm | NO.763 |
| RX008187 | GmsAmsUmUmAmCmAfUfCfAfUmCmUmUmUmCmUmGmAm | NO.511 | UmsCfsAmGmAmAfAmGmAfUmGmAmUmGfU(moe)AfAmUmCmsAmsCm | NO.764 |
| RX008188 | CmsAmsAmGmCmAmGfCfAfAfAmUmCmCmUmGmGmAmUm | NO.512 | AmsUfsCmCmAmGfGmAmUmUmUmGfCmUfG(moe)CfUmUmGmsGmsCm | NO.765 |
| RX008189 | GmsGmsUmCmAmUmCfAfGfCfUmUmUmGmCmUmAmCmUm | NO.513 | AmsGfsUmAmGmCfAmAmAmGmCmUfGmAfU(moe)GfAmCmCmsUmsCm | NO.766 |

(continued)

| Serial number | Modified sense strand (5'-3') | SEQ ID | Modified antisense strand (5'-3') | SEQ ID |
|---|---|---|---|---|
| RX008190 | CmsAmsGmCmUmUmUfGfCfUfAmCmUmGmUmCmAmCmAm | NO.514 | UmsGfsUmGmAmCfAmGmUmAmGmCfAmAfA(moe)GfCmUmGmsAmsUm | NO.767 |
| RX008191 | CmsAmsCmCmCmUmUfCfCfUfGmGmCmAmCmUmCmUmUm | NO.515 | AmsAfsGmAmGmUfGmCmCmAmGmGfAmAfG(moe)GfGmUmGmsGmsGm | NO.768 |
| RX008192 | CmsUmsCmUmUmUmGfCfUfUfGmAmGmGmAmUmCmUmUm | NO.516 | AmsAfsGmAmUmCfCmUmCmAmAmGfCmAfA(moe)AfGmAmGmsUmsGm | NO.769 |
| RX008193 | GmsCmsUmUmGmAmGfGfAfUfCmUmUmCmCmGmAmUmGm | NO.517 | CmsAfsUmCmGmGfAmAmGmAmUmCfCmUfC(moe)AfAmGmCmsAmsAm | NO.770 |
| RX008194 | CmsGmsAmGmAmCmCfAfUfUfAmCmGmUmAmGmAmCmUm | NO.518 | AmsGfsUmCmUmAfCmGmUmAmAmUfGmGfU(moe)CfUmCmGmsCmsCm | NO.771 |
| RX008195 | CmsCmsUmCmCmUmCfAfAfAfGmCmCmAmAmCmAmAmUm | NO.519 | AmsUfsUmGmUmUfGmGmCmUmUmUfGmAfG(moe)GfAmGmGmsCmsUm | NO.772 |
| RX008196 | CmsCmsUmCmUmAmCfCfUfGfGmAmUmCmAmUmAmAmUm | NO.520 | AmsUfsUmAmUmGfAmUmCmCmAmGfGmUfA(moe)GfAmGmGmsAmsGm | NO.773 |
| RX008197 | CmsCmsUmGmGmAmUfCfAfUfAmAmUmGmGmCmAmAmUm | NO.521 | AmsUfsUmGmCmCfAmUmUmAmUmGfAmUfC(moe)CfAmGmGmsUmsAm | NO.774 |
| RX008198 | GmsCmsAmAmUmGmUfGfGfUfCmAmAmGmAmCmGmGmAm | NO.522 | UmsCfsCmGmUmCfUmUmGmAmCmCfAmCfA(moe)UfUmGmCmsCmsAm | NO.775 |
| RX008199 | GmsGmsAmGmUmGmAfAfGfAfGmAmCmCmAmAmGmAmUm | NO.523 | AmsUfsCmUmUmGfGmUmCmUmCmUfUmCfA(moe)CfUmCmCmsAmsAm | NO.776 |
| RX008200 | GmsUmsGmAmAmGmAfGfAfCfCmAmAmGmAmUmGmAmAm | NO.524 | UmsUfsCmAmUmCfUmUmGmGmUmCfUmCfU(moe)UfCmAmCmsUmsCm | NO.777 |

(continued)

| Serial number | Modified sense strand (5'-3') | SEQ ID | Modified antisense strand (5'-3') | SEQ ID |
|---|---|---|---|---|
| RX008201 | GmsCmsAmCmUmAmAfGfCfCfUmAmAmGmAmAmGmUmUm | NO.525 | AmsAfsCmUmUmCfUmUmAmGmGmCfUmUfA(moe)GfUmGmCmsCmsUm | NO.778 |
| RX008202 | CmsAmsCmUmAmAmUfUfUfUfUmGmUmAmUmUmCmUmUm | NO.526 | AmsAfsGmAmAmUfAmCmAmAmAmAfAmUfU(moe)AfGmUmGmsGmsGm | NO.779 |
| RX008203 | UmsCmsCmAmCmAmAfAfGfUfCmAmAmAmGmCmUmAmUm | NO.527 | AmsUfsAmGmCmUfUmUmGmAmCmUfUmUfG(moe)UfGmGmAmsCmsAm | NO.780 |
| RX008204 | CmsCmsAmCmAmAmAfGfUfCfAmAmAmGmCmUmAmUmUm | NO.528 | AmsAfsUmAmGmCfUmUmUmGmAmCfUmUfU(moe)GfUmGmGmsAmsCm | NO.781 |
| RX008205 | CmsAmsCmAmAmAmGfUfCfAfAmAmGmCmUmAmUmUmUm | NO.529 | AmsAfsAmUmAmGfCmUmUmUmGmAfCmUfU(moe)UfGmUmGmsGmsAm | NO.782 |
| RX008206 | CmsAmsAmAmGmCmUfAfUfUfUmUmCmAmUmAmAmUmAm | NO.530 | UmsAfsUmUmAmUfGmAmAmAmAmUfAmGfC(moe)UfUmUmGmsAmsCm | NO.783 |
| RX008207 | CmsUmsAmUmUmUmUfCfAfUfAmAmUmAmAmUmAmCmUm | NO.531 | AmsGfsUmAmUmUfAmUmUmAmUmGfAmAfA(moe)AfUmAmGmsCmsUm | NO.784 |
| RX008208 | CmsAmsUmAmAmUmAfAfUfAfCmUmAmAmCmAmUmGmUm | NO.532 | AmsCfsAmUmGmUfUmAmGmUmAmUfUmAfU(moe)UfAmUmGmsAmsAm | NO.785 |
| RX008209 | CmsAmsUmGmUmUmAfUfUfUfGmCmCmUmUmUmUmGmAm | NO.533 | UmsCfsAmAmAmAfGmGmCmAmAmAfUmAfA(moe)CfAmUmGmsUmsUm | NO.786 |
| RX008210 | GmsUmsUmAmUmUmUfGfCfCfUmUmUmUmGmAmAmUmUm | NO.534 | AmsAfsUmUmCmAfAmAmAmGmGmCfAmAfA(moe)UfAmAmCmsAmsUm | NO.787 |
| RX008211 | GmsGmsAmGmGmCmAfUfCfAfGmAmUmUmCmCmUmGmAm | NO.535 | UmsCfsAmGmGmAfAmUmCmUmGmAfUmGfC(moe)CfUmCmCmsAmsGm | NO.788 |

(continued)

| Serial number | Modified sense strand (5'-3') | SEQ ID | Modified antisense strand (5'-3') | SEQ ID |
|---|---|---|---|---|
| RX008212 | CmsAmsCmUmGmGmGfAfGfAfCmAmAmGmCmAmUmUmUm | NO.536 | AmsAfsAmUmGmCfUmUmGmUmCmUfCmCfC(moe)AfGmUmGmsGmsGm | NO.789 |
| RX008213 | CmsAmsAmGmCmAmUfUfUfAfUmAmCmUmUmUmCmUmUm | NO.537 | AmsAfsGmAmAmAfGmUmAmUmAmAfAmUfG(moe)CfUmUmGmsUmsCm | NO.790 |
| RX008214 | GmsCmsCmUmGmGmCfUfUfAfUmAmCmUmUmUmCmUmUm | NO.538 | AmsAfsGmAmAmAfGmUmAmUmAmAfGmCfC(moe)AfGmGmCmsGmsCm | NO.791 |
| RX008215 | GmsCmsUmUmAmUmAfCfUfUfUmCmUmUmAmAmUmAmAm | NO.539 | UmsUfsAmUmUmAfAmGmAmAmAmGfUmAfU(moe)AfAmGmCmsCmsAm | NO.792 |
| RX008216 | GmsGmsGmUmGmUmCfCfAfCfAmAmAmGmUmCmAmAmAm | NO.540 | UmsUfsUmGmAmCfUmUmUmGmUmGfGmAfC(moe)AfCmCmCmsCmsUm | NO.793 |
| RX008217 | GmsCmsCmUmUmUmUfGfAfAfUmUmCmUmCmAmUmUmAm | NO.541 | UmsAfsAmUmGmAfGmAmAmUmUmCfAmAfA(moe)AfGmGmCmsAmsAm | NO.794 |
| RX008218 | GmsAmsAmUmUmCmUfCfAfUfUmAmUmCmUmUmAmAmAm | NO.542 | UmsUfsUmAmAmGfAmUmAmAmUmGfAmGfA(moe)AfUmUmCmsAmsAm | NO.795 |
| RX008219 | CmsAmsUmUmAmUmCfUfUfAfAmAmAmUmUmGmUmAmUm | NO.543 | AmsUfsAmCmAmAfUmUmUmUmAmAfGmAfU(moe)AfAmUmGmsAmsGm | NO.796 |
| RX008220 | GmsAmsUmUmAmCmAfUfCfAfUmCmUmUmUmCmUmGmAm | NO.544 | UmsCfsAmGmAmAfAmGmAmUmGmAfUmGfU(moe)AfAmUmCmsAmsCm | NO.797 |
| RX008221 | CmsAmsAmGmCmAmGfCfAfAfAmUmCmCmUmGmGmAmUm | NO.545 | AmsUfsCmCmAmGfGmAmUfUmUmGmCmUfGmCfUmUmGmsGmsCm | NO.798 |
| RX008222 | GmsGmsUmCmAmUmCfAfGfCfUmUmUmGmCmUmAmCmUm | NO.546 | AmsGfsUmAmGmCfAmAmAfGmCmUmGmAfUmGfAmCmCmsUmsCm | NO.799 |

(continued)

| Serial number | Modified sense strand (5'-3') | SEQ ID | Modified antisense strand (5'-3') | SEQ ID |
|---|---|---|---|---|
| RX008223 | CmsAmsGmCmUmUmUfGfCfUfAmCmUmGmUmCmAmCmAm | NO.547 | UmsGfsUmGmAmCfAmGmUfAmGmCmAmAfAmGfCmUmGmsAmsUm | NO.800 |
| RX008224 | CmsAmsCmCmCmUmUfCfCfUfGmGmCmAmCmUmCmUmUm | NO.548 | AmsAfsGmAmGmUfGmCmCfAmGmGmAmAfGmGfGmUmGmsGmsGm | NO.801 |
| RX008225 | CmsUmsCmUmUmUmGfCfUfUfGmAmGmGmAmUmCmUmUm | NO.549 | AmsAfsGmAmUmCfCmUmCfAmAmGmCmAfAmAfGmAmGmsUmsGm | NO.802 |
| RX008226 | GmsCmsUmUmGmAmGfGfAfUfCmUmUmCmCmGmAmUmGm | NO.550 | CmsAfsUmCmGmGfAmAmGfAmUmCmCmUfCmAfAmGmCmsAmsAm | NO.803 |
| RX008227 | CmsGmsAmGmAmCmCfAfUfUfAmCmGmUmAmGmAmCmUm | NO.551 | AmsGfsUmCmUmAfCmGmUfAmAmUmGmGfUmCfUmCmGmsCmsCm | NO.804 |
| RX008228 | CmsCmsUmCmCmUmCfAfAfAfGmCmCmAmAmCmAmAmUm | NO.552 | AmsUfsUmGmUmUfGmGmCfUmUmUmGmAfGmGfAmGmGmsCmsUm | NO.805 |
| RX008229 | CmsCmsUmCmUmAmCfCfUfGfGmAmUmCmAmUmAmAmUm | NO.553 | AmsUfsUmAmUmGfAmUmCfCmAmGmGmUfAmGfAmGmGmsAmsGm | NO.806 |
| RX008230 | CmsCmsUmGmGmAmUfCfAfUfAmAmUmGmGmCmAmAmUm | NO.554 | AmsUfsUmGmCmCfAmUmUfAmUmGmAmUfCmCfAmGmGmsUmsAm | NO.807 |
| RX008231 | GmsCmsAmAmUmGmUfGfGfUfCmAmAmGmAmCmGmGmAm | NO.555 | UmsCfsCmGmUmCfUmUmGfAmCmCmAmCfAmUfUmGmCmsCmsAm | NO.808 |
| RX008232 | GmsGmsAmGmUmGmAfAfGfAfGmAmCmCmAmAmGmAmUm | NO.556 | AmsUfsCmUmUmGfGmUmCfUmCmUmUmCfAmCfUmCmCmsAmsAm | NO.809 |
| RX008233 | GmsUmsGmAmAmGmAfGfAfCfCmAmAmGmAmUmGmAmAm | NO.557 | UmsUfsCmAmUmCfUmUmGfGmUmCmUmCfUmUfCmAmCmsUmsCm | NO.810 |

(continued)

| Serial number | Modified sense strand (5'-3') | SEQ ID | Modified antisense strand (5'-3') | SEQ ID |
|---|---|---|---|---|
| RX008234 | GmsCmsAmCmUmAmAfGfCfCfUmAmAmGmAmAmGmUmUm | NO.558 | AmsAfsCmUmUmCfUmUmAfGmGmCmUmUfAmGfUmGmCmsCmsUm | NO.811 |
| RX008235 | CmsAmsCmUmAmAmUfUfUfUfUmGmUmAmUmUmCmUmUm | NO.559 | AmsAfsGmAmAmUfAmCmAfAmAmAmAmUfUmAfGmUmGmsGmsGm | NO.812 |
| RX008236 | UmsCmsCmAmCmAmAfAfGfUfCmAmAmAmGmCmUmAmUm | NO.560 | AmsUfsAmGmCmUfUmUmGfAmCmUmUmUfGmUfGmGmAmsCmsAm | NO.813 |
| RX008237 | CmsCmsAmCmAmAmAfGfUfCfAmAmAmGmCmUmAmUmUm | NO.561 | AmsAfsUmAmGmCfUmUmUfGmAmCmUmUfUmGfUmGmGmsAmsCm | NO.814 |
| RX008238 | CmsAmsCmAmAmAmGfUfCfAfAmAmGmCmUmAmUmUmUm | NO.562 | AmsAfsAmUmAmGfCmUmUfUmGmAmCmUfUmUfGmUmGmsGmsAm | NO.815 |
| RX008239 | CmsAmsAmAmGmCmUfAfUfUfUmUmCmAmUmAmAmUmAm | NO.563 | UmsAfsUmUmAmUfGmAmAfAmAmUmAmGfCmUfUmUmGmsAmsCm | NO.816 |
| RX008240 | CmsUmsAmUmUmUmUfCfAfUfAmAmUmAmAmUmAmCmUm | NO.564 | AmsGfsUmAmUmUfAmUmUfAmUmGmAmAfAmAfUmAmGmsCmsUm | NO.817 |
| RX008241 | CmsAmsUmAmAmUmAfAfUfAfCmUmAmAmCmAmUmGmUm | NO.565 | AmsCfsAmUmGmUfUmAmGfUmAmUmUmAfUmUfAmUmGmsAmsAm | NO.818 |
| RX008242 | CmsAmsUmGmUmUmAfUfUfUfGmCmCmUmUmUmUmGmAm | NO.566 | UmsCfsAmAmAmAfGmGmCfAmAmAmUmAfAmCfAmUmGmsUmsUm | NO.819 |
| RX008243 | GmsUmsUmAmUmUmUfGfCfCfUmUmUmUmGmAmAmUmUm | NO.567 | AmsAfsUmUmCmAfAmAmAfGmGmCmAmAfAmUfAmAmCmsAmsUm | NO.820 |
| RX008244 | GmsGmsAmGmGmCmAfUfCfAfGmAmUmUmCmCmUmGmAm | NO.568 | UmsCfsAmGmGmAfAmUmCfUmGmAmUmGfCmCfUmCmCmsAmsGm | NO.821 |

(continued)

| Serial number | Modified sense strand (5'-3') | SEQ ID | Modified antisense strand (5'-3') | SEQ ID |
|---|---|---|---|---|
| RX008245 | CmsAmsCmUmGmGmGfAfGfAfCmAmAmGmCmAmUmUmUm | NO.569 | AmsAfsAmUmGmCfUmUmGfUmCmUmCmCfCmAfGmUmGmsGmsGm | NO.822 |
| RX008246 | CmsAmsAmGmCmAmUfUfUfAfUmAmCmUmUmUmCmUmUm | NO.570 | AmsAfsGmAmAmAfGmUmAfUmAmAmAmUfGmCfUmUmGmsUmsCm | NO.823 |
| RX008247 | GmsCmsCmUmGmGmCfUfUfAfUmAmCmUmUmUmCmUmUm | NO.571 | AmsAfsGmAmAmAfGmUmAfUmAmAmGmCfCmAfGmGmCmsGmsCm | NO.824 |
| RX008248 | GmsCmsUmUmAmUmAfCfUfUfUmCmUmUmAmAmUmAmAm | NO.572 | UmsUfsAmUmUmAfAmGmAfAmAmGmUmAfUmAfAmGmCmsCmsAm | NO.825 |
| RX008249 | GmsGmsGmUmGmUmCfCfAfCfAmAmAmGmUmCmAmAmAm | NO.573 | UmsUfsUmGmAmCfUmUmUfGmUmGmGmAfCmAfCmCmCmsCmsUm | NO.826 |
| RX008250 | GmsCmsCmUmUmUmUfGfAfAfUmUmCmUmCmAmUmUmAm | NO.574 | UmsAfsAmUmGmAfGmAmAfUmUmCmAmAfAmAfGmGmCmsAmsAm | NO.827 |
| RX008251 | GmsAmsAmUmUmCmUfCfAfUfUmAmUmCmUmUmAmAmAm | NO.575 | UmsUfsUmAmAmGfAmUmAfAmUmGmAmGfAmAfUmUmCmsAmsAm | NO.828 |
| RX008252 | CmsAmsUmUmAmUmCfUfUfAfAmAmAmUmUmGmUmAmUm | NO.576 | AmsUfsAmCmAmAfUmUmUfUmAmAmGmAfUmAfAmUmGmsAmsGm | NO.829 |
| RX008253 | GmsAmsUmUmAmCmAfUfCfAfUmCmUmUmUmCmUmGmAm | NO.577 | UmsCfsAmGmAmAfAmGmAfUmGmAmUmGfUmAfAmUmCmsAmsCm | NO.830 |

In the present disclosure, the base composition and modification symbols are defined as follows: capital letters A, U, G, C, and T represent the base composition of nucleotides; lowercase letter m represents that one nucleotide adjacent to the left of the letter m is a 2'-methoxy modified nucleotide; lowercase letter f represents that one nucleotide adjacent to the left of the letter f is a 2'-fluoro modified nucleotide; lowercase letter d represents that one nucleotide adjacent to the left of the letter d is a deoxyribonucleotide; The lowercase letters indicates that there is a phosphorothioate linkage between the two nucleotides adjacent to the letters.

Information of double-stranded oligonucleotide conjugates with ligand L96 described in the present disclosure is shown in Table 3.

Table 3. Information table of double-stranded oligonucleotide conjugates with ligand L96

| Serial number | Modified sense strand (5'-3') | SEQ ID | Modified antisense strand (5'-3') | SEQ ID |
|---|---|---|---|---|
| RZ000001 | UmsUmsCmUmCmCmGfAfAfCmGmUmGmUmCmAmCmGmUm_L96 | NO.831 | AmsCfsGmUmGmAfCmAmCmGmUmUmCmGfGmAfGmAmAmsCmsUm | NO.889 |
| RZM08001 | CmsCmsUmAmGmGmCfUfAfUmUmGmAmGmGmAmCmAmAm_L96 | NO.832 | UmsUfsGmUmCmCfUmCmAmAmUmAmGmCfCmUfAmGmGmsAmsCm | NO.890 |
| RZM08002 | GmsGmsAmGmCmAmUfGfAfAmGmCmUmUmCmCmAmAmAm_L96 | NO.833 | UmsUfsUmGmGmAfAmGmCmUmUmCmAmUfGmCfUmCmCmsAmsGm | NO.891 |
| RZM08003 | GmsGmsUmGmCmAmGfAfGfUmAmCmAmAmGmAmUmCmUm_L96 | NO.834 | AmsGfsAmUmCmUfUmGmUmAmCmUmCmUfGmCfAmCmCmsCmsAm | NO.892 |
| RZM08004 | GmsCmsAmAmCmCmGfAfGfAmGmAmAmAmGmUmCmAmUm_L96 | NO.835 | AmsUfsGmAmCmUfUmUmCmUmCmUmCmGfGmUfUmGmCmsCmsAm | NO.893 |
| RZM08005 | GmsAmsGmAmGmAmAfAfGfUmCmAmUmCmAmGmCmUmUm_L96 | NO.836 | AmsAfsGmCmUmGfAmUmGmAmCmUmUmUfCmUfCmUmCmsGmsGm | NO.894 |
| RZM08006 | CmsAmsGmCmUmUmUfGfCfUmAmCmCmAmUmCmAmUmAm_L96 | NO.837 | UmsAfsUmGmAmUfGmGmUmAmGmCmAmAfAmGfCmUmGmsAmsUm | NO.895 |
| RZM08007 | GmsCmsUmUmUmGmCfUfAfCmCmAmUmCmAmUmAmGmAm_L96 | NO.838 | UmsCfsUmAmUmGfAmUmGmGmUmAmGmCfAmAfAmGmCmsUmsGm | NO.896 |
| RZM08008 | GmsCmsUmAmCmCmAfUfCfAmUmAmGmAmCmAmAmAmUm_L96 | NO.839 | AmsUfsUmUmGmUfCmUmAmUmGmAmUmGfGmUfAmGmCmsAmsAm | NO.897 |
| RZM08009 | GmsAmsAmAmCmUmCfCfAfAmCmUmGmGmAmAmUmUmUm_L96 | NO.840 | AmsAfsAmUmUmCfCmAmGmUmUmGmGmAfGmUfUmUmCmsAmsCm | NO.898 |
| RZM08010 | GmsUmsCmCmCmUmUfCfUfUmGmGmAmAmCmUmUmAmAm_L96 | NO.841 | UmsUfsAmAmGmUfUmCmCmAmAmGmAmAfGmGfGmAmCmsGmsUm | NO.899 |

(continued)

| Serial number | Modified sense strand (5'-3') | SEQ ID | Modified antisense strand (5'-3') | SEQ ID |
|---|---|---|---|---|
| RZM08011 | GmsGmsAmAmCmUmUfAfAfGmAmUmCmCmGmAmGmCmUm_L96 | NO.842 | AmsGfsCmUmCmGfGmAmUmCmUmUmAmAfGmUfUmCmCmsAmsAm | NO.900 |
| RZM08012 | GmsGmsCmGmAmGmAfCfCfAmCmUmAmUmGmUmAmGmAm_L96 | NO.843 | UmsCfsUmAmCmAfUmAmGmUmGmGmUmCfUmCfGmCmCmsUmsAm | NO.901 |
| RZM08013 | GmsCmsAmCmGmAmAfGfGfCmCmUmCmUmCmUmCmUmCm_L96 | NO.844 | GmsAfsGmAmGmAfGmAmGmGmCmCmUmUfCmGfUmGmCmsAmsGm | NO.902 |
| RZM08014 | UmsCmsUmCmUmCmUfCfUfCmCmUmCmUmAmCmCmUmUm_L96 | NO.845 | AmsAfsGmGmUmAfGmAmGmGmAmGmAmGfAmGfAmGmAmsGmsGm | NO.903 |
| RZM08015 | GmsGmsUmUmCmUmGfGfGfUmGmAmAmGmUmUmCmAmAm_L96 | NO.846 | UmsUfsGmAmAmCfUmUmCmAmCmCmCmAfGmAfAmCmCmsUmsUm | NO.904 |
| RZM08016 | GmsGmsGmUmGmAmAfGfUfUmCmAmAmGmGmUmUmCmAm_L96 | NO.847 | UmsGfsAmAmCmCfUmUmGmAmAmCmUmUfCmAfCmCmCmsAmsGm | NO.905 |
| RZM08017 | GmsGmsUmGmAmAmGfUfUfCmAmAmGmGmUmUmCmAmAm_L96 | NO.848 | UmsUfsGmAmAmCfCmUmUmGmAmAmCmUfUmCfAmCmCmsCmsAm | NO.906 |
| RZM08018 | GmsAmsAmGmUmUmCfAfAfGmGmUmUmCmAmAmGmUmUm_L96 | NO.849 | AmsAfsCmUmUmGfAmAmCmCmUmUmGmAfAmCfUmUmCmsAmsCm | NO.907 |
| RZM08019 | CmsGmsAmGmAmAmAfGfAfAmGmUmUmCmCmUmCmAmAm_L96 | NO.850 | UmsUfsGmAmGmGfAmAmCmUmUmCmUmUfUmCfUmCmGmsGmsGm | NO.908 |
| RZM08020 | GmsAmsAmAmGmAmAfGfUfUmCmCmUmCmAmAmGmUmAm_L96 | NO.851 | UmsAfsCmUmUmGfAmGmGmAmAmCmUmUfCmUfUmUmCmsUmsCm | NO.909 |
| RZM08021 | AmsGmsAmCmAmAmAfCfAfUmAmCmAmGmGmCmUmAmAm_L96 | NO.852 | UmsUfsAmGmCmCfUmGmUmAmUmGmUmUfUmGfUmCmUmsUmsUm | NO.910 |

(continued)

| Serial number | Modified sense strand (5'-3') | SEQ ID | Modified antisense strand (5'-3') | SEQ ID |
|---|---|---|---|---|
| RZM08022 | CmsCmsUmGmCmUmUfUfGfAmCmAmAmGmGmUmCmAmAm_L96 | NO.853 | UmsUfsGmAmCmCfUmUmGmUmCmAmAmAfGmCfAmGmGmsCmsAm | NO.911 |
| RZM08023 | GmsCmsAmCmCmAmGfAfGfAmAmUmUmGmUmGmUmAmAm_L96 | NO.854 | UmsUfsAmCmAmCfAmAmUmUmCmUmCmUfGmGfUmGmCmsUmsUm | NO.912 |
| RZM08024 | GmsCmsUmUmAmCmAfCfUfUmAmUmAmAmCmUmAmCmAm_L96 | NO.855 | UmsGfsUmAmGmUfUmAmUmAmAmGmUmGfUmAfAmGmCmsCmsAm | NO.913 |
| RZ008001 | CmsAmsAmGmCmAmGfCfAfAmAmUmCmCmUmGmGmAmUm_L96 | NO.856 | AmsUfsCmCmAmGfGmAmUmUmUmGmCmUfGmCfUmUmGmsGmsCm | NO.914 |
| RZ008002 | GmsGmsUmCmAmUmCfAfGfCmUmUmUmGmCmUmAmCmUm_L96 | NO.857 | AmsGfsUmAmGmCfAmAmAmGmCmUmGmAfUmGfAmCmCmsUmsCm | NO.915 |
| RZ008003 | CmsAmsGmCmUmUmUfGfCfUmAmCmUmGmUmCmAmCmAm_L96 | NO.858 | UmsGfsUmGmAmCfAmGmUmAmGmCmAmAfAmGfCmUmGmsAmsUm | NO.916 |
| RZ008004 | CmsAmsCmCmCmUmUfCfCfUmGmGmCmAmCmUmCmUmUm_L96 | NO.859 | AmsAfsGmAmGmUfGmCmCmAmGmGmAmAfGmGfGmUmGmsGmsGm | NO.917 |
| RZ008005 | CmsUmsCmUmUmUmGfCfUfUmGmAmGmGmAmUmCmUmUm_L96 | NO.860 | AmsAfsGmAmUmCfCmUmCmAmAmGmCmAfAmAfGmAmGmsUmsGm | NO.918 |
| RZ008006 | GmsCmsUmUmGmAmGfGfAfUmCmUmUmCmCmGmAmUmGm_L96 | NO.861 | CmsAfsUmCmGmGfAmAmGmAmUmCmCmUfCmAfAmGmCmsAmsAm | NO.919 |
| RZ008007 | CmsGmsAmGmAmCmCfAfUfUmAmCmGmUmAmGmAmCmUm_L96 | NO.862 | AmsGfsUmCmUmAfCmGmUmAmAmUmGmGfUmCfUmCmGmsCmsCm | NO.920 |
| RZ008008 | CmsCmsUmCmCmUmCfAfAfAmGmCmCmAmAmCmAmAmUm_L96 | NO.863 | AmsUfsUmGmUmUfGmGmCmUmUmUmGmAfGmGfAmGmGmsCmsUm | NO.921 |

(continued)

| Serial number | Modified sense strand (5'-3') | SEQ ID | Modified antisense strand (5'-3') | SEQ ID |
|---|---|---|---|---|
| RZ008009 | CmsCmsUmCmUmAmCfCfUfGmGmAmUmCmAmUmAmAmUm_L96 | NO.864 | AmsUfsUmAmUmGfAmUmCmCmAmGmGmUfAmGfAmGmGmsAmsGm | NO.922 |
| RZ008010 | CmsCmsUmGmGmAmUfCfAfUmAmAmUmGmGmCmAmAmUm_L96 | NO.865 | AmsUfsUmGmCmCfAmUmUmAmUmGmAmUfCmCfAmGmGmsUmsAm | NO.923 |
| RZ008011 | GmsCmsAmAmUmGmUfGfGfUmCmAmAmGmAmCmGmGmAm_L96 | NO.866 | UmsCfsCmGmUmCfUmUmGmAmCmCmAmCfAmUfUmGmCmsCmsAm | NO.924 |
| RZ008012 | GmsGmsAmGmUmGmAfAfGfAmGmAmCmCmAmAmGmAmUm_L96 | NO.867 | AmsUfsCmUmUmGfGmUmCmUmCmUmUmCfAmCfUmCmCmsAmsAm | NO.925 |
| RZ008013 | GmsUmsGmAmAmGmAfGfAfCmCmAmAmGmAmUmGmAmAm_L96 | NO.868 | UmsUfsCmAmUmCfUmUmGmGmUmCmUmCfUmUfCmAmCmsUmsCm | NO.926 |
| RZ008014 | GmsCmsAmCmUmAmAfGfCfCmUmAmAmGmAmAmGmUmUm_L96 | NO.869 | AmsAfsCmUmUmCfUmUmAmGmGmCmUmUfAmGfUmGmCmsCmsUm | NO.927 |
| RZ008015 | CmsAmsCmUmAmAmUfUfUfUmUmGmUmAmUmUmCmUmUm_L96 | NO.870 | AmsAfsGmAmAmUfAmCmAmAmAmAmAmUfUmAfGmUmGmsGmsGm | NO.928 |
| RZ008016 | UmsCmsCmAmCmAmAfAfGfUmCmAmAmAmGmCmUmAmUm_L96 | NO.871 | AmsUfsAmGmCmUfUmUmGmAmCmUmUmUfGmUfGmGmAmsCmsAm | NO.929 |
| RZ008017 | CmsCmsAmCmAmAmAfGfUfCmAmAmAmGmCmUmAmUmUm_L96 | NO.872 | AmsAfsUmAmGmCfUmUmUmGmAmCmUmUfUmGfUmGmGmsAmsCm | NO.930 |
| RZ008018 | CmsAmsCmAmAmAmGfUfCfAmAmAmGmCmUmAmUmUmUm_L96 | NO.873 | AmsAfsAmUmAmGfCmUmUmUmGmAmCmUfUmUfGmUmGmsGmsAm | NO.931 |
| RZ008019 | CmsAmsAmAmGmCmUfAfUfUmUmUmCmAmUmAmAmUmAm_L96 | NO.874 | UmsAfsUmUmAmUfGmAmAmAmAmUmAmGfCmUfUmUmGmsAmsCm | NO.932 |

(continued)

| Serial number | Modified sense strand (5'-3') | SEQ ID | Modified antisense strand (5'-3') | SEQ ID |
|---|---|---|---|---|
| RZ008020 | CmsUmsAmUmUmUmUfCfAfUmAmAmUmAmAmUmAmCmUm_L96 | NO.875 | AmsGfsUmAmUmUfAmUmUmAmUmGmAmAfAmAfUmAmGmsCmsUm | NO.933 |
| RZ008021 | CmsAmsUmAmAmUmAfAfUfAmCmUmAmAmCmAmUmGmUm_L96 | NO.876 | AmsCfsAmUmGmUfUmAmGmUmAmUmUmAfUmUfAmUmGmsAmsAm | NO.934 |
| RZ008022 | CmsAmsUmGmUmUmAfUfUfUmGmCmCmUmUmUmUmGmAm_L96 | NO.877 | UmsCfsAmAmAmAfGmGmCmAmAmAmUmAfAmCfAmUmGmsUmsUm | NO.935 |
| RZ008023 | GmsUmsUmAmUmUmUfGfCfCmUmUmUmUmGmAmAmUmUm_L96 | NO.878 | AmsAfsUmUmCmAfAmAmAmGmGmCmAmAfAmUfAmAmCmsAmsUm | NO.936 |
| RZ008024 | GmsGmsAmGmGmCmAfUfCfAmGmAmUmUmCmCmUmGmAm_L96 | NO.879 | UmsCfsAmGmGmAfAmUmCmUmGmAmUmGfCmCfUmCmCmsAmsGm | NO.937 |
| RZ008025 | CmsAmsCmUmGmGmGfAfGfAmCmAmAmGmCmAmUmUmUm_L96 | NO.880 | AmsAfsAmUmGmCfUmUmGmUmCmUmCmCfCmAfGmUmGmsGmsGm | NO.938 |
| RZ008026 | CmsAmsAmGmCmAmUfUfUfAmUmAmCmUmUmUmCmUmUm_L96 | NO.881 | AmsAfsGmAmAmAfGmUmAmUmAmAmAmUfGmCfUmUmGmsUmsCm | NO.939 |
| RZ008027 | GmsCmsCmUmGmGmCfUfUfAmUmAmCmUmUmUmCmUmUm_L96 | NO.882 | AmsAfsGmAmAmAfGmUmAmUmAmAmGmCfCmAfGmGmCmsGmsCm | NO.940 |
| RZ008028 | GmsCmsUmUmAmUmAfCfUfUmUmCmUmUmAmAmUmAmAm_L96 | NO.883 | UmsUfsAmUmUmAfAmGmAmAmAmGmUmAfUmAfAmGmCmsCmsAm | NO.941 |
| RZ008029 | GmsGmsGmUmGmUmCfCfAfCmAmAmAmGmUmCmAmAmAm_L96 | NO.884 | UmsUfsUmGmAmCfUmUmUmGmUmGmGmAfCmAfCmCmCmsCmsUm | NO.942 |
| RZ008030 | GmsCmsCmUmUmUmUfGfAfAmUmUmCmUmCmAmUmUmAm_L96 | NO.885 | UmsAfsAmUmGmAfGmAmAmUmUmCmAmAfAmAfGmGmCmsAmsAm | NO.943 |

(continued)

| Serial number | Modified sense strand (5'-3') | SEQ ID | Modified antisense strand (5'-3') | SEQ ID |
|---|---|---|---|---|
| RZ008031 | GmsAmsAmUmUmCmUfCfAfUmUmAmUmCmUmUmAmAmAm_L96 | NO.886 | UmsUfsUmAmAmGfAmUmAmAmUmGmAmGfAmAfUmUmCmsAmsAm | NO.944 |
| RZ008032 | CmsAmsUmUmAmUmCfUfUfAmAmAmAmUmUmGmUmAmUm_L96 | NO.887 | AmsUfsAmCmAmAfUmUmUmUmAmAmGmAfUmAfAmUmGmsAmsGm | NO.945 |
| RZ008033 | GmsAmsUmUmAmCmAfUfCfAmUmCmUmUmUmCmUmGmAm_L96 | NO.888 | UmsCfsAmGmAmAfAmGmAmUmGmAmUmGfUmAfAmUmCmsAmsCm | NO.946 |

Information of double-stranded oligonucleotide conjugates with ligand tri-cluster CR01008 described in the present disclosure is shown in Table 4.

Table 4. Information table of double-stranded oligonucleotide conjugates with ligand (CR01008)×3

| Serial number | Modified sense strand (5'-3') | SEQ ID | Modified antisense strand (5'-3') | SEQ ID |
|---|---|---|---|---|
| RZ008034 | CmsAmsAmGmCmAmGfCfAfAfAmUmCmCmUmGmGmAmUm_CR01008×3 | NO.947 | AmsUfsCmCmAmGfGmAmUfUmUmGmCmUfG(moe)CfUmUmGmsGmsCm | NO. 1046 |
| RZ008035 | GmsGmsUmCmAmUmCfAfGfCfUmUmUmGmCmUmAmCmUm_CR01008×3 | NO.948 | AmsGfsUmAmGmCfAmAmAfGmCmUmGmAfU(moe)GfAmCmCmsUmsCm | NO. 1047 |
| RZ008036 | CmsAmsGmCmUmUmUfGfCfUfAmCmUmGmUmCmAmCmAm_CR01008×3 | NO.949 | UmsGfsUmGmAmCfAmGmUfAmGmCmAmAfA(moe)GfCmUmGmsAmsUm | NO. 1048 |
| RZ008037 | CmsAmsCmCmCmUmUfCfCfUfGmGmCmAmCmUmCmUmUm_CR01008×3 | NO.950 | AmsAfsGmAmGmUfGmCmCfAmGmGmAmAfG(moe)GfGmUmGmsGmsGm | NO. 1049 |
| RZ008038 | CmsUmsCmUmUmUmGfCfUfUfGmAmGmGmAmUmCmUmUm_CR01008×3 | NO.951 | AmsAfsGmAmUmCfCmUmCfAmAmGmCmAfA(moe)AfGmAmGmsUmsGm | NO. 1050 |
| RZ008039 | GmsCmsUmUmGmAmGfGfAfUfCmUmUmCmCmGmAmUmGm_CR01008×3 | NO.952 | CmsAfsUmCmGmGfAmAmGfAmUmCmCmUfC(moe)AfAmGmCmsAmsAm | NO.1051 |

(continued)

| Serial number | Modified sense strand (5'-3') | SEQ ID | Modified antisense strand (5'-3') | SEQ ID |
|---|---|---|---|---|
| RZ008040 | CmsGmsAmGmAmCmCfAfUfUfAmCmGmUmAmGmAmCmUm_CR01008×3 | NO.953 | AmsGfsUmCmUmAfCmGmUfAmAmUmGmGfU(moe)CfUmCmGmsCmsCm | NO. 1052 |
| RZ008041 | CmsCmsUmCmCmUmCfAfAfAfGmCmCmAmAmCmAmAmUm_CR01008×3 | NO.954 | AmsUfsUmGmUmUfGmGmCfUmUmUmGmAfG(moe)GfAmGmGmsCmsUm | NO.1053 |
| RZ008042 | CmsCmsUmCmUmAmCfCfUfGfGmAmUmCmAmUmAmAmUm_CR01008×3 | NO.955 | AmsUfsUmAmUmGfAmUmCfCmAmGmGmUfA(moe)GfAmGmGmsAmsGm | NO. 1054 |
| RZ008043 | CmsCmsUmGmGmAmUfCfAfUfAmAmUmGmGmCmAmAmUm_CR01008×3 | NO.956 | AmsUfsUmGmCmCfAmUmUfAmUmGmAmUfC(moe)CfAmGmGmsUmsAm | NO. 1055 |
| RZ008044 | GmsCmsAmAmUmGmUfGfGfUfCmAmAmGmAmCmGmGmAm_CR01008×3 | NO.957 | UmsCfsCmGmUmCfUmUmGfAmCmCmAmCfA(moe)UfUmGmCmsCmsAm | NO. 1056 |
| RZ008045 | GmsGmsAmGmUmGmAfAfGfAfGmAmCmCmAmAmGmAmUm_CR01008×3 | NO.958 | AmsUfsCmUmUmGfGmUmCfUmCmUmUmCfA(moe)CfUmCmCmsAmsAm | NO. 1057 |
| RZ008046 | GmsUmsGmAmAmGmAfGfAfCfCmAmAmGmAmUmGmAmAm_CR01008×3 | NO.959 | UmsUfsCmAmUmCfUmUmGfGmUmCmUmCfU(moe)UfCmAmCmsUmsCm | NO.1058 |
| RZ008047 | GmsCmsAmCmUmAmAfGfCfCfUmAmAmGmAmAmGmUmUm_CR01008×3 | NO.960 | AmsAfsCmUmUmCfUmUmAfGmGmCmUmUfA(moe)GfUmGmCmsCmsUm | NO. 1059 |
| RZ008048 | CmsAmsCmUmAmAmUfUfUfUfUmGmUmAmUmUmCmUmUm_CR01008×3 | NO.961 | AmsAfsGmAmAmUfAmCmAfAmAmAmAmUfU(moe)AfGmUmGmsGmsGm | NO. 1060 |
| RZ008049 | UmsCmsCmAmCmAmAfAfGfUfCmAmAmAmGmCmUmAmUm_CR01008×3 | NO.962 | AmsUfsAmGmCmUfUmUmGfAmCmUmUmUfG(moe)UfGmGmAmsCmsAm | NO. 1061 |
| RZ008050 | CmsCmsAmCmAmAmAfGfUfCfAmAmAmGmCmUmAmUmUm_CR01008×3 | NO.963 | AmsAfsUmAmGmCfUmUmUfGmAmCmUmUfU(moe)GfUmGmGmsAmsCm | NO. 1062 |

(continued)

| Serial number | Modified sense strand (5'-3') | SEQ ID | Modified antisense strand (5'-3') | SEQ ID |
|---|---|---|---|---|
| RZ008051 | CmsAmsCmAmAmAmGfUfCfAfAmAmGmCmUmAmUmUmUm_CR01008×3 | NO.964 | AmsAfsAmUmAmGfCmUmUfUmGmAmCmUfU(moe)UfGmUmGmsGmsAm | NO. 1063 |
| RZ008052 | CmsAmsAmAmGmCmUfAfUfUfUmUmCmAmUmAmAmUmAm_CR01008×3 | NO.965 | UmsAfsUmUmAmUfGmAmAfAmAmUmAmGfC(moe)UfUmUmGmsAmsCm | NO. 1064 |
| RZ008053 | CmsUmsAmUmUmUmUfCfAfUfAmAmUmAmAmUmAmCmUm_CR01008×3 | NO.966 | AmsGfsUmAmUmUfAmUmUfAmUmGmAmAfA(moe)AfUmAmGmsCmsUm | NO. 1065 |
| RZ008054 | CmsAmsUmAmAmUmAfAfUfAfCmUmAmAmCmAmUmGmUm_CR01008×3 | NO.967 | AmsCfsAmUmGmUfUmAmGfUmAmUmUmAfU(moe)UfAmUmGmsAmsAm | NO. 1066 |
| RZ008055 | CmsAmsUmGmUmUmAfUfUfUfGmCmCmUmUmUmUmGmAm_CR01008×3 | NO.968 | UmsCfsAmAmAmAfGmGmCfAmAmAmUmAfA(moe)CfAmUmGmsUmsUm | NO. 1067 |
| RZ008056 | GmsUmsUmAmUmUmUfGfCfCfUmUmUmUmGmAmAmUmUm_CR01008×3 | NO.969 | AmsAfsUmUmCmAfAmAmAfGmGmCmAmAfA(moe)UfAmAmCmsAmsUm | NO. 1068 |
| RZ008057 | GmsGmsAmGmGmCmAfUfCfAfGmAmUmUmCmCmUmGmAm_CR01008×3 | NO.970 | UmsCfsAmGmGmAfAmUmCfUmGmAmUmGfC(moe)CfUmCmCmsAmsGm | NO. 1069 |
| RZ008058 | CmsAmsCmUmGmGmGfAfGfAfCmAmAmGmCmAmUmUmUm_CR01008×3 | NO.971 | AmsAfsAmUmGmCfUmUmGfUmCmUmCmCfC(moe)AfGmUmGmsGmsGm | NO. 1070 |
| RZ008059 | CmsAmsAmGmCmAmUfUfUfAfUmAmCmUmUmUmCmUmUm_CR01008×3 | NO.972 | AmsAfsGmAmAmAfGmUmAfUmAmAmAmUfG(moe)CfUmUmGmsUmsCm | NO. 1071 |
| RZ008060 | GmsCmsCmUmGmGmCfUfUfAfUmAmCmUmUmUmCmUmUm_CR01008×3 | NO.973 | AmsAfsGmAmAmAfGmUmAfUmAmAmGmCfC(moe)AfGmGmCmsGmsCm | NO.1072 |
| RZ008061 | GmsCmsUmUmAmUmAfCfUfUfUmCmUmUmAmAmUmAmAm_CR01008×3 | NO.974 | UmsUfsAmUmUmAfAmGmAfAmAmGmUmAfU(moe)AfAmGmCmsCmsAm | NO. 1073 |

(continued)

| Serial number | Modified sense strand (5'-3') | SEQ ID | Modified antisense strand (5'-3') | SEQ ID |
|---|---|---|---|---|
| RZ008062 | GmsGmsGmUmGmUmCfCfAfCfAmAmAmGmUmCmAmAmAm_CR01008×3 | NO.975 | UmsUfsUmGmAmCfUmUmUfGmUmGmGmAfC(moe)AfCmCmCmsCmsUm | NO. 1074 |
| RZ008063 | GmsCmsCmUmUmUmUfGfAfAfUmUmCmUmCmAmUmUmAm_CR01008×3 | NO.976 | UmsAfsAmUmGmAfGmAmAfUmUmCmAmAfA(moe)AfGmGmCmsAmsAm | NO. 1075 |
| RZ008064 | GmsAmsAmUmUmCmUfCfAfUfUmAmUmCmUmUmAmAmAm_CR01008×3 | NO.977 | UmsUfsUmAmAmGfAmUmAfAmUmGmAmGfA(moe)AfUmUmCmsAmsAm | NO. 1076 |
| RZ008065 | CmsAmsUmUmAmUmCfUfUfAfAmAmAmUmUmGmUmAmUm_CR01008×3 | NO.978 | AmsUfsAmCmAmAfUmUmUfUmAmAmGmAfU(moe)AfAmUmGmsAmsGm | NO.1077 |
| RZ008066 | GmsAmsUmUmAmCmAfUfCfAfUmCmUmUmUmCmUmGmAm_CR01008×3 | NO.979 | UmsCfsAmGmAmAfAmGmAfUmGmAmUmGfU(moe)AfAmUmCmsAmsCm | NO. 1078 |
| RZ008067 | CmsAmsAmGmCmAmGfCfAfAfAmUmCmCmUmGmGmAmUm_CR01008×3 | NO.980 | AmsUfsCmCmAmGfGmAmUmUmUmGfCmUfG(moe)CfUmUmGmsGmsCm | NO. 1079 |
| RZ008068 | GmsGmsUmCmAmUmCfAfGfCfUmUmUmGmCmUmAmCmUm_CR01008×3 | NO.981 | AmsGfsUmAmGmCfAmAmAmGmCmUfGmAfU(moe)GfAmCmCmsUmsCm | NO. 1080 |
| RZ008069 | CmsAmsGmCmUmUmUfGfCfUfAmCmUmGmUmCmAmCmAm_CR01008×3 | NO.982 | UmsGfsUmGmAmCfAmGmUmAmGmCfAmAfA(moe)GfCmUmGmsAmsUm | NO.1081 |
| RZ008070 | CmsAmsCmCmCmUmUfCfCfUfGmGmCmAmCmUmCmUmUm_CR01008×3 | NO.983 | AmsAfsGmAmGmUfGmCmCmAmGmGfAmAfG(moe)GfGmUmGmsGmsGm | NO. 1082 |
| RZ008071 | CmsUmsCmUmUmUmGfCfUfUfGmAmGmGmAmUmCmUmUm_CR01008×3 | NO.984 | AmsAfsGmAmUmCfCmUmCmAmAmGfCmAfA(moe)AfGmAmGmsUmsGm | NO. 1083 |
| RZ008072 | GmsCmsUmUmGmAmGfGfAfUfCmUmUmCmCmGmAmUmGm_CR01008×3 | NO.985 | CmsAfsUmCmGmGfAmAmGmAmUmCfCmUfC(moe)AfAmGmCmsAmsAm | NO. 1084 |

(continued)

| Serial number | Modified sense strand (5'-3') | SEQ ID | Modified antisense strand (5'-3') | SEQ ID |
|---|---|---|---|---|
| RZ008073 | CmsGmsAmGmAmCmCfAfUfUfAmCmGmUmAmGmAmCmUm_CR01008×3 | NO.986 | AmsGfsUmCmUmAfCmGmUmAmAmUfGmGfU(moe)CfUmCmGmsCmsCm | NO. 1085 |
| RZ008074 | CmsCmsUmCmCmUmCfAfAfAfGmCmCmAmAmCmAmAmUm_CR01008×3 | NO.987 | AmsUfsUmGmUmUfGmGmCmUmUmUfGmAfG(moe)GfAmGmGmsCmsUm | NO. 1086 |
| RZ008075 | CmsCmsUmCmUmAmCfCfUfGfGmAmUmCmAmUmAmAmUm_CR01008×3 | NO.988 | AmsUfsUmAmUmGfAmUmCmCmAmGfGmUfA(moe)GfAmGmGmsAmsGm | NO. 1087 |
| RZ008076 | CmsCmsUmGmGmAmUfCfAfUfAmAmUmGmGmCmAmAmUm_CR01008×3 | NO.989 | AmsUfsUmGmCmCfAmUmUmAmUmGfAmUfC(moe)CfAmGmGmsUmsAm | NO.1088 |
| RZ008077 | GmsCmsAmAmUmGmUfGfGfUfCmAmAmGmAmCmGmGmAm_CR01008×3 | NO.990 | UmsCfsCmGmUmCfUmUmGmAmCmCfAmCfA(moe)UfUmGmCmsCmsAm | NO. 1089 |
| RZ008078 | GmsGmsAmGmUmGmAfAfGfAfGmAmCmCmAmAmGmAmUm_CR01008×3 | NO.991 | AmsUfsCmUmUmGfGmUmCmUmCmUfUmCfA(moe)CfUmCmCmsAmsAm | NO. 1090 |
| RZ008079 | GmsUmsGmAmAmGmAfGfAfCfCmAmAmGmAmUmGmAmAm_CR01008×3 | NO.992 | UmsUfsCmAmUmCfUmUmGmGmUmCfUmCfU(moe)UfCmAmCmsUmsCm | NO. 1091 |
| RZ008080 | GmsCmsAmCmUmAmAfGfCfCfUmAmAmGmAmAmGmUmUm_CR01008×3 | NO.993 | AmsAfsCmUmUmCfUmUmAmGmGmCfUmUfA(moe)GfUmGmCmsCmsUm | NO. 1092 |
| RZ008081 | CmsAmsCmUmAmAmUfUfUfUfUmGmUmAmUmUmCmUmUm_CR01008×3 | NO.994 | AmsAfsGmAmAmUfAmCmAmAmAmAfAmUfU(moe)AfGmUmGmsGmsGm | NO. 1093 |
| RZ008082 | UmsCmsCmAmCmAmAfAfGfUfCmAmAmAmGmCmUmAmUm_CR01008×3 | NO.995 | AmsUfsAmGmCmUfUmUmGmAmCmUfUmUfG(moe)UfGmGmAmsCmsAm | NO. 1094 |
| RZ008083 | CmsCmsAmCmAmAmAfGfUfCfAmAmAmGmCmUmAmUmUm_CR01008×3 | NO.996 | AmsAfsUmAmGmCfUmUmUmGmAmCfUmUfU(moe)GfUmGmGmsAmsCm | NO. 1095 |

(continued)

| Serial number | Modified sense strand (5'-3') | SEQ ID | Modified antisense strand (5'-3') | SEQ ID |
|---|---|---|---|---|
| RZ008084 | CmsAmsCmAmAmAmGfUfCfAfAmAmGmCmUmAmUmUmUm_CR01008×3 | NO.997 | AmsAfsAmUmAmGfCmUmUmUmGmAfCmUfU(moe)UfGmUmGmsGmsAm | NO. 1096 |
| RZ008085 | CmsAmsAmAmGmCmUfAfUfUfUmUmCmAmUmAmAmUmAm_CR01008×3 | NO.998 | UmsAfsUmUmAmUfGmAmAmAmAmUfAmGfC(moe)UfUmUmGmsAmsCm | NO. 1097 |
| RZ008086 | CmsUmsAmUmUmUmUfCfAfUfAmAmUmAmAmUmAmCmUm_CR01008×3 | NO.999 | AmsGfsUmAmUmUfAmUmUmAmUmGfAmAfA(moe)AfUmAmGmsCmsUm | NO. 1098 |
| RZ008087 | CmsAmsUmAmAmUmAfAfUfAfCmUmAmAmCmAmUmGmUm_CR01008×3 | NO. 1000 | AmsCfsAmUmGmUfUmAmGmUmAmUfUmAfU(moe)UfAmUmGmsAmsAm | NO. 1099 |
| RZ008088 | CmsAmsUmGmUmUmAfUfUfUfGmCmCmUmUmUmUmGmAm_CR01008×3 | NO.1001 | UmsCfsAmAmAmAfGmGmCmAmAmAfUmAfA(moe)CfAmUmGmsUmsUm | NO.1100 |
| RZ008089 | GmsUmsUmAmUmUmUfGfCfCfUmUmUmUmGmAmAmUmUm_CR01008×3 | NO. 1002 | AmsAfsUmUmCmAfAmAmAmGmGmCfAmAfA(moe)UfAmAmCmsAmsUm | NO.1101 |
| RZ008090 | GmsGmsAmGmGmCmAfUfCfAfGmAmUmUmCmCmUmGmAm_CR01008×3 | NO. 1003 | UmsCfsAmGmGmAfAmUmCmUmGmAfUmGfC(moe)CfUmCmCmsAmsGm | NO. 1102 |
| RZ008091 | CmsAmsCmUmGmGmGfAfGfAfCmAmAmGmCmAmUmUmUm_CR01008×3 | NO. 1004 | AmsAfsAmUmGmCfUmUmGmUmCmUfCmCfC(moe)AfGmUmGmsGmsGm | NO. 1103 |
| RZ008092 | CmsAmsAmGmCmAmUfUfUfAfUmAmCmUmUmUmCmUmUm_CR01008×3 | NO. 1005 | AmsAfsGmAmAmAfGmUmAmUmAmAfAmUfG(moe)CfUmUmGmsUmsCm | NO. 1104 |
| RZ008093 | GmsCmsCmUmGmGmCfUfUfAfUmAmCmUmUmUmCmUmUm_CR01008×3 | NO. 1006 | AmsAfsGmAmAmAfGmUmAmUmAmAfGmCfC(moe)AfGmGmCmsGmsCm | NO. 1105 |
| RZ008094 | GmsCmsUmUmAmUmAfCfUfUfUmCmUmUmAmAmUmAmAm_CR01008×3 | NO. 1007 | UmsUfsAmUmUmAfAmGmAmAmAmGfUmAfU(moe)AfAmGmCmsCmsAm | NO. 1106 |

(continued)

| Serial number | Modified sense strand (5'-3') | SEQ ID | Modified antisense strand (5'-3') | SEQ ID |
|---|---|---|---|---|
| RZ008095 | GmsGmsGmUmGmUmCfCfAfCfAmAmAmGmUmCmAmAmAm_CR01008×3 | NO. 1008 | UmsUfsUmGmAmCfUmUmUmGmUmGfGmAfC(moe)AfCmCmCmsCmsUm | NO. 1107 |
| RZ008096 | GmsCmsCmUmUmUmUfGfAfAfUmUmCmUmCmAmUmUmAm_CR01008×3 | NO. 1009 | UmsAfsAmUmGmAfGmAmAmUmUmCfAmAfA(moe)AfGmGmCmsAmsAm | NO. 1108 |
| RZ008097 | GmsAmsAmUmUmCmUfCfAfUfUmAmUmCmUmUmAmAmAm_CR01008×3 | NO.1010 | UmsUfsUmAmAmGfAmUmAmAmUmGfAmGfA(moe)AfUmUmCmsAmsAm | NO. 1109 |
| RZ008098 | CmsAmsUmUmAmUmCfUfUfAfAmAmAmUmUmGmUmAmUm_CR01008×3 | NO.1011 | AmsUfsAmCmAmAfUmUmUmUmAmAfGmAfU(moe)AfAmUmGmsAmsGm | NO.1110 |
| RZ008099 | GmsAmsUmUmAmCmAfUfCfAfUmCmUmUmUmCmUmGmAm_CR01008×3 | NO.1012 | UmsCfsAmGmAmAfAmGmAmUmGmAfUmGfU(moe)AfAmUmCmsAmsCm | NO.1111 |
| RZ008100 | CmsAmsAmGmCmAmGfCfAfAfAmUmCmCmUmGmGmAmUm_CR01008×3 | NO.1013 | AmsUfsCmCmAmGfGmAmUfUmUmGmCmUfGmCfUmUmGmsGmsCm | NO.1112 |
| RZ008101 | GmsGmsUmCmAmUmCfAfGfCfUmUmUmGmCmUmAmCmUm_CR01008×3 | NO.1014 | AmsGfsUmAmGmCfAmAmAfGmCmUmGmAfUmGfAmCmCmsUmsCm | NO.1113 |
| RZ008102 | CmsAmsGmCmUmUmUfGfCfUfAmCmUmGmUmCmAmCmAm_CR01008×3 | NO.1015 | UmsGfsUmGmAmCfAmGmUfAmGmCmAmAfAmGfCmUmGmsAmsUm | NO.1114 |
| RZ008103 | CmsAmsCmCmCmUmUfCfCfUfGmGmCmAmCmUmCmUmUm_CR01008×3 | NO.1016 | AmsAfsGmAmGmUfGmCmCfAmGmGmAmAfGmGfGmUmGmsGmsGm | NO.1115 |
| RZ008104 | CmsUmsCmUmUmUmGfCfUfUfGmAmGmGmAmUmCmUmUm_CR01008×3 | NO.1017 | AmsAfsGmAmUmCfCmUmCfAmAmGmCmAfAmAfGmAmGmsUmsGm | NO.1116 |
| RZ008105 | GmsCmsUmUmGmAmGfGfAfUfCmUmUmCmCmGmAmUmGm_CR01008×3 | NO.1018 | CmsAfsUmCmGmGfAmAmGfAmUmCmCmUfCmAfAmGmCmsAmsAm | NO.1117 |

(continued)

| Serial number | Modified sense strand (5'-3') | SEQ ID | Modified antisense strand (5'-3') | SEQ ID |
|---|---|---|---|---|
| RZ008106 | CmsGmsAmGmAmCmCfAfUfUfAmCmGmUmAmGmAmCmUm_CR01008×3 | NO.1019 | AmsGfsUmCmUmAfCmGmUfAmAmUmGmGfUmCfUmCmGmsCmsCm | NO.1118 |
| RZ008107 | CmsCmsUmCmCmUmCfAfAfAfGmCmCmAmAmCmAmAmUm_CR01008×3 | NO. 1020 | AmsUfsUmGmUmUfGmGmCfUmUmUmGmAfGmGfAmGmGmsCmsUm | NO.1119 |
| RZ008108 | CmsCmsUmCmUmAmCfCfUfGfGmAmUmCmAmUmAmAmUm_CR01008×3 | NO. 1021 | AmsUfsUmAmUmGfAmUmCfCmAmGmGmUfAmGfAmGmGmsAmsGm | NO. 1120 |
| RZ008109 | CmsCmsUmGmGmAmUfCfAfUfAmAmUmGmGmCmAmAmUm_CR01008×3 | NO. 1022 | AmsUfsUmGmCmCfAmUmUfAmUmGmAmUfCmCfAmGmGmsUmsAm | NO.1121 |
| RZ008110 | GmsCmsAmAmUmGmUfGfGfUfCmAmAmGmAmCmGmGmAm_CR01008×3 | NO. 1023 | UmsCfsCmGmUmCfUmUmGfAmCmCmAmCfAmUfUmGmCmsCmsAm | NO. 1122 |
| RZ008111 | GmsGmsAmGmUmGmAfAfGfAfGmAmCmCmAmAmGmAmUm_CR01008×3 | NO. 1024 | AmsUfsCmUmUmGfGmUmCfUmCmUmUmCfAmCfUmCmCmsAmsAm | NO. 1123 |
| RZ008112 | GmsUmsGmAmAmGmAfGfAfCfCmAmAmGmAmUmGmAmAm_CR01008×3 | NO. 1025 | UmsUfsCmAmUmCfUmUmGfGmUmCmUmCfUmUfCmAmCmsUmsCm | NO. 1124 |
| RZ008113 | GmsCmsAmCmUmAmAfGfCfCfUmAmAmGmAmAmGmUmUm_CR01008×3 | NO. 1026 | AmsAfsCmUmUmCfUmUmAfGmGmCmUmUfAmGfUmGmCmsCmsUm | NO. 1125 |
| RZ008114 | CmsAmsCmUmAmAmUfUfUfUfUmGmUmAmUmUmCmUmUm_CR01008×3 | NO. 1027 | AmsAfsGmAmAmUfAmCmAfAmAmAmAmUfUmAfGmUmGmsGmsGm | NO. 1126 |
| RZ008115 | UmsCmsCmAmCmAmAfAfGfUfCmAmAmAmGmCmUmAmUm_CR01008×3 | NO. 1028 | AmsUfsAmGmCmUfUmUmGfAmCmUmUmUfGmUfGmGmAmsCmsAm | NO. 1127 |
| RZ008116 | CmsCmsAmCmAmAmAfGfUfCfAmAmAmGmCmUmAmUmUm_CR01008×3 | NO. 1029 | AmsAfsUmAmGmCfUmUmUfGmAmCmUmUfUmGfUmGmGmsAmsCm | NO. 1128 |

(continued)

| Serial number | Modified sense strand (5'-3') | SEQ ID | Modified antisense strand (5'-3') | SEQ ID |
|---|---|---|---|---|
| RZ008117 | CmsAmsCmAmAmAmGfUfCfAfAmAmGmCmUmAmUmUmUm_CR01008×3 | NO. 1030 | AmsAfsAmUmAmGfCmUmUfUmGmAmCmUfUmUfGmUmGmsGmsAm | NO. 1129 |
| RZ008118 | CmsAmsAmAmGmCmUfAfUfUfUmUmCmAmUmAmAmUmAm_CR01008×3 | NO.1031 | UmsAfsUmUmAmUfGmAmAfAmAmUmAmGfCmUfUmUmGmsAmsCm | NO.1130 |
| RZ008119 | CmsUmsAmUmUmUmUfCfAfUfAmAmUmAmAmUmAmCmUm_CR01008×3 | NO.1032 | AmsGfsUmAmUmUfAmUmUfAmUmGmAmAfAmAfUmAmGmsCmsUm | NO.1131 |
| RZ008120 | CmsAmsUmAmAmUmAfAfUfAfCmUmAmAmCmAmUmGmUm_CR01008×3 | NO.1033 | AmsCfsAmUmGmUfUmAmGfUmAmUmUmAfUmUfAmUmGmsAmsAm | NO.1132 |
| RZ008121 | CmsAmsUmGmUmUmAfUfUfUfGmCmCmUmUmUmUmGmAm_CR01008×3 | NO. 1034 | UmsCfsAmAmAmAfGmGmCfAmAmAmUmAfAmCfAmUmGmsUmsUm | NO.1133 |
| RZ008122 | GmsUmsUmAmUmUmUfGfCfCfUmUmUmUmGmAmAmUmUm_CR01008×3 | NO.1035 | AmsAfsUmUmCmAfAmAmAfGmGmCmAmAfAmUfAmAmCmsAmsUm | NO.1134 |
| RZ008123 | GmsGmsAmGmGmCmAfUfCfAfGmAmUmUmCmCmUmGmAm_CR01008×3 | NO.1036 | UmsCfsAmGmGmAfAmUmCfUmGmAmUmGfCmCfUmCmCmsAmsGm | NO.1135 |
| RZ008124 | CmsAmsCmUmGmGmGfAfGfAfCmAmAmGmCmAmUmUmUm_CR01008×3 | NO.1037 | AmsAfsAmUmGmCfUmUmGfUmCmUmCmCfCmAfGmUmGmsGmsGm | NO.1136 |
| RZ008125 | CmsAmsAmGmCmAmUfUfUfAfUmAmCmUmUmUmCmUmUm_CR01008×3 | NO.1038 | AmsAfsGmAmAmAfGmUmAfUmAmAmAmUfGmCfUmUmGmsUmsCm | NO.1137 |
| RZ008126 | GmsCmsCmUmGmGmCfUfUfAfUmAmCmUmUmUmCmUmUm_CR01008×3 | NO.1039 | AmsAfsGmAmAmAfGmUmAfUmAmAmGmCfCmAfGmGmCmsGmsCm | NO.1138 |
| RZ008127 | GmsCmsUmUmAmUmAfCfUfUfUmCmUmUmAmAmUmAmAm_CR01008×3 | NO. 1040 | UmsUfsAmUmUmAfAmGmAfAmAmGmUmAfUmAfAmGmCmsCmsAm | NO.1139 |

(continued)

| Serial number | Modified sense strand (5'-3') | SEQ ID | Modified antisense strand (5'-3') | SEQ ID |
|---|---|---|---|---|
| RZ008128 | GmsGmsGmUmGmUmCfCfAfCfAmAmAmGmUmCmAmAmAm_CR01008×3 | NO. 1041 | UmsUfsUmGmAmCfUmUmUfGmUmGmGmAfCmAfCmCmCmsCmsUm | NO. 1140 |
| RZ008129 | GmsCmsCmUmUmUmUfGfAfAfUmUmCmUmCmAmUmUmAm_CR01008×3 | NO. 1042 | UmsAfsAmUmGmAfGmAmAfUmUmCmAmAfAmAfGmGmCmsAmsAm | NO.1141 |
| RZ008130 | GmsAmsAmUmUmCmUfCfAfUfUmAmUmCmUmUmAmAmAm_CR01008×3 | NO. 1043 | UmsUfsUmAmAmGfAmUmAfAmUmGmAmGfAmAfUmUmCmsAmsAm | NO. 1142 |
| RZ008131 | CmsAmsUmUmAmUmCfUfUfAfAmAmAmUmUmGmUmAmUm_CR01008×3 | NO. 1044 | AmsUfsAmCmAmAfUmUmUfUmAmAmGmAfUmAfAmUmGmsAmsGm | NO. 1143 |
| RZ008132 | GmsAmsUmUmAmCmAfUfCfAfUmCmUmUmUmCmUmGmAm_CR01008×3 | NO. 1045 | UmsCfsAmGmAmAfAmGmAfUmGmAmUmGfUmAfAmUmCmsAmsCm | NO. 1144 |

## Biological Testing Experiments

Unless otherwise specified, the reagents and consumables (Table 5) and instruments and equipment (Table 6) used in the present application are all commercially available products from the following manufacturers.

Table 5. Major Reagents and Consumables

| **Name** | **Manufacturer** |
|---|---|
| 1×PBS | M&C Gene Technology (Beijing) Ltd. |
| DMEM Medium | M&C Gene Technology (Beijing) Ltd. |
| Opti-MEM™ Medium | Gibco |
| Fetal Bovine Serum | Sigma |
| Trypsin | M&C Gene Technology (Beijing) Ltd. |
| Penicillin-Streptomycin | BBI |
| Lipofectamine RNAiMax | Invitrogen |
| Nucleic Acid Extraction or Purification Kit | Zhejiang Hanwei Technology Co., Ltd |
| RevertAid First Strand cDNA Synthesis Kit | Thermo Fisher Scientific |
| TaqMan Fast Advanced Master Mix | Thermo Fisher Scientific |
| Hanwei RNA Extraction Kit | Zhejiang Hanwei Technology Co., Ltd |
| RNALater | Thermo Fisher Scientific |

Table 6. Major Instruments and Equipment

| Name | Manufacturer |
|---|---|
| Automated Nucleic Acid Extraction System | Zhejiang Hanwei Technology Co., Ltd |
| High-Speed Refrigerated Centrifuge | Eppendorf |
| $CO_2$ Incubator | Thermo Fisher Scientific |
| Biosafety Cabinet | Shanghai Lishen |
| Thermostat Water Bath | Shanghai Boxun |
| Automated Cell Counter | Shanghai Countstar |
| Inverted Microscope | Olympus |
| NANODROP OneC | Thermo Fisher Scientific |
| Gradient PCR Thermocycler | Eppendorf |
| CFX Opus 384 | Bio-Rad |
| LightCycler 480 | Roche |
| Real-time fluorescence quantitative (RT-PCR) System QuantStudio TM3 | Thermo Fisher Scientific |
| Gel Imager | Shanghai Tanon Life Science Co.,Ltd. |
| Electrophoresis System | Beijing Liuyi |
| Automated Gel Image Analysis System | SYSMEX |
| Tissuelyser II Automated Tissue Homogenizer | Shanghai Jingxin Industrial Development Co., Ltd. |

Unless otherwise specified, the human hepatoma cell line Huh7 used in the present disclosure was purchased from Wuhan Procell Life Science & Technology Co., Ltd.; the experimental animal C57BL/6J mice used in the present disclosure were purchased from Zhejiang Vital River Laboratory Animal Technology Co., Ltd.; the experimental animal Balb/c mice used in the present disclosure were purchased from Zhejiang Vital River Laboratory Animal Technology Co., Ltd.; the BKS-DB mice used in the present disclosure were purchased from Jiangsu GemPharmatech Co., Ltd.; and the serum triglyceride and total cholesterol levels in BKS-DB mice were measured by Anling Biomedical (Suzhou) Co., Ltd.

In the context of the present disclosure, unless otherwise specified, the ΔΔCt method was used for all Real-time PCR detection data for activity assays involved in the present disclosure to calculate the relative quantification of the mRNA of the target gene in each test group. The calculation method is outlined as follows:

$$\Delta Ct\ (\text{Test Group}) = Ct\ (\text{Target Gene in Test Group}) - Ct\ (\text{Reference Gene in Test Group})$$

ΔCt (Control Group) = Ct (Target Gene in Control Group) - Ct (Reference Gene in Control Group)

$$\Delta\Delta Ct\ (\text{Test Group}) = \Delta Ct\ (\text{Test Group}) - \Delta Ct\ (\text{Average of Control Group})$$

$$\Delta\Delta Ct\ (\text{Control Group}) = \Delta Ct\ (\text{Control Group}) - \Delta Ct\ (\text{Average of Control Group})$$

Using the control group as the baseline, the mRNA expression level of the target gene in the test group is normalized, and the residual mRNA expression level of the target gene in the control group is defined as 100%.

Relative Residual Expression Level of Target Gene mRNA in Test Group = $2^{-\Delta\Delta Ct}$ (Test Group) × 100%

Inhibition Rate of Target Gene mRNA in Test Group = 100% - Relative Expression Level of Target Gene mRNA in Test Group

In the context of the present disclosure, unless otherwise specified, *in vivo* activity experimental data are expressed as X±STDEV, and all experimental data are plotted and analyzed using GraphPad Prism 8.0 software.

**Example 1. *In vitro* activity assessment of siRNA**

In this example, RX008001 to RX008154 with the same INHBE target site were evaluated for inhibitory activity against the target gene INHBE in cells using the evaluation method for target gene inhibitory activity in the human hepatoma cell line Huh7, with RX000001 used as a negative control.

Test article preparation:

Each siRNA test article was centrifuged, and an appropriate amount of PBS was added according to the specification per tube to prepare a 20 μM stock solution. The stock solution was then further serially diluted with PBS to prepare 1 μM and 0.1 μM working solutions, or 0.1 μM and 0.01 μM working solutions, before dose test conducted with final duplex concentrations of 10 nM and 1 nM, or final duplex concentration of 1 nM and 0.1 nM.

96-well transfection and detection:

Huh7 cells grown to near confluence were digested with trypsin, and washed to prepare a cell suspension. 100 μL of the cell suspension was added to each well of a 96-well plate, with 12,000 cells per well, incubated at 37°C in a 5% $CO_2$ incubator. After 24 h of cell adherence, the DMEM medium in the 96-well plate was aspirated and discarded, and 80 μL Opti-MEM™ medium was added to each well, and then the 96-well plate was placed into the incubator for further culture; and 1 μL of 1 μM, 0.1 μM or 0.01 μM working solution was dispersed in 9 μL Opti-MEM to form siRNA mixture, and 0.3 μL RNAiMAX was dispersed in 9.7 μL Opti-MEM to mix with each siRNA mixture to form transfection complex. Transfection complexes were incubated for 10 minutes at room temperature and then added into 96-well plates at 20 μL/well. After 4 hours of culture, 100 μL DMEM medium containing 20% FBS was supplemented to each well, and the 96-well plate was placed into the incubator for further culture for 24 hours.

The 96-well plate was retrieved, and total RNA was extracted using an automated nucleic acid extraction system (purchased from Zhejiang Hanwei Technology Co., Ltd.) and a nucleic acid extraction kit (purchased from Zhejiang Hanwei Technology Co., Ltd., GO-MNTR-100) following the standard operating procedure for total RNA extraction.

A reverse transcription kit (Thermo Fisher Scientific Company, RevertAid First Strand cDNA Synthesis Kit, K1622) was used with Oligo $(dT)_{18}$ reverse transcription primers to set up a 20 μL reverse transcription reaction system according to the manufacturer's instructions, and the reverse transcription reaction was performed. Subsequently, real-time quantitative PCR was conducted using a fluorescent quantitative PCR kit (Thermo Fisher Scientific Company, TaqMan Fast Advanced Master Mix, 444557) on a real-time PCR system (Bio-Rad Company CFX Opus 384) to measure the mRNA expression level of the target gene in HepG2 cells. In this real-time PCR assay, the glyceraldehyde-3-phosphate dehydrogenase (GAPDH) gene was used as the reference gene, and the target gene and the GAPDH reference gene are detected using primers for the target gene and primers for the GAPDH reference gene, respectively. The sequences of the detection primers are shown in Table 7.

Table 7. Sequences of Detection Primers

| Gene | | Primer type | Primer sequence | Fluorophore |
|---|---|---|---|---|
| Target gene | INHBE | Upstream primer | 5'-CAACAACCACCTGGCAATATG-3' (SEQ ID NO. 310) | / |
| | | Downstream primer | 5'-ACATCAGCCAACCTGGAATAA-3' (SEQ ID NO.311) | / |
| | | Probe primer | 5'-CCCAAATGGGCACTTTCTTGTCTGAGA-3' (SEQ ID NO.312) | 5'FAM; 3'MGB |

(continued)

| Gene | | Primer type | Primer sequence | Fluorophore |
|---|---|---|---|---|
| Referen ce gene | GAPDH | Upstream primer | 5'-AAGAAGGTGGTGAAGCAGG-3' (SEQ ID NO:313) | / |
| | | Downstream primer | 5'-CAAAGTGGTCGTTGAGGG-3' (SEQ ID NO:314) | / |
| | | Probe primer | 5'-CAACAGCGACACCCACTC-3' (SEQ ID NO:315) | 5'VIC; 3'MGB |

In this real-time fluorescence quantitative PCR assay, the expression levels of the target gene mRNA in each test group were relatively quantitative calculated using the ΔΔCt method according to the technical approach described in the embodiments.

Table 8. Inhibitory activity of target gene in Huh7 cells at 10nM and 1nM doses after siRNA administration

| Group | 10 nM | | 1 nM | |
|---|---|---|---|---|
| | % Relative remaining expression level | STDEV | % Relative remaining expression level | STDEV |
| Mock | 100.00 | 7.21 | 100.00 | 7.21 |
| RX000001 | 116.05 | 20.91 | 111.92 | 25.15 |
| RX008001 | 94.62 | 34.47 | 89.46 | 16.20 |
| RX008002 | 35.40 | 7.64 | 45.10 | 9.88 |
| RX008003 | 35.30 | 0.91 | 57.79 | 10.61 |
| RX008004 | 10.25 | 0.14 | 50.08 | 6.81 |
| RX008005 | 13.25 | 1.39 | 33.55 | 4.83 |
| RX008006 | 40.64 | 14.08 | 73.66 | 3.18 |
| RX008007 | 11.44 | 0.10 | 21.41 | 1.92 |
| RX008008 | 32.79 | 6.65 | 56.63 | 14.56 |
| RX008009 | 25.07 | 7.75 | 40.42 | 8.19 |
| RX008010 | 43.04 | 3.50 | 79.34 | 15.63 |
| RX008011 | 32.36 | 6.60 | 52.44 | 1.05 |
| RX008012 | 17.97 | 0.01 | 46.89 | 4.59 |
| RX008013 | 25.08 | 6.93 | 74.80 | 0.56 |
| RX008014 | 19.95 | 7.26 | 27.96 | 4.60 |
| RX008015 | 16.92 | 2.12 | 20.92 | 4.73 |
| RX008016 | 23.70 | 0.99 | 49.98 | 25.81 |
| RX008017 | 24.13 | 2.46 | 39.38 | 0.00 |
| RX008018 | 16.15 | 1.62 | 54.14 | 9.00 |
| RX008019 | 17.90 | 6.97 | 69.37 | 0.13 |
| RX008020 | 13.68 | 4.54 | 21.06 | 3.33 |
| RX008021 | 12.85 | 1.63 | 52.10 | 0.77 |
| RX008022 | 25.71 | 12.06 | 38.31 | 11.34 |
| RX008023 | 51.97 | 4.67 | 31.64 | 6.53 |
| RX008024 | 8.36 | 0.75 | 16.16 | 3.99 |
| RX008025 | 14.83 | 0.27 | 38.07 | 7.19 |
| RX008026 | 28.36 | 0.77 | 77.56 | 14.30 |
| RX008027 | 40.69 | 4.73 | 75.08 | 5.23 |
| RX008028 | 76.83 | 11.42 | 162.80 | 46.37 |
| RX008029 | 29.85 | 4.90 | 81.58 | 15.02 |
| RX008030 | 72.17 | 8.75 | 107.83 | 44.61 |
| RX008031 | 19.48 | 1.31 | 36.46 | 7.44 |
| RX008032 | 24.15 | 4.06 | 82.62 | 14.88 |
| RX008033 | 25.29 | 4.52 | 48.18 | 9.99 |

(continued)

| Group | 10 nM | | 1 nM | |
|---|---|---|---|---|
| | % Relative remaining expression level | STDEV | % Relative remaining expression level | STDEV |
| RX008034 | 35.55 | 5.96 | 87.32 | 13.61 |
| RX008035 | 12.03 | 1.38 | 22.02 | 0.06 |
| RX008036 | 42.80 | 9.33 | 74.19 | 10.60 |
| RX008037 | 69.57 | 5.50 | 98.22 | 11.15 |
| RX008038 | 22.00 | 4.87 | 55.88 | 10.18 |
| RX008039 | 26.64 | 4.06 | 52.68 | 4.28 |
| RX008040 | 45.72 | 2.78 | 76.67 | 1.16 |
| RX008041 | 17.46 | 1.22 | 35.31 | 0.36 |
| RX008042 | 26.35 | 3.49 | 41.91 | 7.21 |
| RX008043 | 8.44 | 2.10 | 30.56 | 2.41 |
| RX008044 | 12.03 | 1.38 | 22.02 | 0.06 |
| RX008045 | 42.80 | 9.33 | 74.19 | 10.60 |
| RX008046 | 69.57 | 5.50 | 98.22 | 11.15 |
| RX008047 | 22.00 | 4.87 | 55.88 | 10.18 |
| RX008048 | 26.64 | 4.06 | 52.68 | 4.28 |
| RX008049 | 45.72 | 2.78 | 76.67 | 1.16 |
| RX008050 | 17.46 | 1.22 | 35.31 | 0.36 |
| RX008051 | 26.35 | 3.49 | 41.91 | 7.21 |
| RX008052 | 45.33 | 4.12 | 75.75 | 8.49 |
| RX008053 | 14.71 | 2.29 | 23.92 | 1.71 |
| RX008054 | 14.80 | 0.53 | 27.00 | 0.56 |
| RX008055 | 76.22 | 12.22 | 121.13 | 15.47 |
| RX008056 | 20.38 | 0.07 | 41.21 | 0.82 |
| RX008057 | 22.69 | 1.16 | 88.39 | 16.38 |
| RX008058 | 20.58 | 3.54 | 53.00 | 15.80 |
| RX008059 | 24.07 | 3.82 | 62.99 | 25.85 |
| RX008060 | 65.64 | 0.05 | 89.28 | 23.85 |
| RX008061 | 14.09 | 1.15 | 38.15 | 8.53 |
| RX008062 | 40.05 | 0.44 | 42.50 | 1.00 |
| RX008063 | 21.24 | 0.53 | 35.19 | 7.27 |
| RX008064 | 25.48 | 9.26 | 68.16 | 21.50 |
| RX008065 | 8.78 | 0.01 | 15.47 | 1.12 |
| RX008066 | 19.72 | 7.01 | 27.80 | 4.31 |
| RX008067 | 50.32 | 9.04 | 84.93 | 15.88 |
| RX008068 | 25.10 | 3.15 | 66.74 | 1.15 |
| RX008069 | 12.93 | 0.88 | 67.13 | 24.90 |
| RX008070 | 6.95 | 0.92 | 13.35 | 1.08 |
| RX008071 | 3.95 | 0.27 | 11.45 | 1.71 |
| RX008072 | 12.22 | 2.18 | 38.34 | 2.08 |
| RX008073 | 4.07 | 0.62 | 10.68 | 2.06 |
| RX008074 | 4.83 | 0.05 | 15.25 | 3.09 |
| RX008075 | 8.54 | 1.25 | 10.13 | 0.13 |
| RX008076 | 6.24 | 1.95 | 20.98 | 0.54 |
| RX008077 | 30.25 | 3.67 | 76.15 | 2.37 |
| RX008078 | 4.19 | 0.96 | 8.23 | 0.35 |
| RX008079 | 5.92 | 0.48 | 18.74 | 2.01 |
| RX008080 | 21.69 | 12.01 | 50.86 | 0.85 |
| RX008081 | 13.12 | 0.53 | 41.71 | 4.53 |
| RX008082 | 21.92 | 3.66 | 67.41 | 1.44 |

(continued)

| Group | 10 nM | | 1 nM | |
|---|---|---|---|---|
| | % Relative remaining expression level | STDEV | % Relative remaining expression level | STDEV |
| RX008083 | 10.48 | 1.18 | 34.16 | 2.45 |
| RX008084 | 9.73 | 1.83 | 28.17 | 1.46 |
| RX008085 | 12.56 | 0.20 | 52.28 | 15.00 |
| RX008086 | 16.60 | 6.24 | 37.20 | 10.46 |
| RX008087 | 9.56 | 1.50 | 52.96 | 0.00 |
| RX008088 | 19.66 | 2.20 | 57.90 | 0.42 |
| RX008089 | 52.04 | 0.80 | 144.44 | 17.01 |
| RX008090 | 23.33 | 5.16 | 72.68 | 1.86 |
| RX008091 | 12.04 | 0.07 | 40.12 | 2.99 |
| RX008092 | 8.66 | 0.13 | 18.52 | 7.04 |
| RX008093 | 9.40 | 1.01 | 26.28 | 8.48 |
| RX008094 | 15.56 | 2.89 | 69.94 | 17.34 |
| RX008095 | 26.99 | 0.59 | 92.51 | 4.13 |
| RX008096 | 23.71 | 0.76 | 71.23 | 7.82 |
| RX008097 | 34.12 | 0.54 | 106.00 | 0.00 |
| RX008098 | 22.83 | 5.82 | 28.80 | 1.14 |
| RX008099 | 16.98 | 0.88 | 35.40 | 0.18 |
| RX008100 | 10.61 | 1.21 | 34.68 | 8.44 |
| RX008101 | 8.83 | 1.31 | 23.38 | 0.16 |
| RX008102 | 8.16 | 0.44 | 19.29 | 2.24 |
| RX008103 | 20.57 | 7.41 | 65.58 | 13.94 |
| RX008104 | 10.10 | 0.91 | 19.59 | 1.22 |
| RX008105 | 20.42 | 2.79 | 22.41 | 10.70 |
| RX008106 | 14.29 | 2.39 | 62.93 | 10.63 |
| RX008107 | 45.61 | 14.13 | 42.40 | 4.21 |
| RX008108 | 55.44 | 12.81 | 63.87 | 9.40 |
| RX008109 | 37.45 | 5.92 | 57.33 | 18.11 |
| RX008110 | 16.49 | 1.54 | 39.82 | 5.37 |
| RX008111 | 19.50 | 0.50 | 35.57 | 4.73 |
| RX008112 | 23.58 | 0.55 | 60.61 | 4.44 |
| RX008113 | 8.37 | 0.62 | 24.38 | 5.23 |
| RX008114 | 10.77 | 5.62 | 35.24 | 15.40 |
| RX008115 | 11.67 | 0.22 | 26.35 | 5.38 |
| RX008116 | 6.97 | 0.32 | 9.71 | 0.08 |
| RX008117 | 12.19 | 0.29 | 12.91 | 0.88 |
| RX008118 | 6.59 | 1.45 | 14.36 | 2.95 |
| RX008119 | 14.09 | 5.99 | 15.38 | 1.08 |
| RX008120 | 16.41 | 0.28 | 32.98 | 4.24 |
| RX008121 | 19.74 | 4.34 | 14.45 | 0.46 |
| RX008122 | 14.03 | 0.04 | 12.03 | 0.03 |
| RX008123 | 10.79 | 0.16 | 15.73 | 9.94 |
| RX008124 | 8.75 | 3.10 | 10.35 | 1.86 |
| RX008125 | 9.84 | 1.66 | 9.24 | 1.04 |
| RX008126 | 16.38 | 1.23 | 27.15 | 4.23 |
| RX008127 | 8.44 | 2.10 | 30.56 | 2.41 |
| RX008128 | 10.88 | 2.25 | 16.10 | 4.10 |

Table 9. Inhibitory activity of target gene in Huh7 cells at 1nM and 0.1nM doses after siRNA administration

| Group | 1 nM | | 0.1 nM | |
|---|---|---|---|---|
| | % Relative remaining expression level | STDEV | % Relative remaining expression level | STDEV |
| Mock | 100.00 | 4.64 | 100.00 | 4.64 |
| RX008129 | 61.59 | 4.58 | 88.90 | 2.80 |
| RX008130 | 42.57 | 4.27 | 83.39 | 11.10 |
| RX008131 | 26.85 | 3.14 | 77.32 | 3.28 |
| RX008132 | 23.54 | 0.80 | 69.29 | 5.89 |
| RX008133 | 23.31 | 3.45 | 68.22 | 34.61 |
| RX008134 | 26.05 | 7.75 | 80.57 | 9.47 |
| RX008135 | 15.42 | 0.36 | 81.57 | 6.65 |
| RX008136 | 23.85 | 2.92 | 79.29 | 0.95 |
| RX008137 | 64.21 | 0.43 | 95.59 | 2.45 |
| RX008138 | 37.87 | 2.20 | 81.65 | 6.19 |
| RX008139 | 33.74 | 3.35 | 84.05 | 1.43 |
| RX008140 | 15.34 | 0.80 | 55.54 | 7.34 |
| RX008141 | 15.52 | 0.04 | 73.49 | 2.37 |
| RX008142 | 8.61 | 0.34 | 30.85 | 8.25 |
| RX008143 | 93.95 | 9.14 | 116.49 | 5.58 |
| RX008144 | 11.80 | 0.28 | 53.26 | 2.44 |
| RX008145 | 8.44 | 0.75 | 29.82 | 1.86 |
| RX008146 | 13.76 | 3.64 | 31.66 | 1.02 |
| RX008147 | 20.16 | 0.98 | 48.79 | 3.35 |
| RX008148 | 18.22 | 0.10 | 31.40 | 3.11 |
| RX008149 | 8.24 | 11.65 | 38.51 | 3.07 |
| RX008150 | 54.29 | 3.87 | 65.72 | 15.44 |
| RX008151 | 25.77 | 1.76 | 56.15 | 10.01 |
| RX008152 | 51.23 | 10.65 | 52.47 | 5.92 |
| RX008153 | 78.70 | 5.83 | 61.88 | 12.57 |
| RX008154 | 61.22 | 9.47 | 54.30 | 3.57 |

**Example 2. *In vivo* activity assessment of siRNA conjugates with L96 ligand in high pressure hydrodynamic injection model (HDI) mice**

In this example, siRNA with the same INHBE target site and conjugated with L96 ligand at the 3 'end of the sense strand were evaluated for inhibitory activity against the target gene INHBE using Balb/c mice hydrodynamic injection model.

Plasmid Construction: pcDNA-CMV-RG008 plasmid (ID: NM_031479.5) was constructed by Sangon Biotech (Shanghai) Co., Ltd.

Mouse model construction:

The Balb/c mouse hydrodynamic injection model was established by rapidly injecting of the pcDNA-CMV-RG008 plasmid solution into mice via the tail vein at high pressure. On day 3 of the experiment, mice were hydrodynamically injected with 10 μg pcDNA-CMV-RG008 via the tail vein within 5 seconds at an injection volume of 8% of the mouse body weight. Plasmid DNA for injection was diluted with physiological saline and the solution was prepared prior to injection and stored at 4°C.

Animal grouping, dosing and tissue sample collection:

Balb/c mice (all females) aged 6-8 weeks were randomly divided into groups based on body weight, with 5 mice per group. The dose for all mice was calculated according to body weight. A single dose was administered via

subcutaneous injection in the abdominal region. Each drug conjugate was administered as a solution in PBS at a concentration of 0.1 mg/mL (calculated based on siRNA), with an administration volume of 10 mL per kg of body weight, i.e., an administration dose of 1 mg/kg mouse body weight (calculated as siRNA) per drug conjugate. The PBS control group was given the same volume of PBS solution (without the drug conjugate). The day of administration was recorded as day 0 (recorded as D0), plasmid injection was performed on day 3 after administration (recorded as D3) and 5 mice of all groups were euthanized on day 4 (recorded as D4). A gross dissection was performed on each euthanized mouse, and liver tissue was collected from each mouse. The liver tissue was cut into approximately 2 $mm^3$ pieces and preserved in RNA later.

For each mouse, an appropriate amount of liver tissue samples were taken from RNA later, the liver tissue samples were crushed in Tissuelyser II automatic tissue homogenizer for 60s, and then total RNA was extracted by automatic nucleic acid extractor (purchased from Zhejiang Hanwei Technology Co., Ltd.) and nucleic acid extraction kit (purchased from Zhejiang Hanwei Technology Co., Ltd., GO-MNTR-100) according to the standard operation procedures for total RNA extraction.

For each mouse, 1 μg of total RNA was taken, and reverse transcription kit (Thermo Fisher Scientific Company, RevertAid First Strand cDNA Synthesis Kit, K1622) was used with Oligo $(dT)_{18}$ reverse transcription primers to set up a 20 μL reverse transcription reaction system according to the manufacturer's instructions, and the reverse transcription reaction was performed. After the reaction was complete, 60 μL of RNase-Free water was added to the reverse transcription system to obtain the cDNA solution. Subsequently, real-time quantitative PCR was conducted using a fluorescent quantitative PCR kit (Thermo Fisher Scientific Company, TaqMan Fast Advanced Master Mix, 444557) on a real-time PCR system (Bio-Rad Company CFX Opus 384) to measure the mRNA expression level of the target gene in animal cells. In this real-time PCR assay, the Nero gene on the plasmid backbone gene was used as the reference gene, and the target gene and the Nero reference gene are detected using primers for the target gene and primers for the Nero reference gene, respectively. The sequences of the detection primers are shown in Table 10.

Table 10. Sequences of Detection Primers

| Gene | | Primer type | Primer sequence | Fluorophore |
|---|---|---|---|---|
| Target gene | INHBE | Upstream primer | 5'- CAACAACCACCTGGCAATATG -3' (SEQ ID NO.310) | / |
| | | Downstream primer | 5'- ACATCAGCCAACCTGGAATAA -3' (SEQ ID NO.311) | / |
| | | Probe primer | 5'- CCCAAATGGGCACTTTCTTGTCTGAGA - 3' (SEQ ID NO.312) | 5'FAM; 3'MGB |
| Reference gene | NERO | Upstream primer | 5'-CGTTGGCTACCCGTGATATT-3' (SEQ ID NO.316) | / |
| | | Downstream primer | 5'-CTCGTCAAGAAGGCGATAGAAG-3' (SEQ ID NO.317) | / |
| | | Probe primer | 5'-CCGCTTCCTCGTGCTTTACGGTAT-3' (SEQ ID NO.318) | 5'VIC; 3'MGB |

A 10 μL Real-time PCR reaction mixture was prepared for each PCR detection well according to the instructions of the real-time fluorescence quantitative PCR kit. Each reaction mixture contained 4 μL of the cDNA solution obtained from the reverse transcription reaction described above, 5 μL of TaqMan™ Fast Advanced Master Mix (2×), 0.15 μL of 10 μM forward primer, 0.15 μL of 10 μM reverse primer, 0.15 μL of 10 μM probe, and 0.55 μL of RNase-Free $H_2O$. The prepared reaction mixtures were placed in a real-time fluorescence quantitative PCR instrument (Bio-Rad Company, CFX Opus 384), and Real-time PCR amplification was performed using a two-step method with the following amplification program: 50 °C for 2 min, followed by predenaturation at 95 °C for 20 s, denaturation at 95 °C for 3 s and annealing/extension at 60 °C for 30 s, the process of denaturation, annealing and extension was repeated for 40 cycles. In this real-time fluorescence quantitative PCR assay, the expression levels of the target gene mRNA in each test group were relatively quantitative calculated using the ΔΔCt method according to the technical approach described in the embodiments.

Table 11. Inhibitory activity of target gene in Balb/c-HDI mice after siRNA conjugates administration

| Group | 1 mg/kg | |
|---|---|---|
| | % Relative remaining expression level | STDEV |
| PBS | 100.00 | 11.73 |
| RZ008001 | 74.12 | 20.25 |
| RZ008002 | 68.51 | 20.61 |
| RZ008003 | 28.78 | 10.63 |
| RZ008004 | 57.73 | 15.39 |
| RZ008005 | 49.75 | 8.27 |
| RZ008006 | 83.92 | 8.38 |
| RZ008007 | 72.04 | 23.94 |
| RZ008008 | 91.00 | 10.98 |
| RZ008009 | 59.65 | 6.52 |
| RZ008010 | 41.89 | 11.35 |
| RZ008011 | 85.62 | 5.22 |
| RZ008012 | 77.60 | 8.85 |
| RZ008013 | 105.31 | 35.74 |
| RZ008014 | 82.15 | 31.31 |
| RZ008015 | 36.35 | 6.91 |
| RZ008016 | 48.18 | 15.82 |
| RZ008017 | 33.11 | 3.78 |
| RZ008018 | 42.99 | 15.22 |
| RZ008019 | 23.65 | 4.86 |
| RZ008020 | 16.56 | 2.62 |
| RZ008021 | 55.08 | 11.46 |
| RZ008022 | 36.97 | 8.97 |
| RZ008023 | 24.97 | 7.42 |
| RZ008024 | 92.70 | 22.66 |
| RZ008025 | 56.34 | 14.46 |
| RZ008026 | 28.08 | 4.89 |
| RZ008027 | 68.62 | 14.28 |
| RZ008028 | 28.42 | 10.14 |
| RZ008029 | 44.84 | 13.13 |
| RZ008030 | 46.14 | 14.53 |
| RZ008031 | 33.74 | 6.96 |
| RZ008032 | 58.59 | 8.45 |
| RZ008033 | 119.64 | 20.91 |

As demonstrated by the results in FIG. 1 and Table 11, RZ008003, RZ008019, RZ008020, RZ008023, RZ008026 and RZ008028 can significantly inhibit INHBE mRNA expression at a dose of 1 mg/kg, with an inhibition rate of more than 70%.

**Example 3. Activity assessment of siRNA conjugates with L96 ligand in C57BL/6J Mice**

In this example, siRNA conjugates RX008001 to RX008154 with L96 ligand were evaluated for mRNA inhibitory activity in liver tissue of C57BL/6J mice, with RX000001 used as a negative control.

Animal grouping, dosing and tissue sample collection:

C57BL/6J male mice aged 6-8 weeks (Zhejiang Vital River Laboratory Animal Technology Co., Ltd.) were randomly divided into groups based on body weight, with 5 mice per group. The dose for all mice was calculated according to body weight, with an administration volume of 10 mL per kg of body weight. A single dose was administered via subcutaneous injection in the abdominal region. Each drug conjugate was respectively administered as a solution in PBS

at a concentration of 0.3 mg/mL (calculated based on siRNA), i.e., an administration dose of 3 mg/kg mouse body weight (calculated as siRNA) per drug conjugate. The PBS control group was given the same volume of PBS solution (without the drug conjugate).

The day of administration was recorded as day 0 (recorded as D0), and all groups of mice were euthanized on day 7 after administration (recorded as D7). A gross dissection was performed on each euthanized mouse, and liver tissue was collected from each mouse. The liver tissue was cut into approximately 2 $mm^3$ pieces and preserved in RNA later for determination of INHBE mRNA expression levels. In this real-time fluorescence quantitative PCR assay, the GAPDH gene was used as the reference gene. Specific primers for the target gene and for the GAPDH reference gene were respectively used for detection. The sequences of the detection primers are shown in Table 12.

Table 12. Sequences of Detection Primers

| Gene | | Primer type | Primer sequence | Fluorophore |
|---|---|---|---|---|
| Target gene | INHBE | Upstream primer | 5'-GGATCCATACTGACACCCAATAA-3' (SEQ ID NO. 319) | / |
| | | Downstream primer | 5'-CAGCCTGAAATAGGCCAGAA-3' (SEQ ID NO.320) | / |
| | | Probe primer | 5'-CTGTGTAGCAGTATGCCTGGGTTTGAC-3' (SEQ ID NO.321) | 5'FAM; 3'MGB |
| Reference gene | GAPDH | Upstream primer | 5'-CCTTCCGTGTTCCTACCC-3' (SEQ ID NO:322) | / |
| | | Downstream primer | 5'-GAGACAACCTGGTCCTCA-3' (SEQ ID NO:323) | / |
| | | Probe primer | 5'-CCGCCTGGAGAAACCTGC-3' (SEQ ID NO:324) | 5'VIC; 3'MGB |

In this real-time fluorescence quantitative PCR assay, the expression levels of the target gene mRNA in each test group were relatively quantitative calculated using the ΔΔCt method according to the technical approach described in the embodiments.

Table 13. Inhibitory activity of target gene in liver tissue of C57BL/6J mice after siRNA conjugates administration

| Group | 3 mg/kg | |
|---|---|---|
| | % Relative remaining expression level | STDEV |
| PBS | 100.00 | 28.01 |
| RZ000001 | 114.70 | 39.31 |
| RZM08001 | 88.22 | 28.68 |
| RZM08002 | 72.34 | 18.37 |
| RZM08003 | 64.81 | 22.50 |
| RZM08004 | 93.58 | 22.64 |
| RZM08005 | 119.23 | 45.42 |
| RZM08006 | 72.85 | 15.24 |
| RZM08007 | 113.53 | 24.06 |
| RZM08008 | 92.77 | 37.35 |
| RZM08009 | 122.99 | 13.77 |
| RZM08010 | 112.86 | 36.00 |
| RZM08011 | 68.59 | 27.63 |
| RZM08012 | 112.25 | 23.10 |
| RZM08013 | 78.41 | 26.29 |
| RZM08014 | 78.77 | 39.40 |

(continued)

| Group | 3 mg/kg | |
|---|---|---|
| | % Relative remaining expression level | STDEV |
| RZM08015 | 42.97 | 2.63 |
| RZM08016 | 89.73 | 13.10 |
| RZM08017 | 60.11 | 27.63 |
| RZM08018 | 94.26 | 11.70 |
| RZM08019 | 30.55 | 6.85 |
| RZM08020 | 63.43 | 11.98 |
| RZM08021 | 99.83 | 33.29 |
| RZM08022 | 68.72 | 21.50 |
| RZM08023 | 99.88 | 30.68 |
| RZM08024 | 90.26 | 29.27 |

As demonstrated by the results in FIG. 2 and Table 13, RZM08015 and RZM08019 can significantly reduce the mRNA expression level in liver tissue of C57BL/6J mice 7 days after single subcutaneous administration at 3 mg/kg, among which inhibition effect of RZM08019 mRNA reached 70%.

**Example 4. *In vivo* activity assessment of siRNA conjugates with L96 ligand in C57BL/6J mice**

In this example, L96 carrier conjugate RZM08019 at different doses after single administration were evaluated for mRNA inhibitory activity in liver tissue of C57BL/6J mice.

Animal grouping, dosing and tissue sample collection:

C57BL/6J male mice aged 6-8 weeks (Zhejiang Vital River Laboratory Animal Technology Co., Ltd.) were randomly divided into groups based on body weight, with 5 mice per group. The dose for all mice was calculated according to body weight, with an administration volume of 10 mL per kg of body weight. A single dose was administered via subcutaneous injection in the abdominal region. Each drug conjugate was respectively administered as a solution in PBS at a concentration of 0.9 mg/mL, 0.6 mg/mL, 0.3 mg/mL, 0.1 mg/mL and 0.03 mg/mL (calculated based on siRNA), i.e., an administration dose of 9 mg/kg, 6 mg/kg, 3 mg/kg, 1 mg/kg, and 0.3 mg/kg mouse body weight (calculated as siRNA) per drug conjugate. The PBS control group was given the same volume of PBS solution (without the drug conjugate).

The day of administration was recorded as day 0 (recorded as D0), and all groups of mice were euthanized on day 7 after administration (recorded as D7). A gross dissection was performed on each euthanized mouse, and liver tissue was collected from each mouse. The liver tissue was cut into approximately 2 $mm^3$ pieces and preserved in RNA later for determination of INHBE mRNA expression levels. In this real-time fluorescence quantitative PCR assay, the GAPDH gene was used as the reference gene. Specific primers for the target gene and for the GAPDH reference gene were used for detection of the target gene and the GAPDH reference gene, respectively. The sequences of the detection primers are shown in Table 12.

In this real-time fluorescence quantitative PCR assay, the expression levels and inhibition rate of the target gene mRNA in each test group were relatively quantitative calculated using the ΔΔCt method according to the technical approach described in the embodiments.

Table 14. Inhibitory activity of target genes in C57BL/6J mice after administration of different doses of RZM08019

| Group | Dosage | % Relative remaining expression level | STDEV |
|---|---|---|---|
| PBS | - | 100.00 | 27.00 |
| RZM08019 | 9 mg/kg | 12.30 | 1.92 |
| RZM08019 | 6mg/kg | 15.92 | 4.35 |
| RZM08019 | 3 mg/kg | 32.81 | 7.69 |
| RZM08019 | 1 mg/kg | 58.04 | 17.95 |
| RZM08019 | 0.3 mg/kg | 60.25 | 15.73 |

The data results in Table 14 and FIG. 3 demonstrate that RZM08019 can dose-dependently reduce mRNA

expression levels in liver tissue of C57BL/6J mice.

**Example 5. *In vivo* pharmacodynamic evaluation of siRNA conjugate RZM08019 in BKS-DB mice**

In this example, the inhibitory activity of RZM08019 on target gene INHBE in mice was evaluated by using the method for evaluating the inhibitory activity of target gene in mice, and the lipid regulation effect of RZM08019 in mice was evaluated by detecting the changes of triglyceride and total cholesterol in serum of mice by using an automatic biochemical instrument.

Animal grouping, dosing and tissue sample collection:

BKS-DB male mice aged 6-8 weeks (Jiangsu GemPharmatech Co., Ltd.) were randomly divided into groups based on body weight, 6 mice in the PBS group, and 6 mice in the drug conjugate RZM08019 Q2W×2 group (administered once every two weeks, for a total of two doses). The dose for all mice was calculated according to body weight, with an administration volume of 10 mL per kg of body weight. A RZM08019 repeated dose was administered via subcutaneous injection in the abdominal region, once every two weeks, for a total of two doses. Each drug conjugate was administered as a solution in PBS at a concentration of 0.9 mg/mL (calculated based on siRNA), i.e., an administration dose of 9 mg/kg mouse body weight (calculated as siRNA) per drug conjugate. The PBS control group was given the same volume of PBS solution (without the drug conjugate). The day of administration was recorded as day 0 (recorded as D0), on day 7, day 14 (recorded as D14), day 21 (recorded as D21), day 28 (recorded as D28), and day 35 (recorded as D35) after administration, serum was collected from all groups of mice and sent to Anling Biomedical (Suzhou) Co., Ltd. for the measurement of serum triglyceride and total cholesterol levels using an automated biochemistry analyzer.

All groups of mice were euthanized on day 35 after administration (recorded as D35). A gross dissection was performed on each euthanized mouse, and liver tissue was collected from each mouse. The liver tissue was cut into approximately 2 $mm^3$ pieces and preserved in RNA later for determination of INHBE mRNA expression levels. In this real-time fluorescence quantitative PCR assay, the GAPDH gene was used as the reference gene. Specific primers for the target gene and for the GAPDH reference gene were used for detection of the target gene and the GAPDH reference gene, respectively. The sequences of the detection primers are shown in Table 12.

In this real-time fluorescence quantitative PCR assay, the expression levels and inhibition rate of the target gene mRNA in each test group were relatively quantitative calculated using the ΔΔCt method according to the technical approach described in the embodiments.

Table 15. Triglyceride levels in serum of BKS-DB mice after repeated administration of RZM08019

| Group | DO(mmol/L) | | D7(mmol/L) | | D14(mmol/L) | | D21(mmol/L) | | D28(mmol/L) | | D35(mmol/L) | |
|---|---|---|---|---|---|---|---|---|---|---|---|---|
| | Mean | STDEV | Mean | STDEV | Mean | STDEV | Mean | STDEV | Mean | STDEV | Mean | STDEV |
| PBS | 3.13 | 1.37 | 2.47 | 0.57 | 2.91 | 1.20 | 3.13 | 0.58 | 3.21 | 0.71 | 2.92 | 0.69 |
| RZM08019 9 mg/kg Q2W×2 | 2.08 | 0.35 | 1.76 | 0.51 | 2.48 | 0.66 | 2.06 | 0.72 | 1.56 | 0.36 | 1.49 | 0.28 |

Table 16. Total cholesterol levels in serum of BKS-DB mice after repeated administration of RZM08019

| Group | D0(mmol/L) | | D7(mmol/L) | | D14(mmol/L) | | D21(mmol/L) | | D28(mmol/L) | | D35(mmol/L) | |
|---|---|---|---|---|---|---|---|---|---|---|---|---|
| | Mean | STDEV | Mean | STDEV | Mean | STDEV | Mean | STDEV | Mean | STDEV | Mean | STDEV |
| PBS | 3.00 | 0.85 | 2.96 | 0.75 | 3.32 | 0.68 | 3.19 | 0.72 | 3.12 | 0.29 | 3.02 | 0.48 |
| RZM08019 9 mg/kg Q2W×2 | 3.53 | 0.89 | 2.72 | 0.37 | 2.70 | 0.23 | 1.93 | 0.67 | 1.76 | 0.61 | 1.67 | 0.35 |

Table 17. INHBE mRNA expression level in liver tissue of BKS-DB mice after repeated administration of RZM08019

| Group | D35 | |
|---|---|---|
| | % Relative remaining expression level | STDEV |
| PBS | 100.00 | 31.08 |
| RZM08019 9 mg/kg Q2W×2 | 12.82 | 2.74 |

The data results in Tables 15-17 and FIGs. 4-5 demonstrate that RZM08019 can significantly reduce CHO expression level in serum of BKS-DB mice after treatment by subcutaneous injection of RZM08019 Q2W x2 conjugate at 9 mg/kg; RZM08019 can significantly reduce mRNA expression level in liver tissue of mice observed in D35 of the test, and the inhibition effect is greater than 85%.

**Example 6. *In vitro* activity assessment of siRNA conjugates with ligand CR01008x3**

In this example, CR01008-siRNA conjugate were evaluated for inhibitory activity against the target gene INHBE in cells using the evaluation method for target gene inhibitory activity in the human hepatoma cell line Huh7.

Test article preparation:

Each siRNA test article was centrifuged, and an appropriate amount of PBS was added according to the specification per tube to prepare a 20 μM stock solution. The stock solution was then further serially diluted with PBS to prepare 0.1 μM working solutions, before dose test conducted with final duplex concentrations of 1 nM.

96-well transfection and detection:

Huh7 cells grown to near confluence were digested with trypsin,and washed to prepare a cell suspension. 100 μL of the cell suspension was added to each well of a 96-well plate, with 12,000 cells per well, incubated at 37°C in a 5% $CO_2$ incubator. After 24 h of cell adherence, the DMEM medium in the 96-well plate was aspirated and discarded, and 80 μL Opti-MEM™ medium was added to each well, and then the 96-well plate was placed into the incubator for further culture; and 1 μL of 0.1 μM working solution was dispersed in 9 μL Opti-MEM to form siRNA mixture, and 0.3 μL RNAiMAX was dispersed in 9.7 μL Opti-MEM to mix with each siRNA mixture to form transfection complex. Transfection complexes were incubated for 10 minutes at room temperature and then added into 96-well plates at 20 μL/well. After 4 hours of culture, 100 μL DMEM medium containing 20% FBS was supplemented to each well, and the 96-well plate was placed into the incubator for further culture for 24 hours.

The 96-well plate was retrieved, and total RNA was extracted using an automated nucleic acid extraction system (purchased from Zhejiang Hanwei Technology Co., Ltd.) and a nucleic acid extraction kit (purchased from Zhejiang Hanwei Technology Co., Ltd., GO-MNTR-100) following the standard operating procedure for total RNA extraction.

A reverse transcription kit (Thermo Fisher Scientific Company, RevertAid First Strand cDNA Synthesis Kit, K1622) was used with Oligo $(dT)_{18}$ reverse transcription primers to set up a 20 μL reverse transcription reaction system according to the manufacturer's instructions, and the reverse transcription reaction was performed. Subsequently, real-time quantitative PCR was conducted using a fluorescent quantitative PCR kit (Thermo Fisher Scientific Company, TaqMan Fast Advanced Master Mix, 444557) on a real-time PCR system (Bio-Rad Company CFX Opus 384) to measure the mRNA expression level of the target gene in HepG2 cells. In this real-time PCR assay, the glyceraldehyde-3-phosphate dehydrogenase (GAPDH) gene was used as the reference gene, and the target gene and the GAPDH reference gene are detected using primers for the target gene and primers for the GAPDH reference gene, respectively. The sequences of the detection primers are shown in Table 7.

In this real-time fluorescence quantitative PCR assay, the expression levels and inhibition rate of the target gene mRNA in each test group were relatively quantitative calculated using the ΔΔCt method according to the technical approach described in the embodiments.

Table 18. Inhibitory activity of target genes in Huh7 cells at 1nM dose after siRNA conjugates administration

| Group | 1 nM | |
|---|---|---|
| | % Remaining activity | STDEV |
| Mock | 100.00 | 9.80 |
| RZ008036 | 14.39 | 1.21 |

(continued)

| Group | 1 nM | |
|---|---|---|
| | % Remaining activity | STDEV |
| RZ008053 | 15.50 | 1.04 |
| RZ008056 | 13.44 | 2.81 |
| RZ008059 | 7.68 | 0.50 |
| RZ008061 | 7.10 | 1.24 |
| RZ008064 | 10.83 | 4.62 |

The data results in Table 18 and FIG. 6 demonstrate that siRNA conjugates provided in this example can significantly inhibit INHBE mRNA expression levels in Huh7 cells.

Finally, it should be noted that: The above embodiments are only used to illustrate, rather than limit, the technical solutions of the present disclosure; although the present disclosure has been described in detail with reference to the foregoing examples, those of ordinary skill in the art should understand that: they may still modify the technical solutions described in the foregoing examples, or perform equivalent replacements for some or all of the technical features; however, these modifications or replacements do not cause the essence of the corresponding technical solutions to depart from the scope of the technical solutions of the examples of the present disclosure.

## Claims

1. A double-stranded oligonucleotide targeting the INHBE gene, wherein the double-stranded oligonucleotide comprises a sense strand and an antisense strand, wherein the antisense strand is complementary or substantially complementary to the sense strand; said substantially complementary means that the sense strand and the antisense strand have a mismatch of no more than 3 nucleotides in the duplex region; wherein the sense strand comprises a nucleotide sequence that is identical or substantially identical to at least 15 contiguous nucleotides in the sequence of SEQ ID NO: 309; and said substantially identical means that there are no more than 3 nucleotide differences between the sense strand and at least 15 contiguous nucleotides in the sequence of SEQ ID NO: 309.

2. The double-stranded oligonucleotide according to claim 1, wherein the sense strand comprises a nucleotide sequence having no more than 2 nucleotide differences from at least 15 contiguous nucleotides in the sequence of SEQ ID NO: 309, preferably no more than 1 nucleotide difference.

3. The double-stranded oligonucleotide of claim 1, wherein the antisense strand is complementary or substantially complementary to at least 15, 16, 17, 18, 19, 20, 21, 22, 23 contiguous nucleotides of the nucleotide sequence of SEQ ID NO: 309, said substantially complementary means having a mismatch of no more than 3 nucleotides in the complementary region.

4. The double-stranded oligonucleotide of claim 1, wherein the double-stranded oligonucleotide comprises a sense strand and an antisense strand, wherein the antisense strand comprises at least 15 contiguous nucleotides of any one of the sequences shown in SEQ ID NO: 155 - SEQ ID NO: 308 in Table 1, or a nucleotide sequence having no more than 3 nucleotide differences from the at least 15 contiguous nucleotides;
   the sense strand comprises a nucleotide sequence that is at least partially reverse complementary or substantially complementary to the antisense strand to form a duplex region; said substantially complementary means that the sense strand and the antisense strand have a mismatch of no more than 3 nucleotides in the duplex region.

5. The double-stranded oligonucleotide according to claim 4, wherein the antisense strand comprises at least 15, at least 16, at least 17, at least 18, at least 19, at least 20, at least 21 contiguous nucleotides of any one of the nucleotide sequences shown in SEQ ID NO: 155 - SEQ ID NO: 308 in Table 1, or a nucleotide sequence having no more than 3 nucleotide differences from the contiguous nucleotides;

   optionally, the antisense strand comprises at least 17, at least 18, at least 19, at least 20, at least 21 contiguous nucleotides of any one of the nucleotide sequences shown in SEQ ID NO: 155 - SEQ ID NO: 308 in Table 1, or a nucleotide sequence having no more than 2 nucleotide differences from the contiguous nucleotides;
   optionally, the antisense strand comprises at least 19, at least 20, at least 21 contiguous nucleotides of any one of the nucleotide sequences shown in SEQ ID NO: 155 - SEQ ID NO: 308 in Table 1, or a nucleotide sequence having

no more than 1 nucleotide difference from the contiguous nucleotides;
optionally, the antisense strand is selected from or comprises any one of the nucleotide sequences shown in SEQ ID NO: 155 - SEQ ID NO: 308 in Table 1;
optionally, as numbered in the 5' to 3' direction, positions 2-19 of the antisense strand comprise at least 15 nucleotides of the nucleotides at positions 2-19 of any one of the nucleotide sequences shown in SEQ ID NO. 155 to SEQ ID NO. 308 in Table 1, or a nucleotide sequence having less than 3 nucleotide differences from the at least 15 nucleotides.

**6.** The double-stranded oligonucleotide according to claim 4, wherein the sense strand comprises at least 13, at least 14, at least 15, at least 16, at least 17, at least 18, at least 19 contiguous nucleotides of any one of the nucleotide sequences shown in SEQ ID NO. 1 to SEQ ID NO. 154 in Table 1, or a nucleotide sequence having no more than 3 nucleotide differences from the contiguous nucleotides;

optionally, the sense strand comprises at least 15, at least 16, at least 17, at least 18, at least 19 contiguous nucleotides of any one of the nucleotide sequences shown in SEQ ID NO. 1 to SEQ ID NO. 154 in Table 1;
optionally, the sense strand is selected from or comprises any one of the nucleotide sequences shown in SEQ ID NO. 1 to SEQ ID NO. 154 in Table 1.

**7.** The double-stranded oligonucleotide according to claim 4, wherein each nucleotide in the double-stranded oligonucleotide is independently selected from unmodified or modified nucleotides;

optionally, substantially all nucleotides of the sense strand or the antisense strand are selected from modified nucleotides;
optionally, all nucleotides of the sense strand and the antisense strand are selected from modified nucleotides.

**8.** The double-stranded oligonucleotide according to claim 7, wherein the modified nucleotides are each independently selected from 2'-halo, 2'-deoxy, 2'-O-$(CH_2)_n$-$R_1$ modified nucleotides, or nucleotide analogs; wherein the nucleotide analogs are selected from one or more of PNA, MNA, BNA, LNA, GNA, TNA, and UNA;

n is selected from 0, 1, or 2; $R_1$ is selected from optionally substituted $C_{1-6}$ alkyl, optionally substituted $C_{1-6}$ alkoxy or -Si$(R_{1a})_3$; each $R_{1a}$ is independently selected from optionally substituted $C_{1-6}$ alkyl or optionally substituted $C_{1-6}$ alkoxy;
optionally, 2'-O-$(CH_2)_n$-$R_1$ is selected from 2'-O-$CH_3$, 2'-O-$CH_2$-O-$CH_3$, 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$ or 2'-O-$CH_2$-$CH_2$-O-$CH_3$.

**9.** The double-stranded oligonucleotide according to claim 4, wherein the sense strand or the antisense strand comprises a 3' overhang having at least 1 nucleotide;

optionally, the antisense strand comprises a 3' overhang having at least 1 nucleotide;
optionally, the sense strand or the antisense strand comprises a 3 'overhang having at least 2 nucleotides;
optionally, the antisense strand comprises a 3' overhang having at least 2 nucleotides;
optionally, the antisense strand comprises a 3' overhang having 2 nucleotides;
optionally, the sense strand and/or the antisense strand independently comprise one or more phosphorothioate linkages;
optionally, the sense strand comprises two contiguous phosphorothioate linkages between terminal nucleotides at the 5'-end;
optionally, the antisense strand comprises two contiguous phosphorothioate linkages between terminal nucleotides respectively at the 3' and 5'-end.

**10.** The double-stranded oligonucleotide according to any one of claims 1-9, wherein all nucleotides of the sense strand and all nucleotides of the antisense strand are selected from modified nucleotides; wherein the duplex region formed by the sense strand and the antisense strand is represented by the following formula (I):

SS:

5'- (N)a' - (X)p' - (N)b' - (X)q' - (N)c' - (X)r' - (N) d' -3'

AS:

3' - (N)a - (X)p - (N)b - (X)q - (N)c -5' (I),

wherein SS represents sense strand, AS represents antisense strand;
each N is independently selected from 2'-fluoro modified nucleotides, 2'-O-methyl modified nucleotides or 2'-deoxy modified nucleotides;
each X is independently selected from 2'-O-TBDMS, 2'-O-TIPS, 2'-O-TOM, 2'-O-$CH_2$-O-$CH_2$-$CH_3$, 2'-O-$CH_2$-O-$CH_2$-$CF_3$, 2'-O-$CH_2$-$CH_2$-O-$CH_3$;
the a, a', p, p', b, b', q, q', c, c', r', d' each independently represents the number of nucleotides, wherein: a' is selected from an integer of 3-8; p' is selected from an integer of 0-3; b' is selected from an integer of 4-13; q' is selected from an integer of 0-4; c' is selected from an integer of 3-9; r' is selected from an integer of 0-3; d' is selected from an integer of 0-9; a is selected from an integer of 4-7; p is selected from an integer of 0-1; b is selected from an integer of 4-8; q is selected from an integer of 0-4; c is selected from an integer of 6-10; and p', q', r', p, q are not simultaneously 0, and $0 \leq q'+r' \leq 4$;
optionally, a' is selected from an integer of 3-8, p' is selected from 0 or 1, b' is selected from an integer of 4-13, q' is selected from 0 or 1, c' is selected from an integer of 3-9; r' is selected from 0 or 1, d' is selected from an integer of 1-8, a is selected from an integer of 4-7, p is selected from 1, b is selected from an integer of 4-8, q is selected from 0 or 1, c is selected from an integer of 6-10;
optionally, each N is independently selected from 2'-O-methyl modified nucleotides, 2'-fluoro modified nucleotides;
optionally, each X is independently selected from 2'-O-methyl modified nucleotides, 2'-O-MOE (methoxyethyl) modified nucleotides, 2'-O-TBDMS modified nucleotides, 2'-O-TIPS modified nucleotides, 2'-O-TOM modified nucleotides, 2'-O-$CH_2$-O-$CH_2$-$CH_3$ modified nucleotides, 2'-O-$CH_2$-O-$CH_2$-$CF_3$ modified nucleotides;
preferably, each X is independently selected from 2'-O-methyl modified nucleotides, 2'-O-MOE modified nucleotides, 2'-O-$CH_2$-O-$CH_2$-$CH_3$ modified nucleotides, 2'-O-$CH_2$-O-$CH_2$-$CF_3$ modified nucleotides;
optionally, the duplex region comprises at least one 2'-O-methoxyethyl modified nucleotide or 2'-O-ethoxymethyl modified nucleotide.

**11.** The double-stranded oligonucleotide according to claim 10, wherein in the direction from the 5'-end to the 3'-end, at least four of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides;

optionally, in the direction from the 5'-end to the 3'-end, at least five of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides;
optionally, in the direction from the 5'-end to the 3'-end, arbitrarily five of the nucleotides at positions 2, 6, 9, 12, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides;
optionally, in the direction from the 5'-end to the 3'-end, the nucleotides at positions 2, 6, 9, 14 and 16 of the antisense strand are selected from 2'-fluoro modified nucleotides, and the nucleotides at the remaining positions are selected from 2'-O-methyl modified nucleotides or 2'-O-methoxyethyl modified nucleotides;
optionally, the antisense strand comprises at least one 2'-O-methoxyethyl modified nucleotide;
optionally, in the direction from the 5'-end to the 3'-end, the nucleotide at position 15 of the antisense strand is selected from a 2'-O-methoxyethyl modified nucleotide; at least four of the nucleotides at positions 2, 6, 9, 12, 14, and 16 are selected from 2'-fluoro modified nucleotides; and the nucleotides at the remaining positions are selected from 2'-O-methyl modified nucleotides;
optionally, in the direction from the 5'-end to the 3'-end, the nucleotide at position 15 of the antisense strand is selected from a 2'-O-methoxyethyl modified nucleotide; at least five of the nucleotides at positions 2, 6, 9, 12, 14, and 16 are selected from 2'-fluoro modified nucleotides; and the nucleotides at the remaining positions are selected from 2'-O-methyl modified nucleotides;
optionally, in the direction from the 5'-end to the 3'-end, the nucleotide at position 15 of the antisense strand is selected from a 2'-O-methoxyethyl modified nucleotide; arbitrarily five of the nucleotides at positions 2, 6, 9, 12, 14, and 16 are selected from 2'-fluoro modified nucleotides; and the nucleotides at the remaining positions are selected from 2'-O-methyl modified nucleotides;
optionally, in the direction from the 5'-end to the 3'-end, the nucleotide at position 15 of the antisense strand is

selected from a 2'-O-methoxyethyl modified nucleotide; the nucleotides at positions 2, 6, 9, 14, and 16 are selected from 2'-fluoro modified nucleotides; and the nucleotides at the remaining positions are selected from 2'-O-methyl modified nucleotides;

optionally, in the direction from the 5'-end to the 3'-end, the nucleotide at position 15 of the antisense strand is selected from a 2'-O-methoxyethyl modified nucleotide; the nucleotides at positions 2, 6, 12, 14, and 16 are selected from 2'-fluoro modified nucleotides; and the nucleotides at the remaining positions are selected from 2'-O-methyl modified nucleotides.

**12.** The double-stranded oligonucleotide according to claim 10, wherein in the direction from the 5'-end to the 3'-end, at least three of the nucleotides at positions 7-10 of the sense strand are selected from 2'-fluoro modified nucleotides, and the nucleotides at the remaining positions are selected from 2'-O-methyl modified nucleotides;
optionally, in the direction from the 5'-end to the 3'-end, the nucleotides at positions 7-10 of the sense strand are selected from 2'-fluoro modified nucleotides, and the nucleotides at the remaining positions are selected from 2'-O-methyl modified nucleotides.

**13.** The double-stranded oligonucleotide of claim 10, wherein the antisense strand comprises at least 15, at least 16, at least 17, at least 18, at least 19, at least 20, or at least 21 contiguous nucleotides of any one of the modified antisense strand nucleotide sequences shown in Table 2, or a nucleotide sequence having no more than 3 nucleotide differences from the contiguous nucleotides;

optionally, the antisense strand comprises at least 17, at least 18, at least 19, at least 20, or at least 21 contiguous nucleotides of any one of the modified antisense strand nucleotide sequences shown in Table 2;

optionally, the antisense strand is selected from or comprises any one of the modified antisense strand nucleotide sequences shown in Table 2.

**14.** The double-stranded oligonucleotide according to claim 10, wherein the sense strand comprises at least 15, at least 16, at least 17, at least 18, at least 19 contiguous nucleotides of any one of the modified sense strand nucleotide sequences shown in Table 2, or a nucleotide sequence differing from the contiguous nucleotides by 1, 2, or 3 nucleotides;
optionally, the sense strand of the double-stranded oligonucleotide is selected from or comprises any of the modified sense strand nucleotide sequences shown in Table 2.

**15.** The double-stranded oligonucleotide according to claim 10, wherein the sense strand of the double-stranded oligonucleotide is selected from or comprises any one of the modified sense strand nucleotide sequences shown in Table 2; and the antisense strand is selected from or comprises any one of the modified antisense strand nucleotide sequences shown in Table 2.

**16.** The double-stranded oligonucleotide according to any one of claims 1-15, wherein the double-stranded oligonucleotide is selected from siRNA.

**17.** A double-stranded oligonucleotide conjugate, wherein the conjugate comprising the double-stranded oligonucleotide according to any one of claims 1-15 and one or more ligands capable of binding to a cellular receptor;

optionally, the ligand is conjugated to the sense strand and/or the antisense strand;
optionally, the ligand is conjugated to the 3'-end and/or 5'-end of the sense strand;
optionally, the ligand is conjugated to the 3'-end of the sense strand;
optionally, the cellular receptor is selected from asialoglycoprotein receptor;
optionally, the ligand comprises galactose or cluster of galactose.

**18.** The conjugate according to claim 17, wherein the ligand is selected from the structure of formula (101), or an isomer thereof, or a pharmaceutically acceptable salt thereof:

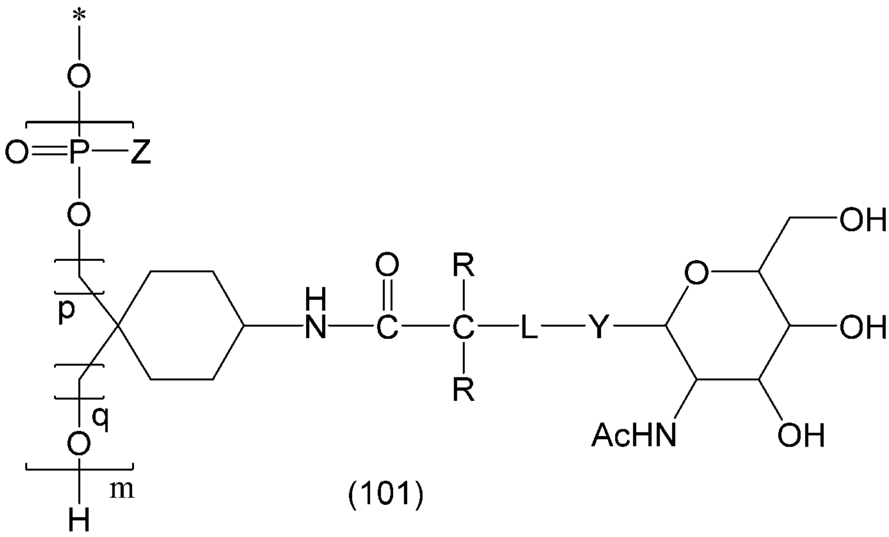

(101)

wherein, * represents the conjugation site between the ligand and the sense strand or the antisense strand;
m is selected from 1, 2, 3, or 4;
each Z is independently selected from hydroxyl or mercapto;
each p is independently selected from 1, 2, or 3;
each q is independently selected from 1, 2, or 3;
each R is independently selected from H, optionally substituted C1-C6 alkyl, or optionally substituted C1-C6 alkoxy;
each L is independently selected from optionally substituted C2-C20 alkylene or

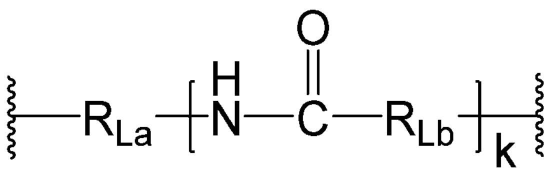

;

$R_{La}$ and $R_{Lb}$ are independently selected from optionally substituted C1-C10 alkylene, and k is selected from 1, 2, 3, 4, or 5;
each Y is independently selected from O, S, or NH;
optionally, m is selected from 1, 2, or 3;
optionally, Z is selected from hydroxyl;
optionally, p is selected from 1;
optionally, q is selected from 1;
optionally, R is selected from H;
optionally, each L is independently selected from C1-C10 alkylene or

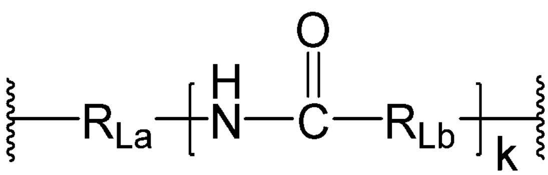

;

wherein, $R_{La}$ and $R_{Lb}$ are independently selected from C1-C5 alkylene, and k is 1, 2, or 3;
optionally, k is selected from 1;
optionally, each L is independently selected from

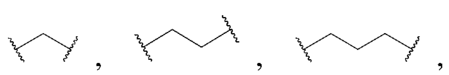

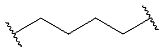 , 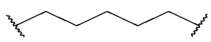 , 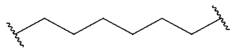 , 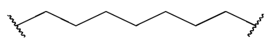 ,

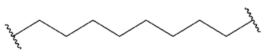 , 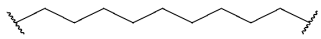 , 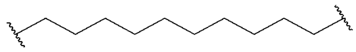 ,

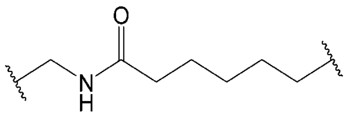 , 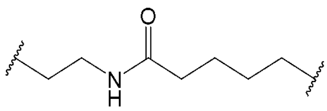 , 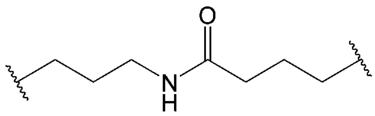 ,

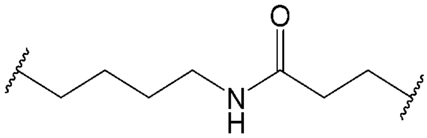 or 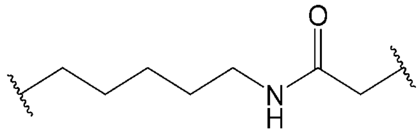 ;

optionally, Y is selected from O.

**19.** The conjugate of claim 17, wherein the ligand is selected from the following structure, or an isomer thereof, or a pharmaceutically acceptable salt thereof:

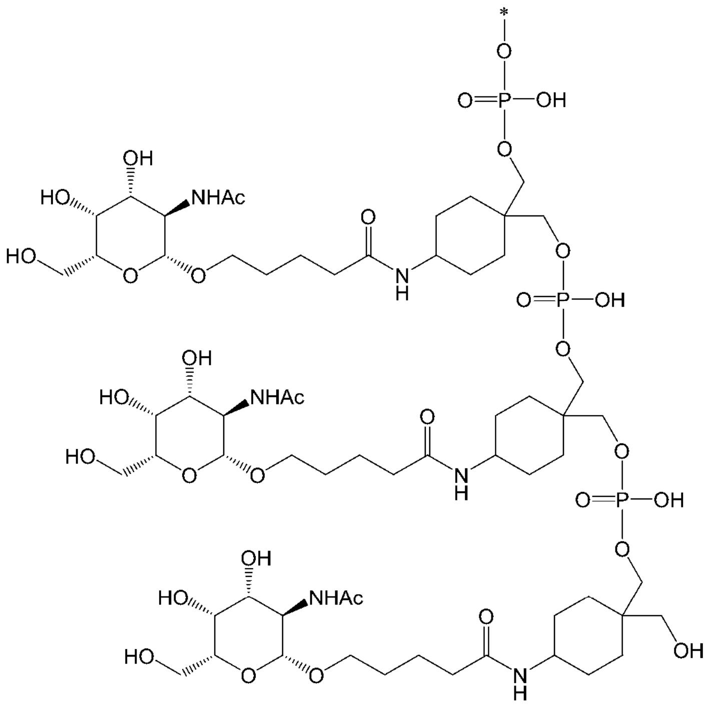

.

**20.** The conjugate according to claim 17, wherein the ligand is selected from the structure shown below, or an isomer thereof, or a pharmaceutically acceptable salt thereof:

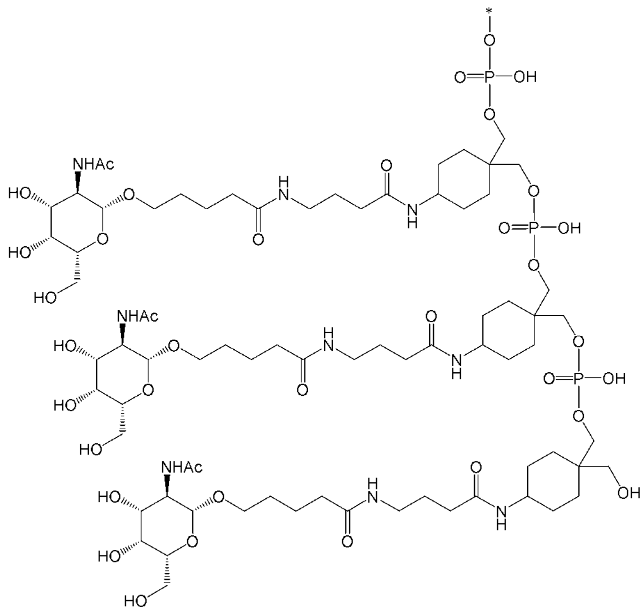

wherein, * represents the conjugation site between the ligand and the sense strand or the antisense strand.

**21.** A composition comprising any one of:

(I) the double-stranded oligonucleotide according to any one of claims 1-16; and/or
(II) the conjugate according to any one of claims 17-20.

**22.** Use of any one of:

(I) the double-stranded oligonucleotide according to any one of claims 1-16; and/or
(II) the conjugate according to any one of claims 17-20; and/or
(III) the composition according to claim 21,
in the manufacture of a medicament for the prevention and/or treatment of a disease or disorder mediated by INHBE gene;
optionally, the disease or disorder comprises, but is not limited to, having or being at the risk of developing metabolic disorder, type 2 diabetes, obesity, elevated triglyceride levels, lipodystrophy, liver inflammation, fatty liver disease, hypercholesterolemia, elevated liver enzymes, non-alcoholic steatohepatitis (NASH), cardiovascular disease, cardiomyopathy, hypertension, and/or heart failure.

**23.** A pharmaceutical composition comprising any one of:

(I) the double-stranded oligonucleotide according to any one of claims 1-16; and/or
(II) the conjugate according to any one of claims 17-20; and/or
(III) the composition according to claim 21, and

a pharmaceutically acceptable excipient or auxiliary agent.

**24.** A method for reducing the expression or activity of INHBE gene, comprising contacting a cell with any one of:

(I) the double-stranded oligonucleotide according to any one of claims 1-16; and/or
(II) the conjugate according to any one of claims 17-20; and/or
(III) the composition according to claim 21; and/or
(IV) the pharmaceutical composition according to claim 23.

**25.** A method for preventing and/or treating a disease or disorder mediated by INHBE gene, comprising administering to a subject pharmaceutically acceptable amount of any one of:

(I) the double-stranded oligonucleotide according to any one of claims 1-16; and/or
(II) the conjugate according to any one of claims 17-20; and/or
(III) the composition according to claim 21; and/or
(IV) the pharmaceutical composition according to claim 23.

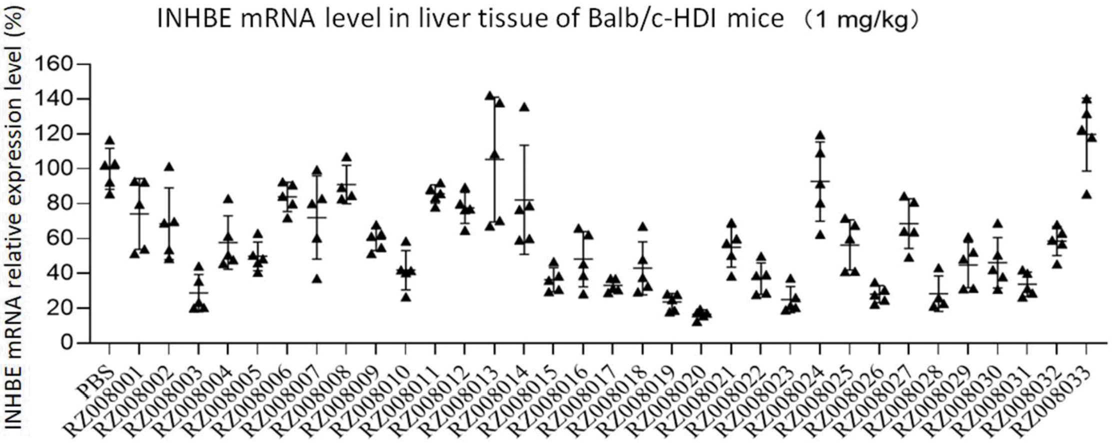
INHBE mRNA level in liver tissue of Balb/c-HDI mice （1 mg/kg）
INHBE mRNA relative expression level (%)
0
20
40
60
80
100
120
140
160
PBS
RZ008001
RZ008002
RZ008003
RZ008004
RZ008005
RZ008006
RZ008007
RZ008008
RZ008009
RZ008010
RZ008011
RZ008012
RZ008013
RZ008014
RZ008015
RZ008016
RZ008017
RZ008018
RZ008019
RZ008020
RZ008021
RZ008022
RZ008023
RZ008024
RZ008025
RZ008026
RZ008027
RZ008028
RZ008029
RZ008030
RZ008031
RZ008032
RZ008033

FIG. 1

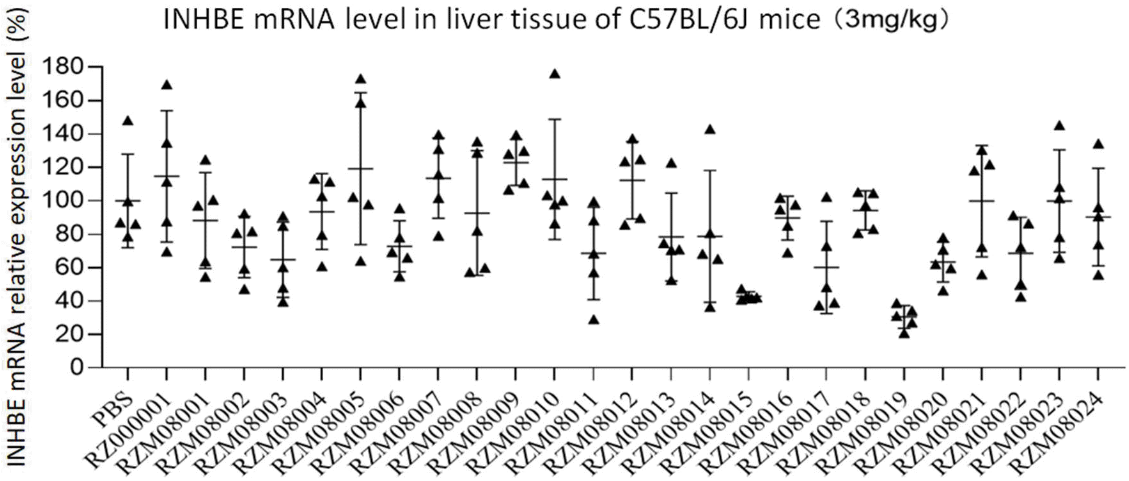
INHBE mRNA level in liver tissue of C57BL/6J mice（3mg/kg）
INHBE mRNA relative expression level (%)
0
20
40
60
80
100
120
140
160
180
PBS
RZ000001
RZM08001
RZM08002
RZM08003
RZM08004
RZM08005
RZM08006
RZM08007
RZM08008
RZM08009
RZM08010
RZM08011
RZM08012
RZM08013
RZM08014
RZM08015
RZM08016
RZM08017
RZM08018
RZM08019
RZM08020
RZM08021
RZM08022
RZM08023
RZM08024

FIG. 2

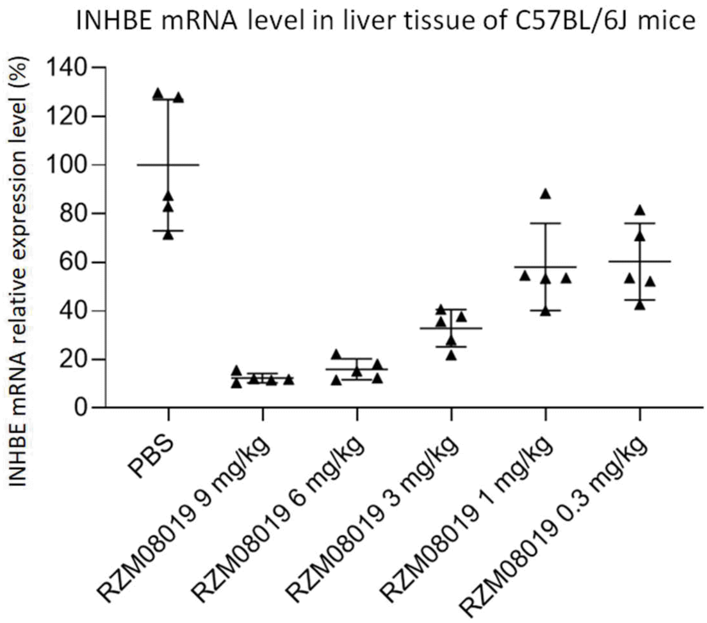
INHBE mRNA level in liver tissue of C57BL/6J mice
INHBE mRNA relative expression level (%)
140
120
100
80
60
40
20
0
PBS
RZM08019 9 mg/kg
RZM08019 6 mg/kg
RZM08019 3 mg/kg
RZM08019 1 mg/kg
RZM08019 0.3 mg/kg

FIG. 3

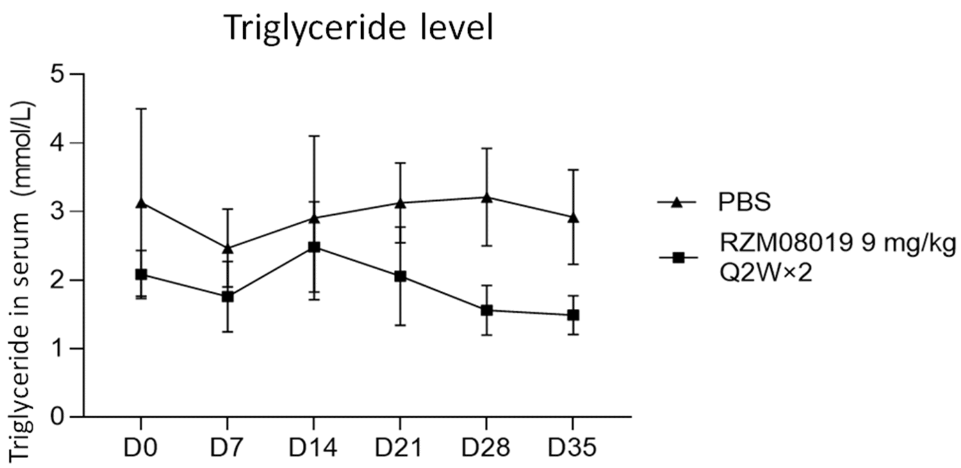
Triglyceride level
Triglyceride in serum (mmol/L)
5
4
3
2
1
0
D0
D7
D14
D21
D28
D35
PBS
RZM08019 9 mg/kg
Q2W×2

FIG. 4

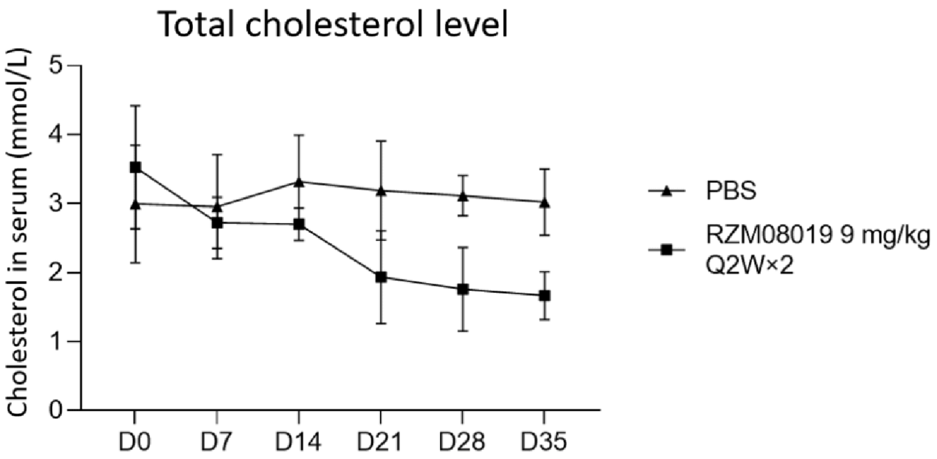
Total cholesterol level
Cholesterol in serum (mmol/L)
0
1
2
3
4
5
D0
D7
D14
D21
D28
D35
PBS
RZM08019 9 mg/kg Q2W×2

FIG. 5

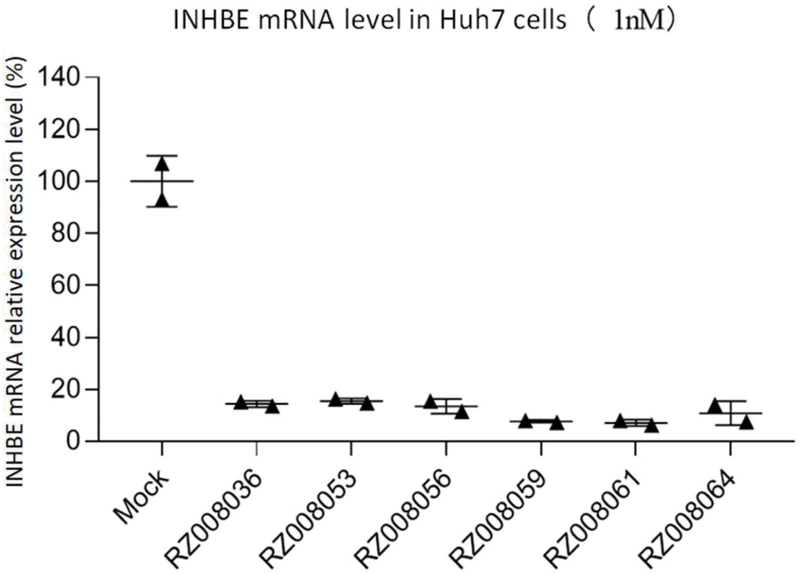
INHBE mRNA level in Huh7 cells（ 1nM）
INHBE mRNA relative expression level (%)
0
20
40
60
80
100
120
140
Mock
RZ008036
RZ008053
RZ008056
RZ008059
RZ008061
RZ008064

FIG. 6

**INTERNATIONAL SEARCH REPORT**

International application No.
**PCT/CN2024/112606**

**A. CLASSIFICATION OF SUBJECT MATTER**

C12N15/113(2010.01)i; A61K31/7125(2006.01)i; A61K31/713(2006.01)i; A61K31/7088(2006.01)i; A61P3/00(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNABS, CNTXT, WOTXT, USTXT, JPTXT, KRTXT, VEN, CNKI, 百度学术, Baidu Scholar, 必应, Bing, pubmed, ISI web of science, STN: 炫景, 黄渊余, 李海涛, 孔丽娜, 双链寡核苷酸, siRNA, INHBE, SEQ ID NOs: 1-309, NM_031479.5, inhibin subunit beta E, 抑制素βE, 抑制素亚基βE, activin beta E, 激活素E, 活化素βE亚基, Activin β, 配体, RZM08019, RZM08015, L96, RZ008003, RZ008019, RZ008020, RZ008023, RZ008026, RZ008028

**C. DOCUMENTS CONSIDERED TO BE RELEVANT**

| Category* | Citation of document, with indication, where appropriate, of the relevant passages | Relevant to claim No. |
|---|---|---|
| X | WO 2023003922 A1 (ALNYLAM PHARMACEUTICALS, INC.) 26 January 2023 (2023-01-26) description, page 4, paragraph 3 from the bottom, page 44, paragraph 4 to page 48, paragraph 5, and page 87, paragraphs 2-4, tables 1-3 and 19-20, and claims 1-173 | 1-17, 21-25 |
| Y | WO 2023003922 A1 (ALNYLAM PHARMACEUTICALS, INC.) 26 January 2023 (2023-01-26) description, page 4, paragraph 3 from the bottom, page 44, paragraph 4 to page 48, paragraph 5, and page 87, paragraphs 2-4, tables 1-3 and 19-20, and claims 1-173 | 18-25 |
| PY | WO 2024140101 A1 (RIGERNA THERAPEUTICS (BEIJING) CO., LTD.) 04 July 2024 (2024-07-04) description, pages 4-6 | 18-25 |
| A | CN 106659769 A (ACCELERON PHARMA, INC.) 10 May 2017 (2017-05-10) description, paragraphs 62 and 303 | 1-25 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 November 2024** | **18 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/112606**

**C. DOCUMENTS CONSIDERED TO BE RELEVANT**

| Category* | Citation of document, with indication, where appropriate, of the relevant passages | Relevant to claim No. |
|---|---|---|
| E | WO 2024179573 A1 (TUOJIE BIOTECH (SHANGHAI) CO., LTD.) 06 September 2024 (2024-09-06)<br>claims 1-17, and sequence listing | 1-9, 17, 21-25 |
| E | WO 2024187190 A2 (BASECURE THERAPEUTICS LLC) 12 September 2024 (2024-09-12)<br>claims 1-81, and sequence listing | 1-9, 17, 21-25 |
| A | SUGIYAMA, M. et al. "Inhibin βE (INHBE) is a Possible Insulin Resistance-associated Hepatokine Identified by Comprehensive Gene Expression Analysis in Human Liver Biopsy Samples"<br>*PLoS One,* Vol. 13, No. (3), 29 March 2018 (2018-03-29), e0194798<br>page 4, paragraph 2 | 1-25 |

Form PCT/ISA/210 (second sheet) (July 2022)

**INTERNATIONAL SEARCH REPORT**

International application No.
**PCT/CN2024/112606**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/112606**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25**
because they relate to subject matter not required to be searched by this Authority, namely:

Claim 25 sets forth a method for preventing and/or treating INHBE gene-mediated diseases or conditions, which method falls within methods for treatment of diseases as defined in PCT Rule 39.1(iv); therefore, a search is performed on the basis of the pharmaceutical use corresponding to the method of claim 25.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/112606**

| Patent document cited in search report | Publication date (day/month/year) | Patent family member(s) | Publication date (day/month/year) |
|---|---|---|---|
| WO 2023003922 A1 | 26 January 2023 | CO 2024000239 A2 | 05 February 2024 |
| | | WO 2023003922 A8 | 16 November 2023 |
| | | MX 2024000981 A | 12 February 2024 |
| | | JP 2024526890 A | 19 July 2024 |
| | | AU 2022314619 A1 | 04 January 2024 |
| | | EP 4373937 A1 | 29 May 2024 |
| | | CL 2024000130 A1 | 16 August 2024 |
| | | US 2024309370 A1 | 19 September 2024 |
| | | KR 20240036041 A | 19 March 2024 |
| | | CA 3226887 A1 | 26 January 2023 |
| | | IL 309897 A | 01 March 2024 |
| | | TW 202421169 A | 01 June 2024 |
| WO 2024140101 A1 | 04 July 2024 | None | |
| CN 106659769 A | 10 May 2017 | CA 2942954 A1 | 24 September 2015 |
| | | AU 2015231022 A1 | 29 September 2016 |
| | | AU 2015231022 B2 | 04 February 2021 |
| | | JP 2017509647 A | 06 April 2017 |
| | | EP 3119418 A1 | 25 January 2017 |
| | | EP 3119418 A4 | 03 January 2018 |
| | | EP 3119418 B1 | 23 February 2022 |
| | | US 2021155672 A1 | 27 May 2021 |
| | | US 2016046690 A1 | 18 February 2016 |
| | | AU 2023219885 A1 | 14 September 2023 |
| | | AU 2021200301 A1 | 18 March 2021 |
| | | KR 20230004942 A | 06 January 2023 |
| | | KR 102520970 B1 | 12 April 2023 |
| | | MA 39722 A | 02 June 2021 |
| | | KR 20160127148 A | 02 November 2016 |
| | | WO 2015143403 A1 | 24 September 2015 |
| | | JP 2020111621 A | 27 July 2020 |
| | | JP 7154250 B2 | 17 October 2022 |
| WO 2024179573 A1 | 06 September 2024 | None | |
| WO 2024187190 A2 | 12 September 2024 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- CN 202311053101

### Non-patent literature cited in the description

- Handbook of Chemistry and Physics. 1994
- Organic Chemistry. Thomas Sorrell, 1999
- **MICHAEL B. SMITH** ; **JERRY MARCH**. March's Advanced Organic Chemistry. John Wiley & Sons, 2007